# EUROPEAN PATENT SPECIFICATION

(11) **EP 3 874 060 B1**
(45) Date of publication and mention of the grant of the patent: **16.10.2024**
(21) Application number: 19809670.3
(22) Date of filing: 31.10.2019
(51) Int. Cl.: C12Q 1/6869, C12Q 1/6806, C12Q 1/6886

(54) **METHOD AND SYSTEM FOR CALIBRATING EPIGENETIC PARTITIONING ASSAYS**
VERFAHREN UND SYSTEM ZUR KALIBRIERUNG EPIGENETISCHER PARTITIONSTESTS
PROCÉDÉE ET SYSTÈME POUR ÉTALONNER DES ESSAIS DE PARTITIONNEMENT ÉPIGÉNÉTIQUE

(30) Priority: 31.10.2018 US 201862753826 P
(43) Date of publication of application: 08.09.2021
(73) Proprietor: Guardant Health, Inc., Palo Alto, CA 94304 (US)
(72) Inventor: KENNEDY, Andrew, La Jolla, California 92037 (US); WESTESSON, Oscar, Redwood City, California 94063 (US); HE, Yupeng, Redwood City, California 94063 (US); SCHULTZ, Matthew, Redwood City, California 94063 (US)
(74) Representative: Carpmaels & Ransford LLP
(86) International application number: PCT/US2019/059217
(87) International publication number: WO 2020/092807

(56) References cited:
- WO-A2-2005/040399
- WO-A2-2008/009365
- WO-A2-2010/033639

## Description

### CROSS-REFERENCE TO RELATED APPLICATION

### BACKGROUND

Cancer is a major cause of disease worldwide. Each year, tens of millions of people are diagnosed with cancer around the world, and more than half eventually die from it. In many countries, cancer ranks as the second most common cause of death following cardiovascular diseases. Early detection is associated with improved outcomes for many cancers.

Cancer can be caused by the accumulation of genetic variations within an individual's normal cells, at least some of which result in improperly regulated cell division. Such variations commonly include copy number variations (CNVs), single nucleotide variations (SNVs), gene fusions, insertions and/or deletions (indels), epigenetic variations include 5-methylation of cytosine (5-methylcytosine) and association of DNA with chromatin and transcription factors.

Cancers are often detected by biopsies of tumors followed by analysis of cells, markers or DNA extracted from cells. But more recently it has been proposed that cancers can also be detected from cell-free nucleic acids in body fluids, such as blood or urine. Such tests have the advantage that they are noninvasive and can be performed without identifying suspected cancer cells in biopsy. However, such liquid biopsy tests are complicated by the fact that amount of nucleic acids in body fluids is very low and what nucleic acid are present are heterogeneous in form (e.g., RNA and DNA, single-stranded and double-stranded, and various states of post-replication modification and association with proteins, such as histones).

### SUMMARY

The present invention is defined by the appended claims. Furthermore described is a method for evaluating partitioning of nucleic acid molecules in a sample of polynucleotides based on epigenetic state, comprising: a) adding a set of epigenetic-control nucleic acid molecules to the nucleic acid molecules in the sample of polynucleotides, thereby producing a spiked-in sample; b) partitioning nucleic acid molecules of at least a subset of the spiked-in sample into a plurality of partitioned sets; c) enriching at least a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides; d) sequencing at least a subset of the set of enriched molecules to produce a set of sequencing reads; e) analyzing at least a subset of the set of sequencing reads to generate one or more epigenetic partition scores of the epigenetic-control nucleic acid molecules; and f) comparing the one or more epigenetic partition scores with one or more epigenetic partition cut-offs.

In another aspect, the present disclosure provides a method for evaluating partitioning of nucleic acid molecules in a sample of polynucleotides based on epigenetic state, comprising: a) adding a set of epigenetic-control nucleic acid molecules to the nucleic acid molecules in the sample of polynucleotides, thereby producing a spiked-in sample; b) partitioning nucleic acid molecules of at least a subset of the spiked-in sample into a plurality of partitioned sets; c) enriching at least a subset of molecules comprising specific target genomic regions from the plurality of partitioned sets to generate a set of enriched molecules, wherein the set of enriched molecules comprises a group of epigenetic-control nucleic acid molecules and a group of nucleic acid molecules from the sample of polynucleotides, wherein the group of nucleic acid molecules from the sample of polynucleotides comprises a set of endogenous control molecules, which correspond to at least one human genomic region with a non-variable epigenetic state; d) sequencing at least a subset of the set of enriched molecules to produce a set of sequencing reads; e) analyzing at least a subset of the set of sequencing reads to generate one or more epigenetic partition scores for the epigenetic-control nucleic acid molecules and the set of endogenous control molecules; and f) comparing the one or more epigenetic partition scores with one or more epigenetic partition cut-offs.

In another aspect, the present disclosure provides a method for evaluating partitioning of nucleic acid molecules in a sample of polynucleotides based on epigenetic state, comprising: a) partitioning nucleic acid molecules from at least a subset of the sample of polynucleotides into a plurality of partitioned sets; b) enriching at least a subset of molecules comprising specific target genomic regions from the plurality of partitioned sets to generate a set of enriched molecules, wherein the set of enriched molecules comprises a group of nucleic acid molecules from the sample of polynucleotides, wherein the group of nucleic acid molecules from the sample of polynucleotides comprises a set of endogenous control molecules, which correspond to at least one human genomic region with a non-variable epigenetic state; c) sequencing at least a subset of the set of enriched molecules to produce a set of sequencing reads; d) analyzing a subset of the set of sequencing reads to generate one or more epigenetic partition scores for the set of endogenous control molecules; and e) comparing the one or more epigenetic partition scores with one or more epigenetic partition cut-offs.

Furthermore described is a method for evaluating partitioning of nucleic acid molecules in a sample of polynucleotides based on epigenetic state, comprising: a) adding a set of epigenetic-control nucleic acid molecules to the nucleic acid molecules in the sample of polynucleotides, thereby producing a spiked-in sample; b) partitioning nucleic acid molecules of at least a subset of the spiked-in sample into a plurality of partitioned sets; c) sequencing at least a subset of the partitioned molecules to produce a set of sequencing reads; d) analyzing at least a subset of the set of sequencing reads to generate one or more epigenetic partition scores of the epigenetic-control nucleic acid molecules; and e) comparing the one or more epigenetic partition scores with one or more epigenetic partition cut-offs. In some embodiments, the method further comprises, prior to the sequencing step, enriching at least a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides.

Furthermore described is a method for evaluating partitioning of nucleic acid molecules in a sample of polynucleotides based on epigenetic state, comprising: a) adding a set of epigenetic-control nucleic acid molecules to the nucleic acid molecules in the sample of polynucleotides, thereby producing a spiked-in sample; b) partitioning nucleic acid molecules of at least a subset of the spiked-in sample into a plurality of partitioned sets; c) sequencing at least a subset of the partitioned molecules to produce a set of sequencing reads; d) analyzing at least a subset of the set of sequencing reads to generate one or more epigenetic partition scores for the epigenetic-control nucleic acid molecules and the set of endogenous control molecules; and e) comparing the one or more epigenetic partition scores with one or more epigenetic partition cut-offs. In some embodiments, the method further comprises, prior to the sequencing, enriching at least a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides, wherein the group of nucleic acid molecules from the sample of polynucleotides comprises a set of endogenous control molecules.

Furthermore described is a method for evaluating partitioning of nucleic acid molecules in a sample of polynucleotides based on epigenetic state, comprising: a) partitioning molecules from at least a subset of the sample of polynucleotides into a plurality of partitioned sets; b) sequencing at least a subset of the set of enriched molecules to produce a set of sequencing reads; d) analyzing a subset of the set of sequencing reads to generate one or more epigenetic partition scores for the set of endogenous control molecules; and e) comparing the one or more epigenetic partition scores with one or more epigenetic partition cut-offs. In some embodiments, the method further comprises, prior to the sequencing, enriching at least a subset of molecules from the plurality of partitioned sets to generate a set of enriched molecules, wherein the set of enriched molecules comprises a group of nucleic acid molecules from the sample of polynucleotides, wherein the group of nucleic acid molecules from the sample of polynucleotides comprises a set of endogenous control molecules.

Furthermore described is a method for evaluating partitioning of nucleic acid molecules in a sample of polynucleotides based on epigenetic state, comprising: a) adding a set of epigenetic-control nucleic acid molecules to the nucleic acid molecules in the sample of polynucleotides, thereby producing a spiked-in sample; b) partitioning nucleic acid molecules of at least a subset of the spiked-in sample into a plurality of partitioned sets; c) enriching at least a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides; and d) sequencing at least a subset of the set of enriched molecules to produce a set of sequencing reads. In some embodiments, the method further comprises, e) analyzing at least a subset of the set of sequencing reads to generate one or more epigenetic partition scores of the epigenetic-control nucleic acid molecules; and f) comparing the one or more epigenetic partition scores with one or more epigenetic partition cut-offs.

Furthermore described is a method for evaluating partitioning of nucleic acid molecules in a sample of polynucleotides based on epigenetic state, comprising: a) adding a set of epigenetic-control nucleic acid molecules to the nucleic acid molecules in the sample of polynucleotides, thereby producing a spiked-in sample; b) partitioning nucleic acid molecules of at least a subset of the spiked-in sample into a plurality of partitioned sets; c) enriching at least a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides, wherein the group of nucleic acid molecules from the sample of polynucleotides comprises a set of endogenous control molecules; and d) sequencing at least a subset of the set of enriched molecules to produce a set of sequencing reads. In some embodiments, the method further comprises, e) analyzing at least a subset of the set of sequencing reads to generate one or more epigenetic partition scores for the epigenetic-control nucleic acid molecules and the set of endogenous control molecules; and f) comparing the one or more epigenetic partition scores with one or more epigenetic partition cut-offs.

Furthermore described is a method for evaluating partitioning of nucleic acid molecules in a sample of polynucleotides based on epigenetic state, comprising: a) partitioning nucleic acid molecules from at least a subset of the sample of polynucleotides into a plurality of partitioned sets; b) enriching at least a subset of molecules from the plurality of partitioned sets to generate a set of enriched molecules, wherein the set of enriched molecules comprises a group of nucleic acid molecules from the sample of polynucleotides, wherein the group of nucleic acid molecules from the sample of polynucleotides comprises a set of endogenous control molecules; and c) sequencing at least a subset of the set of enriched molecules to produce a set of sequencing reads. In some embodiments, the method further comprises, d) analyzing a subset of the set of sequencing reads to generate one or more epigenetic partition scores for the set of endogenous control molecules; and e) comparing the one or more epigenetic partition scores with one or more epigenetic partition cut-offs.

In some embodiments, the analyzing step comprises estimating the number/fraction of the epigenetic-control nucleic acid molecules and/or endogenous control molecules at a given epigenetic state in at least one of the partitioned sets.

In some embodiments, the method further comprises tagging the nucleic acid molecules in a partitioned set of the plurality of partitioned sets with a set of tags to produce a population of tagged nucleic acid molecules, wherein the tagged nucleic acid molecules comprise one or more tags. In some embodiments, the set of tags (molecular barcodes) used in a first partitioned set of the plurality of partitioned sets is different from the set of tags (molecular barcodes) used in a second partitioned set of the plurality of partitioned sets. In some embodiments, the set of tags are attached to the nucleic acid molecules by ligation of adapters to the nucleic acid molecules, wherein the adapters comprise one or more tags (molecular barcodes). The tag (molecular barcode) sequences employed may be correlated with partitioned set, e.g. tags (molecular barcodes) used in one partitioned set are not used in other partitioned sets.

In some embodiments, the method further comprises g) classifying the partitioning method as (i) being a success, if each of the one or more epigenetic partition scores of the epigenetic-control nucleic acid molecules and/or the set of endogenous control molecules is within the corresponding epigenetic partition cut-off; or (ii) being unsuccessful, if at least one of the one or more epigenetic partition scores of the epigenetic control molecules and/or the set of endogenous control molecules is outside the corresponding epigenetic partition cut-offs.

In some embodiments, the set of epigenetic-control nucleic acid molecules comprises two or more subsets of epigenetic-control nucleic acid molecules, wherein a subset of the two or more subsets of epigenetic-control nucleic acid molecules comprises a plurality of epigenetic-control nucleic acid molecules comprising an epigenetic modification region.

In some embodiments, the sequencing of the plurality of enriched molecules is performed by a nucleic acid sequencer. In some embodiments, the nucleic acid sequencer is a next generation sequencer.

In another aspect, the present disclosure provides a set of epigenetic-control nucleic acid molecules, comprising two or more subsets of epigenetic-control nucleic acid molecules, wherein a subset of the two or more subsets of epigenetic-control nucleic acid molecules comprises a plurality of epigenetic-control nucleic acid molecules comprising an epigenetic modification region.

In another aspect, the present disclosure provides a population of nucleic acids, comprising: (i) a set of epigenetic-control nucleic acid molecules, wherein the set of epigenetic-control nucleic acid molecules comprises two or more subsets of epigenetic-control nucleic acid molecules, wherein a subset of the two or more subsets of epigenetic-control nucleic acid molecules comprises a plurality of epigenetic-control nucleic acid molecules comprising an epigenetic modification region; and (ii) a set of nucleic acid molecules in a sample of polynucleotides from a subject.

In some embodiments, the epigenetic-control nucleic acid molecule further comprises an identifier region. In some embodiments, the identifier region is on one or both sides of the epigenetic modification region of the epigenetic-control nucleic acid molecules.

In some embodiments, the epigenetic modification region of the epigenetic-control nucleic acid molecules in at least one subset comprises at least one nucleotide with epigenetic modification. In some embodiments, the subset comprises epigenetic-control nucleic acid molecules with a same number of nucleotides with epigenetic modification. In some embodiments, the number of nucleotides with epigenetic modification in a first subset is different from the number of nucleotides with epigenetic modification in a second subset. In some embodiments, the nucleotide with epigenetic modification comprises a methylated nucleotide. In some embodiments, the methylated nucleotide comprises 5-methylcytosine. In some embodiments, the methylated nucleotide comprises 5-hydroxymethylcytosine.

In some embodiments, the identifier region of the epigenetic-control nucleic acid molecules comprises a molecular barcode. In some embodiments, the identifier region further comprises at least one epigenetic state barcode. In some embodiments, the identifier region comprises one or more primer binding sites.

In some embodiments, the epigenetic modification region of the plurality of epigenetic-control nucleic acid molecules in the two or more subsets comprises an identical nucleic acid sequence.

In some embodiments, the epigenetic modification region of the plurality of epigenetic-control nucleic acid molecules in a first subset comprises a nucleic acid sequence distinguishable from the nucleic acid sequence of the epigenetic modification region of the plurality of epigenetic-control nucleic acid molecules in a second subset.

In some embodiments, the epigenetic modification is DNA methylation.

In some embodiments, each subset of epigenetic-control nucleic acid molecules is in equimolar concentration. In some embodiments, each subset of epigenetic-control nucleic acid molecules is in non-equimolar concentration.

In some embodiments, the number of methylated nucleotides in the epigenetic-control nucleic acid molecules in at least one of the subsets is 0, 1, 2, 3, 4, 5, 6, 7, 8, 9, 10, 11, at least 12, at least 15, at least 20, at least 25, at least 30, at least 40 or at least 50.

In some embodiments, the epigenetic-control nucleic acid molecules comprise a sequence corresponding to lambda phage DNA, human genomic region or a combination of both.

In some embodiments, the epigenetic state is methylation level of the nucleic acid molecules. In some embodiments, the plurality of partitioned sets comprises nucleic acid molecules of the spiked-in sample partitioned based on the methylation level of the nucleic acid molecules.

In some embodiments, the epigenetic modification region of the epigenetic-control nucleic acid molecules comprises of a length of about 160 bp.

In some embodiments, the epigenetic modification region of the epigenetic-control nucleic acid molecules comprises a nucleic acid sequence corresponding to a non-human genome.

In some embodiments, the sample of polynucleotides is selected from the group consisting of a sample of DNA, a sample of RNA, a sample of polynucleotides, a sample of cell-free DNA, and a sample of cell-free RNA. In some embodiments, the sample of polynucleotides is selected from the group consisting of a sample of DNA, a sample of RNA, a sample of polynucleotides, a sample of cell-free DNA, and a sample of cell-free RNA. In some embodiments, the cell-free DNA is between 1 ng and 500 ng.

In some embodiments, the epigenetic-control nucleic acid molecules is between 1 femtomole and 200 femtomoles.

In some embodiments, the partitioning comprises partitioning the nucleic acid molecules based on a differential binding affinity of the nucleic acid molecules to a binding agent that preferentially binds to nucleic acid molecules comprising nucleotides with epigenetic modification.

In another aspect, the present disclosure provides a system for evaluating a partitioning method of nucleic acid molecules in a sample of polynucleotides based on epigenetic state, comprising: a communication interface that receives, over a communication network, a set of sequencing reads of a spiked-in sample generated by a nucleic acid sequencer, wherein the set of sequencing reads comprise (i) at least a first population of sequencing reads generated from polynucleotides originating from the sample, wherein the sequencing reads from the first population comprise a tag sequence and sequence derived from polynucleotide originating from the sample; and (ii) at least a second population of sequencing reads generated from epigenetic-control nucleic acid molecules, wherein the sequencing reads generated from the second population comprise an epigenetic modification region and optionally, an identifier region; a computer in communication with the communication interface, wherein the computer comprises one or more computer processors and a computer readable medium comprising machine-executable code that, upon execution by the one or more computer processors, implements a method comprising: receiving, over the communication network, the set of sequencing reads from the first and second populations of sequencing reads by the nucleic acid sequencer; analyzing at least a subset of the set of sequencing reads to generate one or more epigenetic partition scores of the epigenetic-control nucleic acid molecules and/or endogenous control molecules; and comparing the one or more epigenetic partition scores with one or more epigenetic partition cut-offs.

In another aspect, the present disclosure provides a system, comprising a controller comprising, or capable of accessing, computer readable media comprising non-transitory computer-executable instructions which, when executed by at least one electronic processor perform at least: (a) obtaining a set of sequencing reads of a spiked-in sample generated by a nucleic acid sequencer, wherein the spiked-in sample comprises polynucleotides of a sample and epigenetic-control nucleic acid molecules and the set of sequencing reads comprises (i) a first population of sequencing reads generated from polynucleotides of a sample and (ii) a second population of sequencing reads generated from epigenetic-control nucleic acid molecules; (b) analyzing at least a subset of the set of sequencing reads to generate one or more epigenetic partition scores of the epigenetic-control nucleic acid molecules and/or endogenous control molecules; and (c) comparing the one or more epigenetic partition scores with one or more epigenetic partition cut-offs.

In another aspect, the present disclosure provides a system, comprising a controller comprising, or capable of accessing, computer readable media comprising non-transitory computer-executable instructions which, when executed by at least one electronic processor performs at least: (a) obtaining a set of sequencing reads of a sample generated by a nucleic acid sequencer, wherein the set of sequencing reads comprises sequencing reads generated from polynucleotides of the sample; (b) analyzing at least a subset of the set of sequencing reads to generate one or more epigenetic partition scores of endogenous control molecules; and (c) comparing the one or more epigenetic partition scores with one or more epigenetic partition cut-offs.

The system further comprises g) generating an outcome status of the partitioning method based on the comparison of the epigenetic partition scores. In some embodiments, the outcome status of the partitioning method is classified as (i) successful, if the one or more epigenetic partition scores of the epigenetic-control nucleic acid molecules and/or the set of endogenous control molecules is within the corresponding epigenetic partition cut-offs; or (ii) unsuccessful, if at least one of the one or more epigenetic partition scores of the epigenetic control molecules and/or the endogenous control molecules is outside the corresponding epigenetic partition cut-off.

In some embodiments, the epigenetic partition score comprises a fraction or percentage of number of hypermethylated epigenetic-control nucleic acid molecules and/or hypermethylated control molecules in a partitioned set. In some embodiments, the epigenetic partition score comprises a fraction or percentage of number of hypomethylated epigenetic-control nucleic acid molecules and/or hypomethylated control molecules in a partitioned set. In some embodiments, the partitioned set is hypermethylated partitioned set. In some embodiments, the partitioned set is hypomethylated partitioned set. In some embodiments, the epigenetic partition score is *0 CG score.* In some embodiments, the epigenetic partition score is *hypo score.* In some embodiments, the epigenetic partition score is *methyl-half.* In some embodiments, the epigenetic partition score is *methyl-5.*

In some embodiments, the epigenetic partition cut off for the 0 *CG score* is 0.01%, 0.02%, 0.05%, 0.1%, 0.2%, 0.3%, 0.4%, 0.5%, 0.6%, 0.7%, 0.8%, 0.9%, 1%, 2%, 5%, at least 5% or at least 10%. In some embodiments, the epigenetic partition cut off for the *hypo score* is 0.1%, 0.5%, 1%, 2%, 3%, 4%, 5%, 7% or at least 10%. In some embodiments, the epigenetic partition cut off for the *methyl-half* is 5, 10, 15, 20, 25, 30, 35 or 40 mCGs. In some embodiments, the epigenetic partition cut off for the *methyl-5* is 5, 10, 20, 30, 40 or 50 mCGs.

In some embodiments, the results of the systems and/or methods disclosed herein are used as an input to generate a report. The report may be in a paper or electronic format. For example, information on, and/or information derived from, the partitioning of nucleic acid molecules, as determined by the methods or systems disclosed herein, can be displayed in such a report. The methods or systems disclosed herein may further comprise a step of communicating the report to a third party, such as the subject from whom the sample derived or a health care practitioner.

The various steps of the methods disclosed herein, or the steps carried out by the systems disclosed herein, may be carried out at the same time or different times, and/or in the same geographical location or different geographical locations, e.g. countries. The various steps of the methods disclosed herein can be performed by the same person or different people.

### BRIEF DESCRIPTION OF THE DRAWINGS

The accompanying drawings, which are incorporated in and constitute a part of this specification, illustrate certain embodiments, and together with the written description, serve to explain certain principles of the methods, computer readable media, and systems disclosed herein. The description provided herein is better understood when read in conjunction with the accompanying drawings which are included by way of example and not by way of limitation. It will be understood that like reference numerals identify like components throughout the drawings, unless the context indicates otherwise. It will also be understood that some or all of the figures may be schematic representations for purposes of illustration and do not necessarily depict the actual relative sizes or locations of the elements shown.
**FIG. 1A** and **FIG. 1B** are schematic diagrams of a fully methylated (FIG. 1A) and hemi-methylated (FIG. 1B) CpG dyad in a double-stranded DNA.
**FIG. 2** is a flow chart representation of a method for assessing the partitioning of a sample of polynucleotides according to an embodiment of the disclosure.
**FIG. 3** is a flow chart representation of a method for assessing the partitioning of a sample of polynucleotides according to an embodiment of the disclosure.
**FIG. 4** is a flow chart representation of a method for assessing the partitioning of a sample of polynucleotides according to an embodiment of the disclosure.
**FIG. 5** is a schematic representation of epigenetic-control nucleic acid molecules suitable for use with some embodiments of the disclosure.
**FIG. 6** is a schematic representation of epigenetic-control nucleic acid molecules suitable for use with some embodiments of the disclosure.
**FIG. 7** is a schematic representation of epigenetic-control nucleic acid molecules suitable for use with some embodiments of the disclosure.
**FIG. 8** is a schematic diagram of an example of a system suitable for use with some embodiments of the disclosure.
**FIG. 9A, FIG. 9B** and **FIG. 9C** are graphical representations of epigenetic partition scores of the epigenetic control-nucleic acid molecules belonging to subsets 1, 2, 3, 4, 5, and 6 in hyper partitioned set (FIG. 9A), intermediate partitioned set (FIG. 9B) and hypo partitioned set (FIG. 9C).
**FIG. 10A and FIG. 10B** are graphical representations of fraction of hypermethylated control molecules of Sample 1 in the hyper partitioned set (FIG. 10A) and in the hypo partitioned set (FIG. 10B).
**FIG. 11A and FIG. 11B** are graphical representations of fraction of hypermethylated control molecules of Sample 2 in the hyper partitioned set (FIG. 11A) and in the hypo partitioned set (FIG. 11B).

### DEFINITIONS

In order for the present disclosure to be more readily understood, certain terms are first defined below. Additional definitions for the following terms and other terms may be set forth through the specification. If a definition of a term set forth below is inconsistent with a definition in an application or patent, the definition set forth in this application should be used to understand the meaning of the term.

As used in this specification and the appended claims, the singular forms "a", "an", and "the" include plural references unless the context clearly dictates otherwise. Thus, for example, a reference to "a method" includes one or more methods, and/or steps of the type described herein and/or which will become apparent to those persons of ordinary skill in the art upon reading this disclosure and so forth.

It is also to be understood that the terminology used herein is for the purpose of describing particular embodiments only, and is not intended to be limiting. Further, unless defined otherwise, all technical and scientific terms used herein have the same meaning as commonly understood by one of ordinary skill in the art to which this disclosure pertains. In describing and claiming the methods, computer readable media, and systems, the following terminology, and grammatical variants thereof, will be used in accordance with the definitions set forth below.

***About***: As used herein, "about" or "approximately" as applied to one or more values or elements of interest, refers to a value or element that is similar to a stated reference value or element. In certain embodiments, the term "about" or "approximately" refers to a range of values or elements that falls within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value or element unless otherwise stated or otherwise evident from the context (except where such number would exceed 100% of a possible value or element).

***Adapter***: As used herein, "adapter" refers to a short nucleic acid (e.g., less than about 500 nucleotides, less than about 100 nucleotides, or less than about 50 nucleotides in length) that is typically at least partially double-stranded and is attached to either or both ends of a given sample nucleic acid molecule. Adapters can include nucleic acid primer binding sites to permit amplification of a nucleic acid molecule flanked by adapters at both ends, and/or a sequencing primer binding site, including primer binding sites for sequencing applications, such as various next-generation sequencing (NGS) applications. Adapters can also include binding sites for capture probes, such as an oligonucleotide attached to a flow cell support or the like. Adapters can also include a nucleic acid tag as described herein. Nucleic acid tags are typically positioned relative to amplification primer and sequencing primer binding sites, such that a nucleic acid tag is included in amplicons and sequence reads of a given nucleic acid molecule. Adapters of the same or different sequences can be linked to the respective ends of a nucleic acid molecule. In some embodiments, adapters of the same sequence is linked to the respective ends of the nucleic acid molecule except that the nucleic acid tag differs. In some embodiments, the adapter is a Y-shaped adapter in which one end is blunt ended or tailed as described herein, for joining to a nucleic acid molecule, which is also blunt ended or tailed with one or more complementary nucleotides. In still other example embodiments, an adapter is a bell-shaped adapter that includes a blunt or tailed end for joining to a nucleic acid molecule to be analyzed. Other examples of adapters include T-tailed and C-tailed adapters.

***Amplify***: As used herein, "amplify" or "amplification" in the context of nucleic acids refers to the production of multiple copies of a polynucleotide, or a portion of the polynucleotide, typically starting from a small amount of the polynucleotide (e.g., a single polynucleotide molecule), where the amplification products or amplicons are generally detectable. Amplification of polynucleotides encompasses a variety of chemical and enzymatic processes. Amplification includes but is not limited to polymerase chain reaction (PCR).

***Barcode*:** As used herein, "barcode" or "molecular barcode" in the context of nucleic acids refers to a nucleic acid molecule comprising a sequence that can serve as a molecular identifier. For example, individual "barcode" sequences are typically added to the DNA fragment during next-generation sequencing (NGS) library preparation so that the sequencing read can be identified and sorted before the final data analysis.

***Cancer Type*:** As used herein, "cancer type" refers to a type or subtype of cancer defined, e.g., by histopathology. Cancer type can be defined by any conventional criterion, such as on the basis of occurrence in a given tissue (e.g., blood cancers, central nervous system (CNS), brain cancers, lung cancers (small cell and non-small cell), skin cancers, nose cancers, throat cancers, liver cancers, bone cancers, lymphomas, pancreatic cancers, bowel cancers, rectal cancers, thyroid cancers, bladder cancers, kidney cancers, mouth cancers, stomach cancers, breast cancers, prostate cancers, ovarian cancers, lung cancers, intestinal cancers, soft tissue cancers, neuroendocrine cancers, gastroesophageal cancers, head and neck cancers, gynecological cancers, colorectal cancers, urothelial cancers, solid state cancers, heterogeneous cancers, homogenous cancers), unknown primary origin and the like, and/or of the same cell lineage (e.g., carcinoma, sarcoma, lymphoma, cholangiocarcinoma, leukemia, mesothelioma, melanoma, or glioblastoma) and/or cancers exhibiting cancer markers, such as, but not limited to, Her2, CA15-3, CA19-9, CA-125, CEA, AFP, PSA, HCG, hormone receptor and NMP-22. Cancers can also be classified by stage (e.g., stage 1, 2, 3, or 4) and whether of primary or secondary origin.

***Cell-Free Nucleic Acid***: As used herein, "cell-free nucleic acid" refers to nucleic acids not contained within or otherwise bound to a cell or, in some embodiments, nucleic acids remaining in a sample following the removal of intact cells. Cell-free nucleic acids can include, for example, all non-encapsulated nucleic acids sourced from a bodily fluid (e.g., blood, plasma, serum, urine, cerebrospinal fluid (CSF), etc.) from a subject. Cell-free nucleic acids include DNA (cfDNA), RNA (cfRNA), and hybrids thereof, including genomic DNA, mitochondrial DNA, circulating DNA, siRNA, miRNA, circulating RNA (cRNA), tRNA, rRNA, small nucleolar RNA (snoRNA), Piwi-interacting RNA (piRNA), long non-coding RNA (long ncRNA), and/or fragments of any of these. Cell-free nucleic acids can be double-stranded, single-stranded, or a hybrid thereof. A cell-free nucleic acid can be released into bodily fluid through secretion or cell death processes, e.g., cellular necrosis, apoptosis, or the like. Some cell-free nucleic acids are released into bodily fluid from cancer cells, e.g., circulating tumor DNA (ctDNA). Others are released from healthy cells. CtDNA can be non-encapsulated tumor-derived fragmented DNA. A cell-free nucleic acid can have one or more epigenetic modifications, for example, a cell-free nucleic acid can be acetylated, 5-methylated, and/or hydroxy methylated.

***Cellular Nucleic Acids***: As used herein, "cellular nucleic acids" means nucleic acids that are disposed within one or more cells from which the nucleic acids have originated, at least at the point a sample is taken or collected from a subject, even if those nucleic acids are subsequently removed (e.g., via cell lysis) as part of a given analytical process.

***Coverage*:** As used herein, the terms "coverage", "total molecule count" or "total allele count" are used interchangeably. They refer to the total number of DNA molecules at a particular genomic position in a given sample.

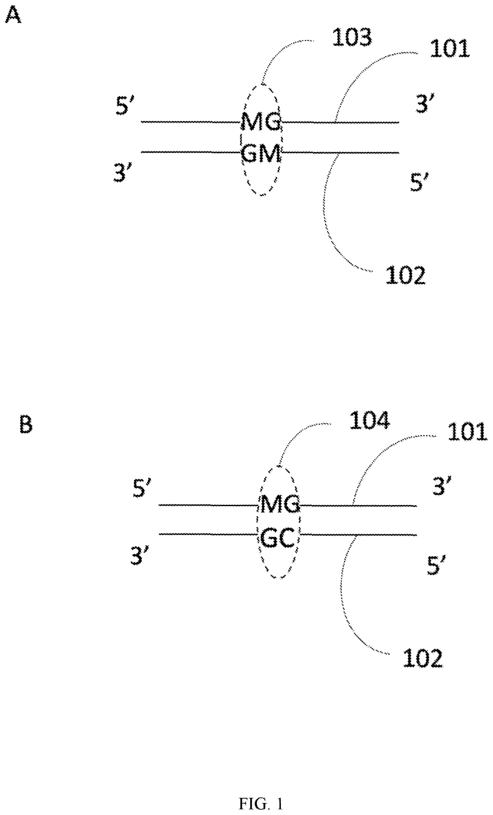

***CpG dyad*:** As used herein, the term "CpG dyad" refers to the dinucleotide CpG (cytosine-phosphate-guanine (i.e., a cytosine followed by a guanine in a 5' → 3' direction of the nucleic acid sequence)) dinucleotide on the sense strand and its complementary CpG on the antisense strand of a double-stranded DNA molecule (shown in FIG. 1).

***Deoxyribonucleic Acid*** or ***Ribonucleic Acid***: As used herein, "deoxyribonucleic acid" or "DNA" refers to a natural or modified nucleotide which has a hydrogen group at the 2'-position of the sugar moiety. DNA typically includes a chain of nucleotides comprising four types of nucleotide bases; adenine (A), thymine (T), cytosine (C), and guanine (G). As used herein, "ribonucleic acid" or "RNA" refers to a natural or modified nucleotide which has a hydroxyl group at the 2'-position of the sugar moiety. RNA typically includes a chain of nucleotides comprising four types of nucleotide bases; A, uracil (U), G, and C. As used herein, the term "nucleotide" refers to a natural nucleotide or a modified nucleotide. Certain pairs of nucleotides specifically bind to one another in a complementary fashion (called complementary base pairing). In DNA, adenine (A) pairs with thymine (T) and cytosine (C) pairs with guanine (G). In RNA, adenine (A) pairs with uracil (U) and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "sequence information," "nucleic acid sequence," "nucleotide sequence", "genomic sequence," "genetic sequence," or "fragment sequence," or "nucleic acid sequencing read" denotes any information or data that is indicative of the order and identity of the nucleotide bases (e.g., adenine, guanine, cytosine, and thymine or uracil) in a molecule (e.g., a whole genome, whole transcriptome, exome, oligonucleotide, polynucleotide, or fragment) of a nucleic acid such as DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, and electronic signature-based systems.

***Endogenous control molecules*:** As used herein, "endogenous control molecules" refer to nucleic acid molecules in the sample of polynucleotides that correspond to at least one human genomic region with a non-variable epigenetic state. In some embodiments, the endogenous control molecules could be consistently highly or lowly methylated across tissues, subjects and cancers. In some embodiments, the endogenous control molecules that correspond to human genomic regions with consistently highly methylated regions can be referred as "hypermethylated control molecules". In some embodiments, the endogenous control molecules that correspond to human genomic regions with consistently lowly methylated regions can be referred as "hypomethylated control molecules".

***Epigenetic-control nucleic acid molecules*:** As used herein, "epigenetic-control nucleic acid molecules" refer to a set of nucleic acid molecules that are added to a sample of polynucleotides to evaluate the partitioning of the sample based on epigenetic modification. For example, the epigenetic modification can be DNA methylation and the epigenetic-control nucleic acid molecules can have different/distinguishable levels of methylation. In some embodiments, epigenetic-control nucleic acid molecules comprise an epigenetic modification region and optionally, an identifier region. In some embodiments, epigenetic-control nucleic acid molecules comprise an epigenetic modification region and an identifier region. The epigenetic-control nucleic acid molecules can be synthetic oligonucleotides. In some embodiments, the epigenetic-control nucleic acid molecules can have a non-naturally occurring nucleic acid sequence. In some embodiments, the epigenetic-control nucleic acid molecules can have a naturally occurring nucleic acid sequence. In some embodiments, epigenetic-control nucleic acid molecules can have a nucleic acid sequence corresponding to a non-human genome. As non-limiting examples, these molecules may either have (i) a sequence corresponding to regions of lambda phage DNA or human genome, (ii) a non-naturally occurring sequence, and/or (iii) a combination of (i) and (ii).

***Epigenetic modification*:** As used herein, "epigenetic modification" refers to a modification of the base of the nucleotide(s) in the nucleic acid molecules. The modification can be a chemical modification of the nucleotides' base. In some cases, the modification can be methylation of the nucleotides' base. For example, the modification can be methylation of cytosine, resulting in 5-methylcytosine.

***Epigenetic modification region*:** As used herein, "epigenetic modification region" refers to a region of the epigenetic-control nucleic acid molecule that represents the level/degree of epigenetic modification of the epigenetic-control nucleic acid molecule. In some embodiments, the epigenetic modification region can comprise nucleotides with epigenetic modification. In some embodiments, the epigenetic modification is DNA methylation. In those embodiments, the epigenetic modification region of the epigenetic-control nucleic acid molecules can have nucleotides that are methylated. The number of methylated nucleotides in the epigenetic modification region can vary among the epigenetic-control nucleic acid molecules. In some embodiments, the epigenetic-control nucleic acid molecules can have 0, 1, 2, 3, 4, 5, 6, 7, 8, 9, at least 10, at least 15, at least 20, at least 30, at least 40 or at least 50 methylated nucleotides in the epigenetic modification region. The epigenetic-control nucleic acid molecules can be grouped into subsets based on the number of nucleotides with epigenetic modification in the epigenetic modification region. The epigenetic modification region among the different subsets can be of same length, for example around 160 bp. The length of the epigenetic modification region between the subsets can be different. For example, epigenetic-control nucleic acid molecules can be grouped into three subsets (subset A, B and C) based on the number of methylated nucleotides in the epigenetic modification region. Subsets A, B and C can have epigenetic-control nucleic acid molecules with 5, 10 and 15 methylated nucleotides respectively in the epigenetic modification region and the length of the epigenetic modification region in subsets A, B and C can be same (e.g. 160 bp) or can be different - 100 bp, 150 bp and 200 bp for subsets A, B and C respectively.

***Epigenetic partition score*:** As used herein, "epigenetic partition score" refers to a numerical value that represents the partitioning of nucleic acid molecules belonging to a particular epigenetic state in a given partitioned set. In some embodiments, the epigenetic partition score of the nucleic acid molecules belonging to an epigenetic state is determined for each partitioned set. For example, the epigenetic partition score of the epigenetic-control nucleic acid molecules and/or endogenous control molecules belonging to a particular epigenetic state can be determined. The epigenetic partition score can be a measure of the number (or statistically estimated number) of nucleic acid molecules belonging to a particular epigenetic state. The epigenetic partition score can be in terms of fraction or percentage. The epigenetic partition score can be a measure of the ratio of the number of epigenetic-control nucleic acid molecules and/or endogenous control molecules belonging to a particular epigenetic state that's partitioned in at least one partitioned set to the number of epigenetic-control nucleic acid molecules and/or endogenous control molecules belonging to that epigenetic state present in the other remaining partitioned set(s). In some embodiments, the epigenetic partition score can be a fraction or percentage of the number of epigenetic-control nucleic acid molecules and/or endogenous control molecules belonging to a particular epigenetic state partitioned in at least one partitioned set to the total number of epigenetic-control nucleic acid molecules and/or endogenous control molecules belonging to that epigenetic state in all the partitioned sets. In some embodiments, the epigenetic partition score is determined for each epigenetic state of the epigenetic-control nucleic acid molecules and/or endogenous control molecules in each of the partitioned sets. In some embodiments, the epigenetic partition score is determined for the epigenetic-control nucleic acid molecules and/or endogenous control molecules with one or more particular epigenetic states in one or more partitioned sets. In some embodiments, the epigenetic partition score is determined for the epigenetic-control nucleic acid molecules and/or endogenous control molecules with a particular epigenetic state in a particular partitioned set.

In some embodiments, the epigenetic partition score can be directed to the efficiency with which the molecules with no CG ('zero' CG) partitioned to hyper partitioned set. This score can be referred to as *0 CG score.* In some embodiments, the *0 CG score* can be expressed in terms of fraction or percentage of molecules with no CG in the hyper partitioned set. In some embodiments, the epigenetic partition score can be a measure to the fraction of epigenetic-control nucleic acid molecules and/or fraction of hypermethylated control molecules with at least one of the following:
(i) 1 methyl CGs (epigenetic partition score can be referred as *1 CG score*),
(ii) 2 methyl CGs (epigenetic partition score can be referred as *2 CG score*),
(iii) 3 methyl CGs (epigenetic partition score can be referred as *3 CG score*),
(iv) 4 methyl CGs (epigenetic partition score can be referred as *4 CG score*) and
(v) 5 methyl CGs (epigenetic partition score can be referred as *5 CG score*) in the hyper partitioned set.

In some embodiments, the epigenetic partition score can be directed to the efficiency of the hypomethylated control molecules or hypomethylated epigenetic-control nucleic acid molecules partitioned to a hypermethylated partitioned set. This score can be referred to as *hypo score.* In some embodiments, the *hypo score* can be expressed in terms of fraction or percentage of the hypomethylated control molecules or hypomethylated epigenetic-control nucleic acid molecules in the hyper methylated partitioned set. In some embodiments, the epigenetic partition score can be a measure of the number of the methylated CGs required for less than a specified value, e.g. 5%, of hypermethylated control molecules and/or hypermethylated epigenetic-control nucleic acid molecules in the hypomethylated partitioned set. In the example of using 5% of hypermethylated control molecules and/or hypermethylated epigenetic-control nucleic acid molecules in the hypomethylated partitioned set - i.e., the epigenetic partition score is a measure of the number of the methylated CGs required for less than 5% of hypermethylated control molecules and/or hypermethylated epigenetic-control nucleic acid molecules in the hypomethylated partitioned set, this score can for the sake of convenience, be referred to as *methyl-5.* In some embodiments, the epigenetic partition score can be a measure of the number of the methylated CGs required for at least a specified value, e.g. 50%, of hypermethylated control molecules and/or hypermethylated epigenetic-control nucleic acid molecules in the hypermethylated partitioned set. In the example of using 50% of hypermethylated control molecules and/or hypermethylated epigenetic-control nucleic acid molecules in the hypermethylated partitioned set - i.e., the epigenetic partition score is a measure of the number of the methylated CGs required for at least 50% of hypermethylated control molecules and/or hypermethylated epigenetic-control nucleic acid molecules in the hypermethylated partitioned set, this score can be referred to as *methyl-half.* A wide range of different values from 0% to 100% (not just 5% and 50%) may be used in different embodiments, and corresponding different names of convenience referring to the specified value may be employed.

For example, three subsets (subsets A, B and C) of epigenetic-control nucleic acid molecules are used and each subset differs in the number of methylated nucleotides. The epigenetic-control nucleic acid molecules in these three subsets can be partitioned into three partitioned sets - P1, P2 and P3, based on their binding affinity to methyl binding protein. For each subset, the epigenetic partition score is determined for each of the partitioned sets (P1, P2 and P3) - i.e. epigenetic-control nucleic acid molecules belonging to subset A will have three epigenetic partition scores - one for each of the three partitioned sets, P1, P2 and P3. Likewise, each of subsets B and C will have three epigenetic partition scores - one for each of the three partitioned sets P1, P2 and P3. The epigenetic partition score can be determined for the endogenous control molecules as well.

In another embodiment, three subsets (subsets A, B and C) of epigenetic-control nucleic acid molecules are used and each subset differs in the number of methylated nucleotides (i.e. each subset has a different epigenetic state). The epigenetic-control nucleic acid molecules in these three subsets can be partitioned into three partitioned sets - P1, P2 and P3, based on their binding affinity to methyl binding protein. In this embodiment, the epigenetic score is determined only for Subset A molecules in P1 partitioned set. This epigenetic score can be a measure of the fraction or percentage of Subset A molecules in P1 partitioned set to the total number of Subset A molecules (in P1, P2 and P3 partitioned sets).

***Epigenetic partition cut-off*:** As used herein, "epigenetic partition cut-off' refers to a predetermined cut-off value or cut-off range used to evaluate the partitioning of the nucleic acid molecules belonging to a particular epigenetic state in a particular partitioned set. In some embodiments, the epigenetic partition cut-off is determined from analyzing in-house sample dataset. Each partitioned set can have an epigenetic partition cut-off for the nucleic acid molecules belonging to an epigenetic state. If one or more epigenetic partition scores of epigenetic-control nucleic acid molecules belonging to one or more epigenetic states (used for evaluating the partitioning) is within their corresponding epigenetic partition cut-offs, then the partitioning method is a success. Otherwise, the partitioning method is a failure. The epigenetic partition cut-offs differ with the epigenetic state of the nucleic acid molecules and partitioned set, i.e., each epigenetic state will have its own epigenetic partition cut-off and every partitioned set has a separate epigenetic partition cut-off for that epigenetic state. The cut-off can be in terms of percentage or fraction and the cut-off can be a cut-off range instead of a particular cut-off value. For example, the epigenetic partition cut-offs for the epigenetic-control nucleic acid molecules belonging to a particular epigenetic state can be between 70% - 79%, between 10% - 15% and less than 5% for partitioned sets P1, P2 and P3 respectively. If the epigenetic partition scores of the epigenetic-control nucleic acid molecules belonging to that epigenetic state is within the corresponding epigenetic partition cut-offs, then partitioning method is a success.

***Epigenetic state*:** As used herein, "epigenetic state" refers to the level/degree of epigenetic modification of the nucleic acid molecules. For example, if the epigenetic modification is DNA methylation (or hydroxy methylation), then the epigenetic state can refer to the presence or absence of methylation on a DNA base (e.g. cytosine) or to the degree of methylation in a nucleic acid sequence (e.g., highly methylated, low methylated, intermediately methylated or unmethylated nucleic acid molecules). The epigenetic state can also refer to the number of nucleotides with epigenetic modification. For example, if the epigenetic modification is DNA methylation, then an epigenetic state can refer to the number of methylated nucleotides of the nucleic acid molecules.

***Epigenetic state barcode*:** As used herein, "epigenetic state barcode" refers to a nucleic acid sequence that is used to identify the epigenetic state of the epigenetic-control nucleic acid molecule. Identification can be achieved by having a predetermined correlation between a particular epigenetic state barcode or barcodes and the epigenetic state of the epigenetic-control nucleic acid molecule. It can refer to the number of nucleotides with epigenetic modification in the epigenetic modification region of the epigenetic-control nucleic acid molecule. In some embodiments, the identifier region of the epigenetic-control nucleic acid molecule comprises at least one epigenetic state barcode. For example, if the epigenetic modification is DNA methylation and a subset of the epigenetic-control nucleic acid molecules have 5 methylated nucleotides, then all the epigenetic-control nucleic acid molecules within that subset with have the same epigenetic state barcode. In some embodiments, the epigenetic state barcode can be used to identify the level/degree of epigenetic modification of the epigenetic modification region of the epigenetic-control nucleic acid molecule. The epigenetic-control nucleic acid molecules can be grouped into subsets based on the number of cytosine or CpG nucleotides in the epigenetic modification region. In some embodiments, within each subset, the level of methylation can vary (for e.g., highly methylated, intermediately methylated, low methylated or unmethylated) and each level of methylation can have a separate epigenetic state barcode. For example, within subset A, all the epigenetic-control nucleic acid molecules that are low methylated will have an epigenetic state barcode- e.g. ESB1 and all the epigenetic-control nucleic molecules that are highly methylated will have another epigenetic state barcode - e.g. ESB3. In this example, the epigenetic state barcode is used to identify the level/degree of methylation.

***Human genomic region with non-variable epigenetic state*:** As used herein, "human genomic region with non-variable epigenetic state" refers to a region in the human genome with a particular epigenetic state and the epigenetic state of that region does not vary/change often and always remains the same or remains consistent with different subjects and/or different types of disease/disease stages. For example, the human genomic region with non-variable epigenetic state can be predominantly methylated or predominantly unmethylated.

***Identifier region*:** As used herein, "identifier region" refers to a region of the epigenetic-control nucleic acid molecule that is used in distinguishing an epigenetic-control nucleic acid molecule from the other epigenetic-control nucleic acid molecules. The identifier region can have molecular barcodes and/or epigenetic state barcodes. The identifier region can be present in one or both the sides of the epigenetic modification region. The molecular barcode serves as the identifier of an epigenetic-control nucleic acid molecule whereas the epigenetic state barcode serves as the identifier of the epigenetic state of the epigenetic-control nucleic acid molecule. The identifier region can have an additional region facilitating binding of one or more primers (primer binding sites).

***Mutant Allele Count***: As used herein, the term "mutant allele count" refers to the number of DNA molecules harboring the mutant allele at a particular genomic locus

***Mutant Allele Fraction***: As used herein, "mutant allele fraction", "mutation dose," or "MAF" refers to the fraction of nucleic acid molecules harboring an allelic alteration or mutation at a given genomic position/ locus in a given sample. MAF is generally expressed as a fraction or a percentage. For example, an MAF of a somatic variant may be less than 0.15.

***Mutation*:** As used herein, "mutation" refers to a variation from a known reference sequence and includes mutations such as, for example, single nucleotide variants (SNVs), and insertions or deletions (indels). A mutation can be a germline or somatic mutation. In some embodiments, a reference sequence for purposes of comparison is a wildtype genomic sequence of the species of the subject providing a test sample, typically the human genome.

***Mutation Caller*:** As used herein, "mutation caller" means an algorithm (typically, embodied in software or otherwise computer implemented) that is used to identify mutations in test sample data (e.g., sequence information obtained from a subject).

***Neoplasm*:** As used herein, the terms "neoplasm" and "tumor" are used interchangeably. They refer to abnormal growth of cells in a subject. A neoplasm or tumor can be benign, potentially malignant, or malignant. A malignant tumor is a referred to as a cancer or a cancerous tumor.

***Next Generation Sequencing*:** As used herein, "next generation sequencing" or "NGS" refers to sequencing technologies having increased throughput as compared to traditional Sanger- and capillary electrophoresis-based approaches, for example, with the ability to generate hundreds of thousands of relatively small sequence reads at a time. Some examples of next generation sequencing techniques include, but are not limited to, sequencing by synthesis, sequencing by ligation, and sequencing by hybridization. In some embodiments, next generation sequencing includes the use of instruments capable of sequencing single molecules.

***Nucleic Acid Tag***: As used herein, "nucleic acid tag" refers to a short nucleic acid (e.g., less than about 500 nucleotides, about 100 nucleotides, about 50 nucleotides, or about 10 nucleotides in length), used to distinguish nucleic acids from different samples (e.g., representing a sample index), or different nucleic acid molecules in the same sample (e.g., representing a molecular barcode), of different types, or which have undergone different processing. The nucleic acid tag comprises a predetermined, fixed, non-random, random or semi-random oligonucleotide sequence. Such nucleic acid tags may be used to label different nucleic acid molecules or different nucleic acid samples or sub-samples. Nucleic acid tags can be single-stranded, double-stranded, or at least partially double-stranded. Nucleic acid tags optionally have the same length or varied lengths. Nucleic acid tags can also include double-stranded molecules having one or more blunt-ends, include 5' or 3' single-stranded regions (e.g., an overhang), and/or include one or more other single-stranded regions at other locations within a given molecule. Nucleic acid tags can be attached to one end or to both ends of the other nucleic acids (e.g., sample nucleic acids to be amplified and/or sequenced). Nucleic acid tags can be decoded to reveal information such as the sample of origin, form, or processing of a given nucleic acid. For example, nucleic acid tags can also be used to enable pooling and/or parallel processing of multiple samples comprising nucleic acids bearing different molecular barcodes and/or sample indexes in which the nucleic acids are subsequently being deconvolved by detecting (e.g., reading) the nucleic acid tags. Nucleic acid tags can also be referred to as identifiers (e.g. molecular identifier, sample identifier). Additionally, or alternatively, nucleic acid tags can be used as molecular identifiers (e.g., to distinguish between different molecules or amplicons of different parent molecules in the same sample or sub-sample). This includes, for example, uniquely tagging different nucleic acid molecules in a given sample, or non-uniquely tagging such molecules. In the case of non-unique tagging applications, a limited number of tags (i.e., molecular barcodes) may be used to tag each nucleic acid molecule such that different molecules can be distinguished based on their endogenous sequence information (for example, start and/or stop positions where they map to a selected reference genome, a sub-sequence of one or both ends of a sequence, and/or length of a sequence) in combination with at least one molecular barcode. Typically, a sufficient number of different molecular barcodes are used such that there is a low probability (e.g., less than about a 10%, less than about a 5%, less than about a 1%, or less than about a 0.1% chance) that any two molecules may have the same endogenous sequence information (e.g., start and/or stop positions, subsequences of one or both ends of a sequence, and/or lengths) and also have the same molecular barcode.

***Partitioning*:** As used herein, the "partitioning" and "epigenetic partitioning" are used interchangeably. It refers to separating or fractionating the nucleic acid molecules based on a characteristic (e.g. the level/degree of epigenetic modification) of the nucleic acid molecules. The partitioning can be physical partitioning of molecules. Partitioning can involve separating the nucleic acid molecules into groups or sets based on the level of epigenetic modification (i.e. epigenetic state). For example, the nucleic acid molecules can be partitioned based on the level of methylation of the nucleic acid molecules. In some embodiments, the methods and systems used for partitioning may be found in PCT Patent Application No. PCT/US2017/068329 which is incorporated by reference in its entirety.

***Partitioned set*:** As used herein, "partitioned set" refers to a set of nucleic acid molecules partitioned into a set/group based on the differential binding affinity of the nucleic acid molecules to a binding agent. The binding agent binds preferentially to the nucleic acid molecules comprising nucleotides with epigenetic modification. For example, if the epigenetic modification is methylation, the binding agent can be a methyl binding domain (MBD) protein. In some embodiments, a partitioned set can comprise nucleic acid molecules belonging to a particular level/degree of epigenetic modification (i.e., epigenetic state). For example, the nucleic acid molecules can be partitioned into three sets: one set for highly methylated nucleic acid molecules (or hypermethylated nucleic acid molecules), which can be referred as hypermethylated partitioned set or hyper partitioned set, another set for low methylated nucleic acid molecules (or hypomethylated nucleic acid molecules), which can be referred as hypomethylated partitioned set or hypo partitioned set and a third set for intermediately methylated nucleic acid molecules, which can be referred as intermediately methylated partitioned set or intermediate partitioned set. In another example, the nucleic acid molecules can be partitioned based on the number of nucleotides with epigenetic modification - one partitioned set can have nucleic acid molecules with nine methylated nucleotides and another partitioned set can have unmethylated nucleic acid molecules (zero methylated nucleotides).

***Polynucleotide*:** As used herein, "polynucleotide", "nucleic acid", "nucleic acid molecule", or "oligonucleotide" refers to a linear polymer of nucleosides (including deoxyribonucleosides, ribonucleosides, or analogs thereof) joined by inter-nucleosidic linkages. Typically, a polynucleotide comprises at least three nucleosides. Oligonucleotides often range in size from a few monomeric units, e.g., 3-4, to hundreds of monomeric units. Whenever a polynucleotide is represented by a sequence of letters, such as "ATGCCTG", it will be understood that the nucleotides are in 5' → 3' order from left to right and that in the case of DNA, "A" denotes deoxyadenosine, "C" denotes deoxycytidine, "G" denotes deoxyguanosine, and "T" denotes deoxythymidine, unless otherwise noted. The letters A, C, G, and T may be used to refer to the bases themselves, to nucleosides, or to nucleotides comprising the bases, as is standard in the art.

***Reference Sequence*:** As used herein, "reference sequence" refers to a known sequence used for purposes of comparison with experimentally determined sequences. For example, a known sequence can be an entire genome, a chromosome, or any segment thereof. A reference typically includes at least about 20, at least about 50, at least about 100, at least about 200, at least about 250, at least about 300, at least about 350, at least about 400, at least about 450, at least about 500, at least about 1000, or more than 1000 nucleotides. A reference sequence can align with a single contiguous sequence of a genome or chromosome or can include non-contiguous segments that align with different regions of a genome or chromosome. Examples of reference sequences include, for example, human genomes, such as, hG19 and hG38.

***Sample*:** As used herein, "sample" means anything capable of being analyzed by the methods and/or systems disclosed herein.

***Sequencing*:** As used herein, "sequencing" refers to any of a number of technologies used to determine the sequence (e.g., the identity and order of monomer units) of a biomolecule, e.g., a nucleic acid such as DNA or RNA. Examples of sequencing methods include, but are not limited to, targeted sequencing, single molecule real-time sequencing, exon or exome sequencing, intron sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and a combination thereof. In some embodiments, sequencing can be performed by a gene analyzer such as, for example, gene analyzers commercially available from Illumina, Inc., Pacific Biosciences, Inc., or Applied Biosystems/Thermo Fisher Scientific, among many others.

***Sequence Information*:** As used herein, "sequence information" in the context of a nucleic acid polymer means the order and identity of monomer units (e.g., nucleotides, etc.) in that polymer.

***Somatic Mutation*:** As used herein, the terms "somatic mutation" or "somatic variation" are used interchangeably. They refer to a mutation in the genome that occurs after conception. Somatic mutations can occur in any cell of the body except germ cells and accordingly, are not passed on to progeny.

***Spiked-in sample*:** As used herein, "spiked-in sample" is a sample in which epigenetic-control nucleic acid molecules are added to the sample of polynucleotides from a subject.

***Subject*:** As used herein, "subject" refers to an animal, such as a mammalian species (e.g., human) or avian (e.g., bird) species, or other organism, such as a plant. More specifically, a subject can be a vertebrate, e.g., a mammal such as a mouse, a primate, a simian or a human. Animals include farm animals (e.g., production cattle, dairy cattle, poultry, horses, pigs, and the like), sport animals, and companion animals (e.g., pets or support animals). A subject can be a healthy individual, an individual that has or is suspected of having a disease or a predisposition to the disease, or an individual in need of therapy or suspected of needing therapy. The terms "individual" or "patient" are intended to be interchangeable with "subject."

For example, a subject can be an individual who has been diagnosed with having a cancer, is going to receive a cancer therapy, and/or has received at least one cancer therapy. The subject can be in remission of a cancer. As another example, the subject can be an individual who is diagnosed of having an autoimmune disease. As another example, the subject can be a female individual who is pregnant or who is planning on getting pregnant, who may have been diagnosed of or suspected of having a disease, e.g., a cancer, an auto-immune disease.

### DETAILED DESCRIPTION

### I. Overview

Genomic/epigenetic partitioning-based methods can allow for multi-analyte, simultaneous signal detection in one assay. However, detected signals of the partitioning-based analyte may have poor resolution and are subject to variable assay conditions that alter signal sensitivity and specificity. It is desirable to increase the sensitivity of liquid biopsy assays while reducing the loss of circulating nucleic acid (original material) or data in the process. It is also desirable to provide for the ability to compare results across different experiments by controlling for assay variability by using one or more controls as described herein. WO2008009365 describes a method for determining the methylation rate of a nucleic acid by the use of a reference. WO2005040399 describes a method for quantifying the presence of methylated nucleic acids in a sample by the use of a control.

The present disclosure provides methods and compositions for calibrating epigenetic partitioning assays. The invention comprises using a set of epigenetic-control nucleic acid molecules with completely resolved genomic/epigenetic features (e.g. discrete number of methylated cytosines in a synthetic oligonucleotide duplex) as a control or reference to increase signal sensitivity and specificity of the sample being analyzed. These molecules can be used to evaluate the partitioning of the nucleic acid molecules in the sample based on an epigenetic modification and also to determine the epigenetic state of nucleic acid molecule(s) in the sample.

Nucleic acids molecules, such as cell-free polynucleotides, can differ based on epigenetic characteristics such as methylation. Nucleic acids can possess different nucleotide sequences, e.g., specific genes or genetic loci. Characteristics can differ in terms of degree. For example, DNA molecules can differ in their extent of epigenetic modification. Extent of modification can refer to a number of modifying events to which a molecule has been subject, such as number of methylation groups (extent of methylation) or other epigenetic changes. For example, methylated DNA may be hypomethylated or hypermethylated. Forms can be characterized by combinations of characteristics, for example, single stranded- unmethylated or double stranded-methylated. Fractionation of molecules based on one or a combination of characteristics can be useful for multidimensional analysis of single molecules. These methods accommodate multiple forms and/or modifications of nucleic acid in a sample, such that sequence information can be obtained for multiple forms. The methods also preserve the identity of the initial multiple forms or modified states through processing and analysis, such that analysis of nucleic base sequences can be combined with epigenetic analysis. Some methods involve separation, tagging and subsequent pooling of different forms or modification states reducing the number of processing steps required to analyze multiple forms present in a sample. Analyzing multiple forms of nucleic acid in samples provides greater information in part because there are more molecules to analyze (which can be significant when very low total amounts of nucleic acid are available) but also because the different forms or modification states can provide different information (for example, a mutation may be present only in RNA), and because different types of information (e.g. genetic and epigenetic) can be correlated with one another, thereby producing greater accuracy, certainty, or resulting in the discovery of new correlations with a medical condition.

A characteristic of nucleic acid molecules may be a modification, which may include various chemical modifications (i.e. epigenetic modifications). Non-limiting examples of chemical modification may include, but are not limited to, covalent DNA modifications, including DNA methylation. In some embodiments, DNA methylation comprises addition of a methyl group to a cytosine at a CpG site (cytosine-phosphate-guanine site (i.e., a cytosine followed by a guanine in a 5' → 3' direction of the nucleic acid sequence)). In some embodiments, DNA methylation comprises addition of a methyl group to adenine, such as in N⁶-methyladenine. In some embodiments, DNA methylation is 5-methylation (modification of the 5th carbon of the 6-carbon ring of cytosine). In some embodiments, 5-methylation comprises addition of a methyl group to the 5C position of the cytosine to create 5-methylcytosine (m5c). In some embodiments, methylation comprises a derivative of m5c. Derivatives of m5c include, but are not limited to, 5-hydroxymethylcytosine (5-hmC), 5-formylcytosine (5-fC), and 5-caryboxylcytosine (5-caC). In some embodiments, DNA methylation is 3C methylation (modification of the 3rd carbon of the 6-carbon ring of cytosine). In some embodiments, 3C methylation comprises addition of a methyl group to the 3C position of the cytosine to generate 3-methylcytosine (3mC). Methylation can also occur at non CpG sites, for example, methylation can occur at a CpA, CpT, or CpC site. DNA methylation can change the activity of methylated DNA region. For example, when DNA in a promoter region is methylated, transcription of the gene may be repressed. DNA methylation is critical for normal development and abnormality in methylation may disrupt epigenetic regulation. The disruption, e.g., repression, in epigenetic regulation may cause diseases, such as cancer. Promoter methylation in DNA may be indicative of cancer.

A CpG dyad is the dinucleotide CpG (cytosine-phosphate-guanine, i.e. a cytosine followed by a guanine in a 5' → 3' direction of the nucleic acid sequence) on the sense strand and its complementary CpG on the antisense strand of a double-stranded DNA molecule. CpG dyads can be either fully methylated or hemi-methylated. FIG. 1 is a schematic diagram of a fully methylated and hemi-methylated CpG dyad in a double-stranded DNA. FIG. 1A shows a fully methylated CpG dyad 103, where the cytosine nucleotide of the CpG dyad on both the strands 101 and 102 is methylated (M - methylcytosine; G - guanine). FIG. 1B shows a hemi-methylated CpG dyad 104, where the cytosine nucleotide of the CpG dyad on one strand 101 is methylated whereas the cytosine nucleotide of the CpG dyad on the complementary strand 102 is unmethylated (C - unmethylated cytosine; G - guanine).

The CpG dinucleotide is underrepresented in the normal human genome, with the majority of CpG dinucleotide sequences being transcriptionally inert (e.g. DNA heterochromatic regions in pericentromeric parts of the chromosome and in repeat elements) and methylated. However, many CpG islands are protected from such methylation especially around transcription start sites (TSS).

Cancer can be indicated by epigenetic variations, such as methylation. Examples of methylation changes in cancer include local gains of DNA methylation in the CpG islands at the TSS of genes involved in normal growth control, DNA repair, cell cycle regulation, and/or cell differentiation. This hypermethylation can be associated with an aberrant loss of transcriptional capacity of involved genes and occurs at least as frequently as point mutations and deletions as a cause of altered gene expression. DNA methylation profiling can be used to detect regions with different extents of methylation ("differentially methylated regions" or "DMRs") of the genome that are altered during development or that are perturbed by disease, for example, cancer or any cancer-associated disease.

Methylation profiling can involve determining methylation patterns across different regions of the genome. For example, after partitioning molecules based on extent of methylation (e.g., relative number of methylated nucleotides per molecule) and sequencing, the sequences of molecules in the different partitions can be mapped to a reference genome. This can show regions of the genome that, compared with other regions, are more highly methylated or are less highly methylated. In this way, genomic regions, in contrast to individual molecules, may differ in their extent of methylation. In addition to methylation, other epigenetic modifications may be similarly profiled.

Nucleic acid molecules in a sample may be fractionated or partitioned based on one or more characteristics. Partitioning nucleic acid molecules in a sample can increase a rare signal. For example, a genetic variation present in hypermethylated DNA but less (or not) present in hypomethylated DNA can be more easily detected by partitioning a sample into hypermethylated and hypomethylated nucleic acid molecules. By analyzing multiple fractions of a sample, a multidimensional analysis of a single molecule can be performed and hence, greater sensitivity can be achieved. Partitioning may include physically partitioning nucleic acid molecules into subsets or groups based on the presence or absence of a genomic characteristic. Fractionation may include physically partitioning nucleic acid molecules into partition groups based on the degree to which a genomic characteristic, such as an epigenetic modification, is present. A sample may be fractionated or partitioned into one or more groups partitions based on a characteristic that is indicative of differential gene expression or a disease state. A sample may be fractionated based on a characteristic, or combination thereof that provides a difference in signal between a normal and diseased state during analysis of nucleic acids, e.g., cell free DNA ("cfDNA"), non-cfDNA, tumor DNA, circulating tumor DNA ("ctDNA") and cell free nucleic acids ("cfNA").

The present disclosure provides methods, compositions and systems for assessing or evaluating the partitioning of nucleic acid molecules and determining the epigenetic state (e.g. methylation state) and the number of epigenetically modified nucleotides (e.g. number of methylated nucleotides) in the nucleic acid molecules. The methods may include partitioning the nucleic acid molecules into different partitioned sets based on one or a plurality of epigenetic modifications, followed by sequencing (alone or together) and analyzing the nucleic acid molecules in each partition. In some embodiments, the partitions of nucleic acids are enriched for specific target genomic regions. In some embodiments, the partitions of nucleic acid molecules are amplified prior to and/or after enriching. In some embodiments, the enrichment may be performed after the partitioned sets have been differentially tagged with molecular barcodes and recombined into a mixture of the differentially tagged partitioned sets. The methods can be used in various applications, such as prognosis, diagnosis and/or for monitoring of a disease. In some embodiments, the disease is cancer.

The partitioning method of nucleic acid molecules can be evaluated by using epigenetic-control nucleic acid molecules. Epigenetic-control nucleic acid molecules are synthetic nucleic acid molecules that can have epigenetically modified nucleotides. In some embodiments, epigenetic-control nucleic acid molecules can comprise nucleic acid molecules with different epigenetic states. Epigenetic state can refer to the level/degree of epigenetic modification of the nucleic acid molecules. For example, if the epigenetic modification is DNA methylation, then the epigenetic state can refer to highly methylated, low methylated or intermediately methylated nucleic acid molecules. The epigenetic state can also refer to the number of nucleotides with epigenetic modification. For example, if the epigenetic modification is DNA methylation, then an epigenetic state can refer to the number of methylated nucleotides of the nucleic acid molecules. Epigenetic modification can be any modification of the base of the nucleotide(s) without changing the sequence and/or the base pairing specificity of the nucleic acid molecule. The modification can be a chemical modification of the nucleotides' base. In some cases, the modification can be methylation of the nucleotides' base. For example, the modification can be methylation of cytosine, resulting in 5-methylcytosine.

In some embodiments, the epigenetic-control nucleic acid molecules are synthetic molecules, the sequence of the epigenetic-control nucleic acid molecules and the position and number of epigenetically modified nucleotides in the epigenetic-control nucleic acid molecules are already known prior to analysis. Hence, by adding the epigenetic-control nucleic acid molecules to the sample of polynucleotides and by tracking the epigenetic-control nucleic acid molecules in the partitioned sets, one can analyze the effectiveness of the partitioning of the epigenetic-control nucleic acid molecules.

Accordingly, in one aspect, the present disclosure provides a method for evaluating the partitioning of nucleic acid molecules in a sample of polynucleotides based on epigenetic state, comprising: (a) adding a set of epigenetic-control nucleic acid molecules to the nucleic acid molecules in the sample of polynucleotides, whereby producing a spiked-in sample; (b) partitioning nucleic acid molecules at least a subset of the spiked-in sample into a plurality of partitioned sets; (c) enriching a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides; (d) sequencing the set of enriched molecules to produce a set of sequencing reads; (e) analyzing at least a subset of the set of sequencing reads to generate one or more epigenetic partition scores of the epigenetic-control nucleic acid molecules; and (f) comparing the one or more epigenetic partition scores with one or more of epigenetic partition cut-offs. In these embodiments, the partitioning of the nucleic acid molecules of the sample and the epigenetic-control nucleic acid molecules necessarily take place concurrently. In some embodiments, the analyzing step comprises estimating the number/fraction of the epigenetic-control nucleic acid molecules at a given epigenetic state in at least one of the partitioned sets.

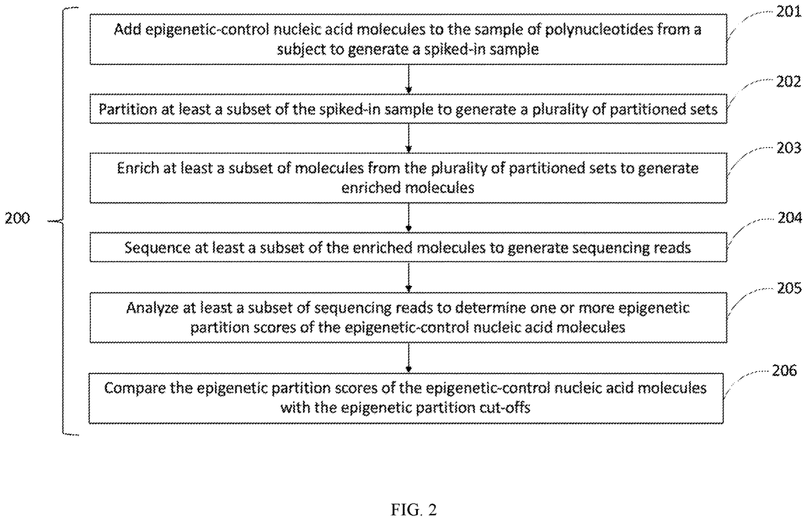

FIG. 2 illustrates an example embodiment of a method 200 for evaluating partitioning of nucleic acid molecules in a sample of polynucleotides based on epigenetic state. In 201, the epigenetic-control nucleic acid molecules are added to the sample, whose partitioning is to be evaluated, to generate a spiked-in sample.

In some embodiments, the epigenetic-control nucleic acid molecules may comprise one or more subsets of nucleic acid molecules with different levels of epigenetic state (i.e., different number of epigenetically modified nucleotides). In some embodiments, epigenetic-control nucleic acid molecules can comprise nucleic acid molecules with different sequences and/or different lengths. In other embodiments, the epigenetic-control nucleic acid molecules may comprise nucleic acid molecules with identical sequences or of identical length.

In 202, the nucleic acid molecules of at least a subset of the spiked-in sample, which comprises both epigenetic-control nucleic acid molecules and nucleic acid molecules from the sample of polynucleotides, are partitioned or fractionated into a plurality of partitioned sets based on the epigenetic state of the molecules. Partitioning can be based on the presence or absence of an epigenetic modification and/or can be based on the degree of epigenetic modification. Examples of epigenetic modification include, but not limited to the presence or absence of methylation, level of methylation and type of methylation (5' cytosine). In some embodiments, epigenetic modification can be DNA methylation. In those embodiments, molecules of the spiked-in sample are partitioned based on the different levels of methylation (different number of methylated nucleotides). In some embodiments, the spiked-in sample can be partitioned into two or more partitioned sets (e.g. at least 3, 4, 5, 6, or 7 partitioned sets). In some embodiments, partitioning is based on the differential binding affinity of the nucleic acid molecules to a binding agent. Examples of binding agents include, but not limited to methyl binding domain (MBDs) and methyl binding proteins (MBPs). Examples of MBPs contemplated herein include, but are not limited to:
(a) MeCP2 is a protein preferentially binding to 5-methyl-cytosine over unmodified cytosine;
(b) RPL26, PRP8 and the DNA mismatch repair protein MHS6 preferentially bind to 5-hydroxymethyl-cytosine over unmodified cytosine;
(c) FOXK1, FOXK2, FOXP1, FOXP4 AND FOXI3 preferably bind to 5-formyl-cytosine over unmodified cytosine (Iurlaro et al., Genome Biol. 14, R119 (2013)).; and
(d) Antibodies specific to one or more methylated nucleotide bases.

Although for some affinity agents and modifications, binding to the agent may occur in an essentially all or none manner depending on whether a nucleic acid bears a modification, the separation may be one of degree. In such embodiments, nucleic acids overrepresented in a modification bind to the agent at a greater extent than nucleic acids underrepresented in the modification. Alternatively, nucleic acids having modifications may bind in an all or nothing manner. But then, various levels of modifications may be sequentially eluted from the binding agent.

For example, in some embodiments, partitioning can be binary or based on degree/level of modifications. For example, all methylated fragments can be partitioned from unmethylated fragments using methyl-binding domain proteins (e.g., MethylMiner Methylated DNA Enrichment Kit (ThermoFisher Scientific)). Subsequently, additional partitioning may involve eluting fragments having different levels of methylation by adjusting the salt concentration in a solution with the methyl-binding domain and bound fragments. As salt concentration increases, fragments having greater methylation levels are eluted.

In some embodiments, the partitioning comprises partitioning the nucleic acid molecules based on a differential binding affinity of the nucleic acid molecules to a binding agent that preferentially binds to nucleic acid molecules comprising nucleotides with epigenetic modification.

In some embodiments, the partitioned sets are representatives of nucleic acids having different extents of modifications (over representative or under representative of modifications). Over representation and under representation can be defined by the number of modifications born by a nucleic acid relative to the median number of modifications per strand in a population. For example, if the median number of 5-methylcytosine nucleotides in nucleic acid molecules in a sample is 2, a nucleic acid molecule including more than two 5-methylcytosine residues is over represented in this modification and a nucleic acid with 1 or zero 5-methylcytosine residues is under represented. The effect of the affinity separation is to partition for nucleic acids over represented in a modification in a bound phase and for nucleic acids underrepresented in a modification in an unbound phase (i.e., in solution). The nucleic acids in the bound phase can be eluted before subsequent processing.

When using MethylMiner Methylated DNA Enrichment Kit (ThermoFisher Scientific) various levels of methylation can be partitioned using sequential elutions. For example, a hypomethylated partition (no methylation) can be separated from a methylated partition by contacting the nucleic acid population with the MBD from the kit, which is attached to magnetic beads. The beads are used to separate out the methylated nucleic acids from the non-methylated nucleic acids. Subsequently, one or more elution steps are performed sequentially to elute nucleic acids having different levels of methylation. For example, a first set of methylated nucleic acids can be eluted at a salt concentration of about 150 mM or about 160 mM or higher, e.g., at least 150 mM, 200mM, 300 mM, 400mM, 500mM, 600mM, 700mM, 800mM, 900mM, 1000mM, or 2000mM. After such methylated nucleic acids are eluted, magnetic separation is once again used to separate higher level of methylated nucleic acids from those with lower level of methylation. The elution and magnetic separation steps can repeat themselves to create various partitions such as a hypomethylated partition (representative of no methylation), a methylated partition (representative of low level of methylation), and a hyper methylated partition (representative of high level of methylation).

In some methods, nucleic acids bound to an agent used for affinity separation are subjected to a wash step. The wash step washes off nucleic acids weakly bound to the affinity agent. Such nucleic acids can be enriched in nucleic acids having the modification to an extent close to the mean or median (i.e., intermediate between nucleic acids remaining bound to the solid phase and nucleic acids not binding to the solid phase on initial contacting of the sample with the agent). The affinity separation results in at least two, and sometimes three or more partitions of nucleic acids with different extents of a modification.

The partitioning of the nucleic acid molecules can be analyzed by sequencing of the nucleic acid molecules partitioned or by digital droplet PCR (ddPCR) or by quantitative PCR (qPCR). Prior to analyzing the partitioning, the nucleic acid molecules in the partitioned sets can be enriched so that the signal from the nucleic acid molecules of interest can be increased and hence improving the sensitivity. In 203, at least a subset of the nucleic acid molecules in the plurality of partitioned sets are enriched such that the epigenetic-control nucleic acid molecules and nucleic acid molecules from the sample of polynucleotides belonging to the regions of interest are enriched.

In some embodiments, prior to the enrichment, each of the plurality of partitioned sets is differentially tagged. The tagged partitioned sets are then pooled together for collective sample preparation and/or sequencing. Differential tagging of the partitioned sets helps in keeping track of the nucleic acid molecules belonging to a particular partitioned set. The tags are usually provided as components of adapters. The nucleic acid molecules in different partitioned sets receive different tags that can distinguish members of one partitioned set from another. The tags linked to nucleic acid molecules of the same partition set can be the same or different from one another. But if different from one another, the tags can have part of their sequence in common so as to identify the molecules to which they are attached as being of a particular partitioned set. For example, if the molecules of the spiked-in sample are partitioned into two partitioned sets - P1 and P2, then the molecules in P1 can be tagged with A1, A2, A3 and so forth and molecules in P2 can be tagged with B1, B2, B3 and so forth. Such a tagging system allows distinguishing the partitioned sets and between the molecules within a partitioned set.

In 204, at least a subset of the enriched molecules are sequenced. The sequence information obtained comprises sequence of the nucleic acid molecules and the tags attached to the nucleic acid molecules. From the sequence of the tags attached to the nucleic acid molecules, one can correlate the tag with the partitioned set of the nucleic acid molecule. The sequence information is used to identify the epigenetic-control nucleic acid molecules and their corresponding partitioned sets. This information is used analyze the partitioning of the epigenetic-control nucleic acid molecules. In 205, one or more epigenetic partition score of the epigenetic-control nucleic acid molecules belonging to one or more epigenetic states in one or more partitioned sets is determined. In some embodiments, the sensitivity and/or specificity of the partitioning method can be assessed by the epigenetic partition scores. Epigenetic partition score is a score that represents the partitioning of nucleic acid molecules belonging to a particular epigenetic state. The epigenetic partition score of the nucleic acid molecules belonging to an epigenetic state is determined for each partitioned set. For example, the epigenetic partition score of the epigenetic-control nucleic acid molecules belonging to a particular epigenetic state can be determined. The epigenetic partition score can be a measure of the number (or statistically estimated number) of nucleic acid molecules belonging to a particular epigenetic state. The epigenetic partition score can be in terms of fraction or percentage. The epigenetic partition score can be a measure of the ratio of the number of epigenetic-control nucleic acid molecules belonging to a particular epigenetic state that's partitioned in at least one partitioned set to the number of epigenetic-control nucleic acid molecules belonging to that epigenetic state present in the other remaining partitioned set(s). In some embodiments, the epigenetic partition score can be a fraction or percentage of the number of epigenetic-control nucleic acid molecules belonging to a particular epigenetic state partitioned in at least one partitioned set to the total number of epigenetic-control nucleic acid molecules belonging to that epigenetic state in all the partitioned sets. In some embodiments, the epigenetic partition score is determined for each epigenetic state of the epigenetic-control nucleic acid molecules in each of the partitioned sets. In some embodiments, the epigenetic partition score is determined for the epigenetic-control nucleic acid molecules with one or more particular epigenetic states in one or more partitioned sets. In some embodiments, the epigenetic partition score is determined for the epigenetic-control nucleic acid molecules with a particular epigenetic state in a particular partitioned set.

In some embodiments, the epigenetic partition score can be directed to the efficiency with which the molecules with no CG ('zero' CG) partitioned to hyper partitioned set. This score can be referred to as *0 CG score.* In some embodiments, the *0 CG score* can be expressed in terms of fraction or percentage of molecules with no CG in the hyper partitioned set. In some embodiments, the epigenetic partition score can be a measure of the fraction of epigenetic-control nucleic acid molecules and/or fraction of hypermethylated control molecules with at least one of the following:
(i) 1 methyl CGs (epigenetic partition score can be referred as *1 CG score*),
(ii) 2 methyl CGs (epigenetic partition score can be referred as *2 CG score*),
(iii) 3 methyl CGs (epigenetic partition score can be referred as *3 CG score*),
(iv) 4 methyl CGs (epigenetic partition score can be referred as *4 CG score*) and
(v) 5 methyl CGs (epigenetic partition score can be referred as *5 CG score*) in the hypermethylated partitioned set (i.e., highly methylated partitioned set).

In some embodiments, the epigenetic partition score can be directed to the efficiency of the hypomethylated (i.e., low methylated) epigenetic-control nucleic acid molecules partitioned to a hypermethylated partitioned set. This score can be referred to as *hypo score.* In some embodiments, the *hypo score* can be expressed in terms of fraction or percentage of the hypomethylated epigenetic-control nucleic acid molecules in the hyper methylated partitioned set. In some embodiments, the epigenetic partition score can be a measure of the number of the methylated CGs required for less than 5% of hypermethylated epigenetic-control nucleic acid molecules in the hypomethylated partitioned set. This score can be referred to as *methyl-5.* In some embodiments, the epigenetic partition score can be a measure of the number of the methylated CGs required for at least 50% of hypermethylated epigenetic-control nucleic acid molecules in the hypermethylated partitioned set. This score can be referred to as *methyl-half.*

For example, three subsets (subsets A, B and C) of epigenetic-control nucleic acid molecules are used and each subset differs in the number of methylated nucleotides. The epigenetic-control nucleic acid molecules in these three subsets can be partitioned into three partitioned sets - P1, P2 and P3, based on their binding affinity to methyl binding protein. For each subset, the epigenetic partition score is determined for each of the partitioned sets (P1, P2 and P3) - i.e. epigenetic-control nucleic acid molecules belonging to subset A will have three epigenetic partition scores - one for each of the three partitioned sets, P1, P2 and P3. Likewise, each of subsets B and C will have three epigenetic partition scores - one for each of the three partitioned sets P1, P2 and P3. The epigenetic partition score can be determined for the endogenous control molecules as well.

In another embodiment, three subsets (subsets A, B and C) of epigenetic-control nucleic acid molecules are used and each subset differs in the number of methylated nucleotides (i.e. each subset has a different epigenetic state). The epigenetic-control nucleic acid molecules in these three subsets can be partitioned into three partitioned sets - P1, P2 and P3, based on their binding affinity to methyl binding protein. In this embodiment, the epigenetic score is determined only for Subset A molecules in P1 partitioned set. This epigenetic score can be a measure of the fraction or percentage of Subset A molecules in P1 partitioned set to the total number of Subset A molecules (in P1, P2 and P3 partitioned sets).

Epigenetic partition score can be any value or range between 0 - 1 (in terms of fraction) or between 0 - 100% (in in terms of percentage). In some embodiments, epigenetic partition score can be in terms of the number of methylated CGs (for e.g., *methyl-half* and *methyl-5*).

In 206, the epigenetic partition scores of the epigenetic-control nucleic acid molecules are compared to epigenetic partition cut-offs (predetermined cut-offs) to evaluate the partitioning method. Epigenetic partition cut-off is a predetermined cut-off value or cut-off range used to evaluate the partitioning of the nucleic acid molecules belonging to a particular epigenetic state and each partitioned set has an epigenetic partition cut-off for the nucleic acid molecules belonging to an epigenetic state. The epigenetic partition cut-offs differ with the epigenetic state of the nucleic acid molecules and partitioned set, i.e., each epigenetic state will have its own epigenetic partition cut-off and every partitioned set has a separate epigenetic partition cut-off for that epigenetic state. The cut-off can be in terms of percentage or fraction and the cut-off can be a cut-off range instead of a particular cut-off value. For example, the epigenetic partition cut-offs for the epigenetic-control nucleic acid molecules belonging to a particular epigenetic state can be between 70% - 79%, between 10% - 15% and less than 5% for partitioned sets P1, P2 and P3 respectively. If the epigenetic partition scores of the epigenetic-control nucleic acid molecules belonging to that epigenetic state is within the corresponding epigenetic partition cut-offs, then partitioning method is a success. In some embodiments, the epigenetic partition cut-off for 0 CG score can be 0.01%, 0.02%, 0.05%, 0.1%, 0.2%, 0.3%, 0.4%, 0.5%, 0.6%, 0.7%, 0.8%, 0.9%, 1%, 2%, 5%, at least 5% or at least 10%. In some embodiments, the epigenetic partition cut-off for 0 CG score can be 0.01%. In some embodiments, the epigenetic partition cut-off for 0 CG score can be 0.02%. In some embodiments, the epigenetic partition cut-off for 0 CG score can be 0.03. In some embodiments, the epigenetic partition cut-off for 0 CG score can be 0.04%. In some embodiments, the epigenetic partition cut-off for 0 CG score can be 0.05%. In some embodiments, the epigenetic partition cut-off for 0 CG score can be 0.1%. In some embodiments, the epigenetic partition cut-off for 0 CG score can be 0.2%. In some embodiments, the epigenetic partition cut-off for 0 CG score can be 0.3%. In some embodiments, the epigenetic partition cut-off for 0 CG score can be 0.4%. In some embodiments, the epigenetic partition cut-off for 0 CG score can be 0.5%. In some embodiments, the epigenetic partition cut-off for 0 CG score can be 0.6%. In some embodiments, the epigenetic partition cut-off for 0 CG score can be 0.7%. In some embodiments, the epigenetic partition cut-off for 0 CG score can be 0.8%. In some embodiments, the epigenetic partition cut-off for 0 CG score can be 0.9%. In some embodiments, the epigenetic partition cut-off for 0 CG score can be 1%.

In some embodiments, the epigenetic partition cut-off for the hypo score can be 0.1%, 0.5%, 1%, 2%, 3%, 4%, 5%, 7% or at least 10%. In some embodiments, the epigenetic partition cut-off for the hypo score can be 0.1%. In some embodiments, the epigenetic partition cut-off for the hypo score can be 0.5%. In some embodiments, the epigenetic partition cut-off for the hypo score can be 1%. In some embodiments, the epigenetic partition cut-off for the hypo score can be 2%. In some embodiments, the epigenetic partition cut-off for the hypo score can be 3%. In some embodiments, the epigenetic partition cut-off for the hypo score can be 4%. In some embodiments, the epigenetic partition cut-off for the hypo score can be 5%.

In some embodiments, the cut-off can be in terms of the number of methylated CGs (for e.g., in *methyl-5* and *methyl-half*). In some embodiments, the epigenetic partition cut-off for the methyl-5 can be 5, 10, 20, 30, 40 or 50 mCGs. In some embodiments, the epigenetic partition cut-off for the methyl-5 can be 5 mCGs. In some embodiments, the epigenetic partition cut-off for the methyl-5 can be 10 mCGs. In some embodiments, the epigenetic partition cut-off for the methyl-5 can be 20 mCGs. In some embodiments, the epigenetic partition cut-off for the methyl-5 can be 30 mCGs. In some embodiments, the epigenetic partition cut-off for the methyl-5 can be 40 mCGs. In some embodiments, the epigenetic partition cut-off for the methyl-5 can be 50 mCGs.

In some embodiments, the epigenetic partition cut-off for the methyl-half score can be 5, 10, 15, 20, 25, 30, 35 or 40 mCGs. In some embodiments, the epigenetic partition cut-off for the methyl-half score can be 5 mCGs. In some embodiments, the epigenetic partition cut-off for the methyl-half score can be 10 mCGs. In some embodiments, the epigenetic partition cut-off for the methyl-half score can be 15 mCGs. In some embodiments, the epigenetic partition cut-off for the methyl-half score can be 20 mCGs. In some embodiments, the epigenetic partition cut-off for the methyl-half score can be 25 mCGs. In some embodiments, the epigenetic partition cut-off for the methyl-half score can be 30 mCGs. In some embodiments, the epigenetic partition cut-off for the methyl-half score can be 35 mCGs. In some embodiments, the epigenetic partition cut-off for the methyl-half score can be 40 mCGs.

In some embodiments, if the one or more epigenetic partition scores of epigenetic-control nucleic acid molecules belonging to one or more epigenetic states in one or more partitioned sets is within the corresponding epigenetic partition cut-offs, then the partitioning method may be classified as being a success. Otherwise, the partitioning method may be classified as being unsuccessful is the epigenetic partition scores are outside of the cut-offs for all partitioned sets. For example, there are two subsets of epigenetic-control nucleic acid molecules - subset A and B, and each subset differs in the degree of epigenetic modification (i.e., each subset differs in the epigenetic state). These epigenetic-control nucleic acid molecules can be partitioned into two partitioned sets - P1 and P2. For molecules belonging to Subset A, two epigenetic partition scores (e.g. S1 and S2), one for each partitioned set P1 and P2, will be determined based on their partitioning. Likewise, for molecules belonging to subset B two epigenetic partition scores (e.g. S3 and S4), one for P1 and one for P2, will be determined. Each subset of molecules with a particular epigenetic state will have a predetermined epigenetic partition cut-off for each of the partitioned sets. In this example, epigenetic-control nucleic acid molecules of subset A will have two epigenetic partition cut-offs, C1 and C2 (for two partitioned sets P1 and P2) and likewise, epigenetic-control nucleic acid molecules of subset B will have two epigenetic partition cut-offs, C3 and C4. The epigenetic partition scores of both the subsets are compared with their corresponding epigenetic partition cut-offs. In this example, the partitioning method will be considered successful only if all the four epigenetic partition scores are within their corresponding epigenetic partition cut-offs i.e., in this example, S1 < C1 and S2 < C2 and S3 < C3 and S4 < C4. Otherwise, the partitioning method may be classified as being unsuccessful is the epigenetic partition scores are outside of the cut-offs for all partitioned sets.

In another embodiment, three subsets (subsets A, B and C) of epigenetic-control nucleic acid molecules are used and each subset differs in the number of methylated nucleotides (i.e. each subset has a different epigenetic state). The epigenetic-control nucleic acid molecules in these three subsets can be partitioned into three partitioned sets - P1, P2 and P3, based on their binding affinity to methyl binding protein. In this embodiment, the epigenetic score is determined only for Subset A molecules in P1 partitioned set. This epigenetic score can be a measure of the fraction or percentage of Subset A molecules in P1 partitioned set to the total number of Subset A molecules (in P1, P2 and P3 partitioned sets). If this epigenetic partition score is within its corresponding epigenetic partition cut-off, then the partitioning method is classified as being successful. Otherwise the partitioning method is classified as being unsuccessful.

In another aspect, the present disclosure provides a method for evaluating partitioning of nucleic acid molecules in a sample of polynucleotides based on epigenetic state, comprising: (a) adding a set of epigenetic-control nucleic acid molecules to the nucleic acid molecules in the sample of polynucleotides, thereby producing a spiked-in sample; (b) partitioning nucleic acid molecules of at least a subset of the spiked-in sample into a plurality of partitioned sets; (c) enriching at least a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides, wherein the group of nucleic acid molecules from the sample of polynucleotides comprises a set of endogenous control molecules; (d) sequencing at least a subset of the set of enriched molecules to produce a set of sequencing reads; (e) analyzing at least a subset of the set of sequencing reads to generate one or more epigenetic partition scores for the epigenetic-control nucleic acid molecules and the set of endogenous control molecules; and (f) comparing the one or more epigenetic partition scores with one or more epigenetic partition cut-offs. In these embodiments, the partitioning of the nucleic acid molecules of the sample and the epigenetic-control nucleic acid molecules necessarily take place concurrently. In some embodiments, the analyzing step comprises estimating the number/fraction of the epigenetic-control nucleic acid molecules and/or endogenous control molecules at a given epigenetic state in at least one of the partitioned sets.

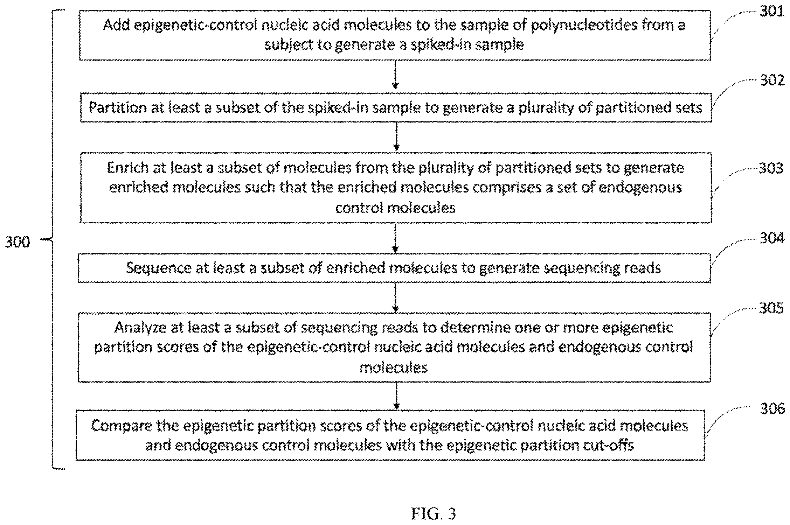

FIG. 3 illustrates an example embodiment of a method 300 for evaluating partitioning of nucleic acid molecules in a sample of polynucleotides based on epigenetic state. In this embodiment, the partitioning of both epigenetic-control nucleic acid molecules and endogenous control molecules are analyzed to evaluate the partitioning method. There are regions in the human genome with a particular epigenetic state and the epigenetic state of that region does not vary/change often and always remains the same/remains consistent with different subjects and/or different types of disease/disease stages. Nucleic acid molecules in the sample of polynucleotides that correspond to such human genomic regions with non-variable epigenetic state are referred as endogenous control molecules. In 301, the epigenetic-control nucleic acid molecules are added to the sample of polynucleotides, whose partitioning is to be evaluated, to generate a spiked-in sample.

In some embodiments, the epigenetic-control nucleic acid molecules can comprise one or more subsets of nucleic acid molecules with different levels of epigenetic state (i.e., different number of epigenetically modified nucleotides). In some embodiments, epigenetic-control nucleic acid molecules can comprise nucleic acid molecules with different sequences and/or different lengths. In other embodiments, the epigenetic-control nucleic acid molecules can comprise nucleic acid molecules with identical sequence or of identical length.

In 302, the nucleic acid molecules of at least a subset of the spiked-in sample, which comprises both epigenetic-control nucleic acid molecules and nucleic acid molecules from the sample of polynucleotides, are partitioned or fractionated into a plurality of partitioned sets based on the epigenetic state of the molecules. Partitioning can be based on the presence or absence of an epigenetic modification and/or can be based on the degree of epigenetic modification. Examples of epigenetic modification include, but not limited to presence or absence of methylation, level of methylation and type of methylation (5' cytosine). In some embodiments, epigenetic modification can be DNA methylation. In those embodiments, molecules of the spiked-in sample are partitioned based on the different levels of methylation (different number of methylated nucleotides). In some embodiments, the spiked-in sample can be partitioned into two or more partitioned sets (e.g. at least 3, 4, 5, 6, or 7 partitioned sets). In some embodiments, partitioning is based on the differential binding affinity of the nucleic acid molecules to a binding agent.

The partitioning of the nucleic acid molecules can be analyzed by sequencing of the nucleic acid molecules partitioned, by digital droplet PCR (ddPCR) or by quantitative PCR(qPCR). Prior to analyzing the partitioning, the nucleic acid molecules in the partitioned sets can be enriched so that the signal from the nucleic acid molecules of interest can be increased and hence improving the sensitivity. In 303, at least a subset of the nucleic acid molecules in the plurality of partitioned sets are enriched such that the epigenetic-control nucleic acid molecules, endogenous control molecules (from the sample of polynucleotides) and other nucleic acid molecules from the sample of polynucleotides belonging to the regions of interest are enriched.

In some embodiments, prior to the enrichment, each of the plurality of partitioned sets is differentially tagged. The tagged partitioned sets are then pooled together for collective sample preparation and/or sequencing. Differential tagging of the partitioned sets helps in keeping track of the nucleic acid molecules belonging to a particular partitioned set. The tags are usually provided as components of adapters. The nucleic acid molecules in different partitioned sets receive different tags that can distinguish members of one partitioned set from another. The tags linked to nucleic acid molecules of the same partition set can be the same or different from one another. But if different from one another, the tags can have part of their sequence in common so as to identify the molecules to which they are attached as being of a particular partitioned set.

In 304, at least a subset of the enriched molecules are sequenced. The sequence information obtained comprises sequence of the nucleic acid molecules and the tags attached to the nucleic acid molecules. From the sequence of the tags attached to the nucleic acid molecules, one can correlate the tag with the partitioned set of the nucleic acid molecule. The sequence information is used to identify epigenetic-control nucleic acid molecules and endogenous control molecules and their corresponding partitioned sets. This information is used analyze the partitioning of the epigenetic-control nucleic acid molecules and endogenous control molecules. In 305, one or more epigenetic partition scores of the epigenetic-control nucleic acid molecules and endogenous control molecules belonging to one or more epigenetic state in one or more partitioned sets is determined. In some embodiments, the sensitivity and/or specificity of the partitioning method can be assessed by the epigenetic partition scores. Epigenetic partition score is a score that represents the partitioning of nucleic acid molecules belonging to a particular epigenetic state. The epigenetic partition score of the nucleic acid molecules belonging to an epigenetic state is determined for each partitioned set. For example, the epigenetic partition scores of the epigenetic-control nucleic acid molecules and endogenous control molecules belonging to a particular epigenetic state can be determined. The epigenetic partition score can be a measure of the number (or statistically estimated number) of nucleic acid molecules belonging to a particular epigenetic state. The epigenetic partition score can be in terms of fraction or percentage. The epigenetic partition score can be a measure of (i) for epigenetic-control nucleic acid molecules: the ratio of the number of epigenetic-control nucleic acid molecules belonging to a particular epigenetic state that's partitioned in at least one partitioned set to the number of epigenetic-control nucleic acid molecules belonging to that epigenetic state present in the other remaining partitioned set(s) and (ii) for endogenous control molecules: ratio of the number of endogenous control molecules belonging to a particular epigenetic state that's partitioned in at least one partitioned set to the number of endogenous control molecules belonging to that epigenetic state present in the other remaining partitioned set(s). In some embodiments, the epigenetic partition score can be (i) for epigenetic-control nucleic acid molecules: a fraction or percentage of the number of epigenetic-control nucleic acid molecules belonging to a particular epigenetic state partitioned in at least one partitioned set to the total number of epigenetic-control nucleic acid molecules belonging to that epigenetic state in all the partitioned sets and (ii) for endogenous control molecules: a fraction or percentage of the number of endogenous control molecules belonging to a particular epigenetic state partitioned in at least one partitioned set to the total number of endogenous control molecules belonging to that epigenetic state in all the partitioned sets. In some embodiments, the epigenetic partition score is determined for each epigenetic state of the epigenetic-control nucleic acid molecules and endogenous control molecules in each of the partitioned sets. In some embodiments, the epigenetic partition score is determined for the epigenetic-control nucleic acid molecules and endogenous control molecules with one or more particular epigenetic states in one or more partitioned sets. In some embodiments, the epigenetic partition score is determined for the epigenetic-control nucleic acid molecules and endogenous control molecules with a particular epigenetic state in a particular partitioned set.

In some embodiments, the epigenetic partition score can be directed to the efficiency with which the molecules with no CG ('zero' CG) partitioned to hyper partitioned set. This score can be referred to as *0 CG score.* In some embodiments, the *0 CG score* can be expressed in terms of fraction or percentage of molecules with no CG in the hyper partitioned set. In some embodiments, the epigenetic partition score can be a measure of the fraction of epigenetic-control nucleic acid molecules and/or fraction of hypermethylated control molecules with at least one of the following:
(vi) 1 methyl CGs (epigenetic partition score can be referred as *1 CG score*),
(vii) 2 methyl CGs (epigenetic partition score can be referred as *2 CG score*),
(viii) 3 methyl CGs (epigenetic partition score can be referred as *3 CG score*),
(ix) 4 methyl CGs (epigenetic partition score can be referred as *4 CG score*) and
(x) 5 methyl CGs (epigenetic partition score can be referred as *5 CG score*) in the hypermethylated partitioned set (i.e. highly methylated partitioned set).

In some embodiments, the epigenetic partition score can be directed to the efficiency of the hypomethylated control molecules or hypomethylated epigenetic-control nucleic acid molecules partitioned to a hypermethylated partitioned set. This score can be referred to as *hypo score.* In some embodiments, the *hypo score* can be expressed in terms of fraction or percentage of the hypomethylated control molecules or hypomethylated epigenetic-control nucleic acid molecules in the hyper methylated partitioned set. In some embodiments, the epigenetic partition score can be a measure of the number of the methylated CGs required for less than 5% of hypermethylated control molecules and/or hypermethylated epigenetic-control nucleic acid molecules in the hypomethylated partitioned set. This score can be referred to as *methyl-5.* In some embodiments, the epigenetic partition score can be a measure of the number of the methylated CGs required for at least 50% of hypermethylated control molecules and/or hypermethylated epigenetic-control nucleic acid molecules in the hypermethylated partitioned set. This score can be referred to as *methyl-half.*

For example, three subsets (subsets A, B and C) of epigenetic-control nucleic acid molecules are used and each subset differs in the number of methylated nucleotides. The epigenetic-control nucleic acid molecules in these three subsets can be partitioned into three partitioned sets - P1, P2 and P3, based on their binding affinity to methyl binding protein. For each subset, the epigenetic partition score is determined for each of the partitioned sets (P1, P2 and P3) - i.e. epigenetic-control nucleic acid molecules belonging to Subset A will have three epigenetic partition scores - one for each of the three partitioned sets, P1, P2 and P3. Likewise, each of subsets B and C will have three epigenetic partition scores - one for each of the three partitioned sets P1, P2 and P3. The epigenetic partition score can be determined for the endogenous control molecules as well.

In another embodiment, three subsets (subsets A, B and C) of epigenetic-control nucleic acid molecules are used and each subset differs in the number of methylated nucleotides (i.e. each subset has a different epigenetic state). The epigenetic-control nucleic acid molecules in these three subsets can be partitioned into three partitioned sets - P1, P2 and P3, based on their binding affinity to methyl binding protein. In this embodiment, the epigenetic score is determined only for Subset A molecules in P1 partitioned set. This epigenetic score can be a measure of the fraction or percentage of Subset A molecules in P1 partitioned set to the total number of Subset A molecules (in P1, P2 and P3 partitioned sets).

Epigenetic partition score can be any value or range between 0 - 1 (in terms of fraction) or between 0 - 100% (in terms of percentage). In some embodiments, epigenetic partition score can be in terms of number of methylated CGs (for e.g., in methyl-half and methyl-5).

In 306, the epigenetic partition scores of the epigenetic-control nucleic acid molecules and endogenous control molecules are compared to their corresponding epigenetic partition cut-offs (predetermined cut-offs) to evaluate the partitioning method. Epigenetic partition cut-off is a predetermined cut-off value or cut-off range used to evaluate the partitioning of the nucleic acid molecules belonging to a particular epigenetic state and each partitioned set has an epigenetic partition cut-off for the nucleic acid molecules belonging to an epigenetic state. The epigenetic partition cut-offs differ with the epigenetic state of the nucleic acid molecules and partitioned set, i.e., each epigenetic state will have its own epigenetic partition cut-off and every partitioned set has a separate epigenetic partition cut-off for that epigenetic state. The cut-off can be in terms of percentage or fraction and the cut-off can be a cut-off range instead of a particular cut-off value. For example, the epigenetic partition cut-offs for the epigenetic-control nucleic acid molecules belonging to a particular epigenetic state can be between 70% - 79%, between 10% - 15% and less than 5% for partitioned sets P1, P2 and P3 respectively. If the epigenetic partition scores of the epigenetic-control nucleic acid molecules belonging to that epigenetic state is within the corresponding epigenetic partition cut-offs, then partitioning method is a success.

In some embodiments, the epigenetic partition cut-off for 0 CG score can be 0.01%, 0.02%, 0.05%, 0.1%, 0.2%, 0.3%, 0.4%, 0.5%, 0.6%, 0.7%, 0.8%, 0.9%, 1%, 2%, 5%, at least 5% or at least 10%. In some embodiments, the epigenetic partition cut-off for 0 CG score can be 0.01%. In some embodiments, the epigenetic partition cut-off for 0 CG score can be 0.02%. In some embodiments, the epigenetic partition cut-off for 0 CG score can be 0.03. In some embodiments, the epigenetic partition cut-off for 0 CG score can be 0.04%. In some embodiments, the epigenetic partition cut-off for 0 CG score can be 0.05%. In some embodiments, the epigenetic partition cut-off for 0 CG score can be 0.1%. In some embodiments, the epigenetic partition cut-off for 0 CG score can be 0.2%. In some embodiments, the epigenetic partition cut-off for 0 CG score can be 0.3%. In some embodiments, the epigenetic partition cut-off for 0 CG score can be 0.4%. In some embodiments, the epigenetic partition cut-off for 0 CG score can be 0.5%. In some embodiments, the epigenetic partition cut-off for 0 CG score can be 0.6%. In some embodiments, the epigenetic partition cut-off for 0 CG score can be 0.7%. In some embodiments, the epigenetic partition cut-off for 0 CG score can be 0.8%. In some embodiments, the epigenetic partition cut-off for 0 CG score can be 0.9%. In some embodiments, the epigenetic partition cut-off for 0 CG score can be 1%.

In some embodiments, the epigenetic partition cut-off for the hypo score can be 0.1%, 0.5%, 1%, 2%, 3%, 4%, 5%, 7% or at least 10%. In some embodiments, the epigenetic partition cut-off for the hypo score can be 0.1%. In some embodiments, the epigenetic partition cut-off for the hypo score can be 0.5%. In some embodiments, the epigenetic partition cut-off for the hypo score can be 1%. In some embodiments, the epigenetic partition cut-off for the hypo score can be 2%. In some embodiments, the epigenetic partition cut-off for the hypo score can be 3%. In some embodiments, the epigenetic partition cut-off for the hypo score can be 4%. In some embodiments, the epigenetic partition cut-off for the hypo score can be 5%.

In some embodiments, the epigenetic partition cut-off for the methyl-5 can be 5, 10, 20, 30, 40 or 50 mCGs. In some embodiments, the epigenetic partition cut-off for the methyl-5 can be 5 mCGs. In some embodiments, the epigenetic partition cut-off for the methyl-5 can be 10 mCGs. In some embodiments, the epigenetic partition cut-off for the methyl-5 can be 20 mCGs. In some embodiments, the epigenetic partition cut-off for the methyl-5 can be 30 mCGs. In some embodiments, the epigenetic partition cut-off for the methyl-5 can be 40 mCGs. In some embodiments, the epigenetic partition cut-off for the methyl-5 can be 50 mCGs.

In some embodiments, the epigenetic partition cut-off for the methyl-half score can be 5, 10, 15, 20, 25, 30, 35 or 40 mCGs. In some embodiments, the epigenetic partition cut-off for the methyl-half score can be 5 mCGs. In some embodiments, the epigenetic partition cut-off for the methyl-half score can be 10 mCGs. In some embodiments, the epigenetic partition cut-off for the methyl-half score can be 15 mCGs. In some embodiments, the epigenetic partition cut-off for the methyl-half score can be 20 mCGs. In some embodiments, the epigenetic partition cut-off for the methyl-half score can be 25 mCGs. In some embodiments, the epigenetic partition cut-off for the methyl-half score can be 30 mCGs. In some embodiments, the epigenetic partition cut-off for the methyl-half score can be 35 mCGs. In some embodiments, the epigenetic partition cut-off for the methyl-half score can be 40 mCGs.

In some embodiments, if the one or more epigenetic partition scores of epigenetic-control nucleic acid molecules and endogenous control molecules belonging to one or more epigenetic states in one or more partitioned sets is within the corresponding epigenetic partition cut-offs, then the partitioning method may be classified as being successful. Otherwise, the partitioning method may be classified as unsuccessful.

In another aspect, the present disclosure provides a method for evaluating partitioning of nucleic acid molecules in a sample of polynucleotides based on epigenetic state, comprising: (a) partitioning nucleic acid molecules of at least a subset of the sample of polynucleotides into a plurality of partitioned sets; (c) enriching at least a subset of molecules from the plurality of partitioned sets to generate a set of enriched molecules, wherein the set of enriched molecules comprises a group of nucleic acid molecules from the sample of polynucleotides, wherein the group of nucleic acid molecules from sample the cell-free polynucleotides comprises a set of endogenous control molecules; (d) sequencing at least a subset of the set of enriched molecules to produce a set of sequencing reads; (e) analyzing at least a subset of the set of sequencing reads to generate one or more epigenetic partition scores for the set of endogenous control molecules; and (f) comparing the one or more epigenetic partition scores with one or more of epigenetic partition cut-offs. In these embodiments, the partitioning of the nucleic acid molecules of the sample and the epigenetic-control nucleic acid molecules necessarily take place concurrently. In some embodiments, the analyzing step comprises estimating the number/fraction of the endogenous control molecules at a given epigenetic state in at least one of the partitioned sets.

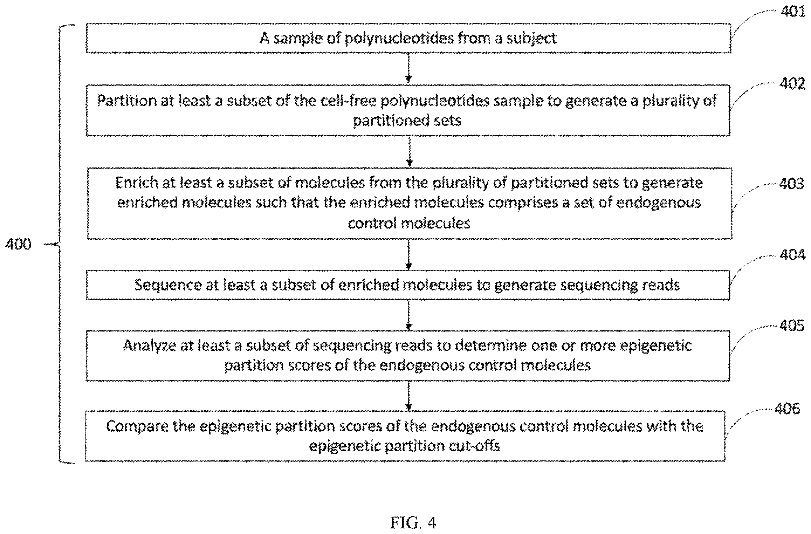

FIG. 4 illustrates an example embodiment of a method 400 for evaluating partitioning of nucleic acid molecules in a sample of polynucleotides based on epigenetic state. In this embodiment, the partitioning of endogenous control molecules in the sample of polynucleotides is analyzed to evaluate the partitioning method. There are regions in the human genome with a particular epigenetic state and the epigenetic state of that region does not vary/change often and always remains the same/remains consistent with different subjects and/or different types of disease/disease stages. Nucleic acid molecules in the sample of polynucleotides that correspond to such human genomic regions with non-variable epigenetic state are referred as endogenous control molecules. In 401, a sample of polynucleotides from a subject is considered. In 402, the nucleic acid molecules of at least a subset of the sample of polynucleotides are partitioned or fractionated into a plurality of partitioned sets based on the epigenetic state of the molecules. Partitioning can be based on the presence or absence of an epigenetic modification and/or can be based on the degree of epigenetic modification. Examples of epigenetic modification may include, but are not limited to, the presence or absence of methylation, level of methylation and type of methylation (5' cytosine). In some embodiments, epigenetic modification can be DNA methylation. In those embodiments, molecules of the spiked-in sample are partitioned based on the different levels of methylation (different number of methylated nucleotides). In some embodiments, the spiked-in sample can be partitioned into two or more partitioned sets (e.g. at least 3, 4, 5, 6, or 7 partitioned sets). In some embodiments, partitioning is based on the differential binding affinity of the nucleic acid molecules to a binding agent.

The partitioning of the nucleic acid molecules can be analyzed by sequencing of the nucleic acid molecules partitioned or by digital droplet PCR (ddPCR). Prior to analyzing the partitioning, the nucleic acid molecules in the partitioned sets can be enriched so that the signal from the nucleic acid molecules of interest can be increased and hence improving the sensitivity. In 403, at least a subset of the nucleic acid molecules in the plurality of partitioned sets are enriched such that the endogenous control molecules (from the sample of polynucleotides) and other nucleic acid molecules from the sample of polynucleotides belonging to the regions of interest are enriched.

In some embodiments, prior to the enrichment, each of the plurality of partitioned sets is differentially tagged. The tagged partitioned sets are then pooled together for collective sample preparation and/or sequencing. Differential tagging of the partitioned sets helps in keeping track of the nucleic acid molecules belonging to a particular partitioned set. The tags are usually provided as components of adapters. The nucleic acid molecules in different partitioned sets receive different tags that can distinguish members of one partitioned set from another. The tags linked to nucleic acid molecules of the same partition set can be the same or different from one another. But if different from one another, the tags can have part of their sequence in common so as to identify the molecules to which they are attached as being of a particular partitioned set.

In 404, at least a subset of the enriched molecules are sequenced. The sequence information obtained comprises sequence of the nucleic acid molecules and the tags attached to the nucleic acid molecules. From the sequence of the tags attached to the nucleic acid molecules, one can correlate the tag with the partitioned set of the nucleic acid molecule. The sequence information is used to identify endogenous control molecules and their corresponding partitioned sets. This information is used analyze the partitioning of the endogenous control molecules. In 405, one or more epigenetic partition scores of the endogenous control molecules belonging to one or more partitioned sets is determined. In some embodiments, the sensitivity and/or specificity of the partitioning method can be assessed by the epigenetic partition scores. Epigenetic partition score is a score that represents the partitioning of nucleic acid molecules belonging to a particular epigenetic state. In some embodiments, the epigenetic partition score of the nucleic acid molecules belonging to an epigenetic state is determined for each partitioned set. For example, the epigenetic partition scores of the endogenous control molecules belonging to a particular epigenetic state can be determined. The epigenetic partition score can be a measure of the number (or statistically estimated number) of nucleic acid molecules belonging to a particular epigenetic state. The epigenetic partition score can be in terms of fraction or percentage. The epigenetic partition score can be a measure of ratio of the number of endogenous control molecules belonging to a particular epigenetic state that's partitioned in at least one partitioned set to the number of endogenous control molecules belonging to that epigenetic state present in the other remaining partitioned set(s). In some embodiments, the epigenetic partition score can be a fraction or percentage of the number of endogenous control molecules belonging to a particular epigenetic state partitioned in at least one partitioned set to the total number of endogenous control molecules belonging to that epigenetic state in all the partitioned sets. In some embodiments, the epigenetic partition score is determined for each epigenetic state of the endogenous control molecules in each of the partitioned sets. In some embodiments, the epigenetic partition score is determined for the endogenous control molecules with one or more particular epigenetic states in one or more partitioned sets. In some embodiments, the epigenetic partition score is determined for the endogenous control molecules with a particular epigenetic state in a particular partitioned set.

In some embodiments, the epigenetic partition score can be directed to the efficiency with which the molecules with no CG ('zero' CG) partitioned to hyper partitioned set. This score can be referred to as *0 CG score.* In some embodiments, the *0 CG score* can be expressed in terms of fraction or percentage of molecules with no CG in the hyper partitioned set. In some embodiments, the epigenetic partition score can be a measure of the fraction of hypermethylated control molecules with at least one of the following:
(xi) 1 methyl CGs (epigenetic partition score can be referred as *1 CG score*),
(xii) 2 methyl CGs (epigenetic partition score can be referred as *2 CG score*),
(xiii) 3 methyl CGs (epigenetic partition score can be referred as *3 CG score*),
(xiv) 4 methyl CGs (epigenetic partition score can be referred as *4 CG score*) and
(xv) 5 methyl CGs (epigenetic partition score can be referred as 5 *CG score*) in the hypermethylated partitioned set (i.e. highly methylated partitioned set).

In some embodiments, the epigenetic partition score can be directed to the efficiency of the hypomethylated control molecules partitioned to a hypermethylated partitioned set. This score can be referred to as *hypo score.* In some embodiments, the *hypo score* can be expressed in terms of fraction or percentage of the hypomethylated control molecules in the hyper methylated partitioned set. In some embodiments, the epigenetic partition score can be a measure of the number of the methylated CGs required for less than 5% of hypermethylated control molecules in the hypomethylated partitioned set. This score can be referred to as *methyl-5.* In some embodiments, the epigenetic partition score can be a measure of the number of the methylated CGs required for at least 50% of hypermethylated control molecules in the hypermethylated partitioned set. This score can be referred to as *methyl-half.*

For example, two subsets (subsets A and B) of endogenous control molecules are analzyed and each subset differs in the level/degree of methylation (i.e. each subset has a different epigenetic state). The endogenous control molecules in these two subsets can be partitioned into three partitioned sets - P1, P2 and P3, based on their binding affinity to methyl binding protein. For each subset, the epigenetic partition score is determined for each of the partitioned sets (P1, P2 and P3) - i.e. epigenetic-control nucleic acid molecules belonging to Subset A will have three epigenetic partition scores - one for each of the three partitioned sets, P1, P2 and P3. Likewise, Subset B will have three epigenetic partition scores - one for each of the three partitioned sets P1, P2 and P3.

In another embodiment, three subsets (subsets A, B and C) of endogenous control molecules are analyzed and each subset differs in the level/degree of methylation (i.e. each subset has a different epigenetic state). The endogenous control molecules in these three subsets can be partitioned into three partitioned sets - P1, P2 and P3, based on their binding affinity to methyl binding protein. In this embodiment, the epigenetic score is determined only for endogenous molecules of Subset A in P1 partitioned set. This epigenetic score can be a measure of the fraction or percentage of endogenous control molecules of Subset A in P1 partitioned set to the total number of Subset A endogenous control molecules (in P1, P2 and P3 partitioned sets).

Epigenetic partition score can be any value or range between 0 - 1 (in terms of fraction) or between 0 - 100% (in terms of percentage). In some embodiments, epigenetic partition score can be in terms of number of methylated CGs (for e.g., in methyl-half and methyl-5)

In 406, the epigenetic partition scores of the endogenous control molecules are compared to their corresponding epigenetic partition cut-offs (predetermined cut-offs) to evaluate the partitioning method. Epigenetic partition cut-off is a predetermined cut-off value or cut-off range used to evaluate the partitioning of the nucleic acid molecules belonging to a particular epigenetic state and each partitioned set has an epigenetic partition cut-off for the nucleic acid molecules belonging to an epigenetic state. The epigenetic partition cut-offs differ with the epigenetic state of the nucleic acid molecules and partitioned set, i.e., each epigenetic state will have its own epigenetic partition cut-off and every partitioned set has a separate epigenetic partition cut-off for that epigenetic state. The cut-off can be in terms of percentage or fraction and the cut-off can be a cut-off range instead of a particular cut-off value. For example, the epigenetic partition cut-offs for the endogenous control molecules belonging to a particular epigenetic state can be between 70% - 79%, between 10% - 15% and less than 5% for partitioned sets P1, P2 and P3 respectively. If the epigenetic partition scores of the endogenous control molecules belonging to that epigenetic state is within the corresponding epigenetic partition cut-offs, then partitioning method is a success.

In some embodiments, the epigenetic partition cut-off for 0 CG score can be 0.01%, 0.02%, 0.05%, 0.1%, 0.2%, 0.3%, 0.4%, 0.5%, 0.6%, 0.7%, 0.8%, 0.9%, 1%, 2%, 5%, at least 5% or at least 10%. In some embodiments, the epigenetic partition cut-off for 0 CG score can be 0.01%. In some embodiments, the epigenetic partition cut-off for 0 CG score can be 0.02%. In some embodiments, the epigenetic partition cut-off for 0 CG score can be 0.03. In some embodiments, the epigenetic partition cut-off for 0 CG score can be 0.04%. In some embodiments, the epigenetic partition cut-off for 0 CG score can be 0.05%. In some embodiments, the epigenetic partition cut-off for 0 CG score can be 0.1%. In some embodiments, the epigenetic partition cut-off for 0 CG score can be 0.2%. In some embodiments, the epigenetic partition cut-off for 0 CG score can be 0.3%. In some embodiments, the epigenetic partition cut-off for 0 CG score can be 0.4%. In some embodiments, the epigenetic partition cut-off for 0 CG score can be 0.5%. In some embodiments, the epigenetic partition cut-off for 0 CG score can be 0.6%. In some embodiments, the epigenetic partition cut-off for 0 CG score can be 0.7%. In some embodiments, the epigenetic partition cut-off for 0 CG score can be 0.8%. In some embodiments, the epigenetic partition cut-off for 0 CG score can be 0.9%. In some embodiments, the epigenetic partition cut-off for 0 CG score can be 1%.

In some embodiments, the epigenetic partition cut-off for the hypo score can be 0.1%, 0.5%, 1%, 2%, 3%, 4%, 5%, 7% or at least 10%. In some embodiments, the epigenetic partition cut-off for the hypo score can be 0.1%. In some embodiments, the epigenetic partition cut-off for the hypo score can be 0.5%. In some embodiments, the epigenetic partition cut-off for the hypo score can be 1%. In some embodiments, the epigenetic partition cut-off for the hypo score can be 2%. In some embodiments, the epigenetic partition cut-off for the hypo score can be 3%. In some embodiments, the epigenetic partition cut-off for the hypo score can be 4%. In some embodiments, the epigenetic partition cut-off for the hypo score can be 5%.

In some embodiments, the epigenetic partition cut-off for the methyl-5 can be 5, 10, 20, 30, 40 or 50 mCGs. In some embodiments, the epigenetic partition cut-off for the methyl-5 can be 5 mCGs. In some embodiments, the epigenetic partition cut-off for the methyl-5 can be 10 mCGs. In some embodiments, the epigenetic partition cut-off for the methyl-5 can be 20 mCGs. In some embodiments, the epigenetic partition cut-off for the methyl-5 can be 30 mCGs. In some embodiments, the epigenetic partition cut-off for the methyl-5 can be 40 mCGs. In some embodiments, the epigenetic partition cut-off for the methyl-5 can be 50 mCGs.

In some embodiments, the epigenetic partition cut-off for the methyl-half score can be 5, 10, 15, 20, 25, 30, 35 or 40 mCGs. In some embodiments, the epigenetic partition cut-off for the methyl-half score can be 5 mCGs. In some embodiments, the epigenetic partition cut-off for the methyl-half score can be 10 mCGs. In some embodiments, the epigenetic partition cut-off for the methyl-half score can be 15 mCGs. In some embodiments, the epigenetic partition cut-off for the methyl-half score can be 20 mCGs. In some embodiments, the epigenetic partition cut-off for the methyl-half score can be 25 mCGs. In some embodiments, the epigenetic partition cut-off for the methyl-half score can be 30 mCGs. In some embodiments, the epigenetic partition cut-off for the methyl-half score can be 35 mCGs. In some embodiments, the epigenetic partition cut-off for the methyl-half score can be 40 mCGs.

In some embodiments, if the one or more epigenetic partition scores of the endogenous control molecules belonging to one or more epigenetic states in one or more partitioned sets is within the corresponding epigenetic partition cut-offs, then the partitioning method may be classified as being successful. Otherwise, the partitioning method may be classified as unsuccessful.

In another aspect, the present disclosure provides a method for determining the epigenetic state of nucleic acid molecule(s) in the sample of polynucleotides comprising: (a) adding a set of epigenetic-control nucleic acid molecules to the nucleic acid molecules in the sample of polynucleotides, whereby producing a spiked-in sample; (b) partitioning nucleic acid molecules of at least a subset of the spiked-in sample into a plurality of partitioned sets; (c) enriching at least a subset of molecules from the plurality of partitioned sets to generate a set of enriched molecules, wherein the set of enriched molecules comprises a group of epigenetic-control nucleic acid molecules and a group of nucleic acid molecules from the sample of polynucleotides; (d) sequencing at least a subset of the set of enriched molecules to produce a set of sequencing reads; (e) analyzing at least a subset the set of sequence reads to generate a plurality of partition profiles of the epigenetic-control nucleic acid molecules at different epigenetic states in the plurality of partitioned sets; and (f) using the plurality of partitioned profiles of epigenetic-control nucleic acid molecules to estimate a probability of epigenetic state of the nucleic acid molecules of the sample. In these embodiments, the partitioning of the nucleic acid molecules of the sample and the epigenetic-control nucleic acid molecules necessarily take place concurrently.

In some embodiments, the analyzing step comprises determining the number or fraction of epigenetic-control nucleic acid molecules per epigenetic state in the plurality of partitioned sets. The partition profile can refer to a representation of the fraction/number of epigenetic-control nucleic acid molecules at each epigenetic state in the two or more partitioned sets. In some embodiments, the partition profile further comprises information on the number of nucleotides with epigenetic modification in the epigenetic-control nucleic acid molecules, position of nucleotides with epigenetic modification in the epigenetic-control nucleic acid molecules and/or sequence composition of the epigenetic-control nucleic acid molecules. This partition profile can be used in estimating the probability of epigenetic state of the nucleic acid molecules in the sample. In some embodiments, if the epigenetic modification is methylation, then the partition profiles can be used in estimating the probability of methylation state (i.e., the level/degree of methylation or the number of methylated nucleotides) of the nucleic acid molecules of the sample.

In another aspect, the present disclosure provides a method for determining the epigenetic state of nucleic acid molecule(s) in the sample of polynucleotides comprising: (a) partitioning nucleic acid molecules from at least a subset of the sample into a plurality of partitioned sets; (b) enriching at least a subset of molecules from the plurality of partitioned sets to generate a set of enriched molecules, wherein the set of enriched molecules comprises a group of nucleic acid molecules from the sample of polynucleotides, wherein the group of nucleic acid molecules from the sample of polynucleotides comprises a set of endogenous control molecules; (c) sequencing at least a subset of the set of enriched molecules to produce a set of sequencing reads; (e) analyzing at least a subset the set of sequence reads to generate a plurality of partition profiles of the endogenous control molecules at different epigenetic states in the plurality of partitioned sets; and (f) using the plurality of partitioned profiles of endogenous control molecules to estimate a probability of epigenetic state of the nucleic acid molecules.

In some embodiments, the analyzing step comprises determining the number of endogenous control molecules per epigenetic state in the plurality of partitioned sets. The partition profile can refer to a representation of the fraction/number of endogenous control molecules at each epigenetic state in the two or more partitioned sets. In some embodiments, the partition profile further comprises information on the number of nucleotides with epigenetic modification in the epigenetic-control nucleic acid molecules, position of nucleotides with epigenetic modification in the epigenetic-control nucleic acid molecules and/or sequence composition of the epigenetic-control nucleic acid molecules. In some embodiments, the number of methylated CpGs in the endogenous control molecules are determined based on previous experimental data and/or from the literature. This partition profile can be used in estimating the probability of epigenetic state of the nucleic acid molecules in the sample. In some embodiments, if the epigenetic modification is methylation, then the partition profiles can be used in estimating the probability of methylation state (i.e., the level/degree of methylation or the number of methylated nucleotides) of the nucleic acid molecules of the sample.

In some embodiments, endogenous control molecules (e.g., hypermethylated control molecules and hypomethylated control molecules) can be used to estimate the methylation state of the nucleic acid molecules of the sample. If there are three partitioned sets - P1, P2 and P3, the partition profiles of the hypermethylated control molecules can be generated for P1, P2 and P3 based on the fraction of hypermethylated control molecules in each of the three partitioned sets and the number of methylated CpGs present in the hypermethylated control molecules. Likewise, for the hypomethylated control molecules, the partition profiles of the hypomethylated control molecules can be generated for P1, P2 and P3 based on the fraction of hypomethylated control molecules in each of the three partitioned sets and the number of unmethylated CpGs present in the hypomethylated control molecules. In some embodiments, where endogenous control molecules are used, the number of methylated CpGs in the endogenous control molecules are determined based on previous experimental data and/or from the literature. These six partition profiles can be used in estimating the probability of the level/degree of methylation or number of methylated nucleotides present in the nucleic acid molecules of the sample at a particular region.

In some embodiments, epigenetic-control nucleic acid molecules (e.g., highly methylated and low methylated epigenetic-control nucleic acid molecules) can be used to estimate the methylation state of the nucleic acid molecules of the sample. If there are three partitioned sets - P1, P2 and P3, the partition profiles of the highly methylated epigenetic-control nucleic acid molecules can be generated for P1, P2 and P3 based on the fraction of highly methylated epigenetic-control nucleic acid molecules in each of the three partitioned sets and the number of methylated CpGs present in the highly methylated epigenetic-control nucleic acid molecules. Likewise, for the low methylated epigenetic-control nucleic acid molecules the partition profiles of the low methylated epigenetic-control nucleic acid molecules can be generated for P1, P2 and P3 based on the fraction of low methylated epigenetic-control nucleic acid molecules in each of the three partitioned sets and the number of unmethylated CpGs present in the low methylated epigenetic-control nucleic acid molecules. These six partition profiles can be used in estimating the probability of the level/degree of methylation or number of methylated nucleotides present in the nucleic acid molecules of the sample at a particular region.

### II. Epigenetic-control nucleic acid molecules

Epigenetic-control nucleic acid molecules are used as control or reference molecules to evaluate the partitioning of the nucleic acid molecules in the sample based on an epigenetic modification. These epigenetic-control nucleic acid molecules can also be used to determine the epigenetic state of nucleic acid molecule(s) in the sample. For example, the epigenetic modification can be DNA methylation and the epigenetic-control nucleic acid molecules can have different/distinguishable levels of methylation. The epigenetic-control nucleic acid molecules can be synthetic oligonucleotides. In some embodiments, the epigenetic-control nucleic acid molecules can have a non-naturally occurring nucleic acid sequence. In some embodiments, the epigenetic-control nucleic acid molecules can have a naturally occurring nucleic acid sequence. In some embodiments, epigenetic-control nucleic acid molecules can have a nucleic acid sequence corresponding to a non-human genome. For example, these molecules can either have (i) a sequence corresponding to regions of lambda phage DNA or human genome, (ii) a non-naturally occurring sequence, and/or (iii) a combination of (i) and (ii). Also, the epigenetic-control nucleic acid molecules can be grouped into subsets and each subset can have a particular number of nucleotides representing the degree of epigenetic modification and that number is different from the number of nucleotides representing the degree of epigenetic modification in every other set.

In another aspect, the present disclosure provides a set of epigenetic-control nucleic acid molecules, comprising one or more subsets of epigenetic-control nucleic acid molecules, wherein each subset comprises a plurality of epigenetic-control nucleic acid molecules, and each epigenetic-control nucleic acid molecule comprises an epigenetic modification region. Epigenetic modification region is a region of the epigenetic-control nucleic acid molecule that represents the epigenetic state of the epigenetic-control nucleic acid molecule. The epigenetic state is the level/degree of epigenetic modification of the nucleic acid molecules. For example, if the epigenetic modification is DNA methylation, then the epigenetic state can refer to highly methylated, low methylated or intermediately methylated nucleic acid molecules. The epigenetic state can also refer to the number of nucleotides with epigenetic modification. For example, if the epigenetic modification is DNA methylation, then an epigenetic state can refer to the number of methylated nucleotides of the nucleic acid molecules.

In some embodiments, the epigenetic-control nucleic acid molecules comprise at least one of the following: (i) epigenetic modification region and (ii) identifier region. In some embodiments, the epigenetic modification region comprises nucleotides with epigenetic modification. In some embodiments, the epigenetic modification is DNA methylation. In those embodiments, the epigenetic modification region of the epigenetic-control nucleic acid molecules can have nucleotides that are methylated. The number of methylated nucleotides in the epigenetic modification region can vary among the epigenetic-control nucleic acid molecules. In some embodiments, the epigenetic-control nucleic acid molecules can have 0, 1, 2, 3, 4, 5, 6, 7, 8, 9, at least 10, at least 15, at least 20, at least 30, at least 40 or at least 50 methylated nucleotides in the epigenetic modification region. The epigenetic-control nucleic acid molecules can be grouped into subsets based on the epigenetic state (i.e., number of nucleotides with epigenetic modification/level of epigenetic modification) in the epigenetic modification region. The epigenetic modification region among the different subsets can be of same length, for example around 160 bp. The length of the epigenetic modification region between the subsets can be different. For example, epigenetic-control nucleic acid molecules can be grouped into three subsets (subset A, B and C) based on the number of methylated nucleotides in the epigenetic modification region. Subsets A, B and C can have epigenetic-control nucleic acid molecules with 5, 10 and 15 methylated nucleotides respectively in the epigenetic modification region and the length of the epigenetic modification region in subsets A, B and C can be same (e.g. 160 bp) or can be different - 100 bp, 150 bp and 200 bp for subsets A, B and C respectively.

In certain embodiments, the epigenetic-control nucleic acid molecules can be grouped into subsets with each subset representing a degree of epigenetic modification and the number of polynucleotides within each subset being different from the number of nucleotides in every other set. In some embodiments, the number of methylated nucleotides in the subset is 0, 1, 2, 3, 4, 5, 6, 7, 8, 9, 10, 11, at least 12, at least 15, at least 20, at least 25, at least 30, at least 40 or at least 50. In some embodiments, the epigenetic modification region of the epigenetic-control nucleic acid molecules in at least one subset comprises at least one nucleotide with epigenetic modification. In some embodiments, at least one subset of the epigenetic-control nucleic acid molecules can comprise nucleotides without any epigenetic modification (i.e., epigenetically unmodified nucleotides) in the epigenetic modification region of the epigenetic-control nucleic acid molecule.

In some embodiments, the epigenetic modification region of every epigenetic-control nucleic acid molecule within a subset comprises a same number of nucleotides with epigenetic modification. In some embodiments, the number of nucleotides with epigenetic modification in a first subset is different from the number of nucleotides with epigenetic modification in a second subset. In some embodiments, the epigenetic modification region of the plurality of epigenetic-control nucleic acid molecules in the one or more subsets comprises identical nucleic acid sequence. In some embodiments, the epigenetic modification region of the plurality of epigenetic-control nucleic acid molecules in a first subset comprises a nucleic acid sequence distinguishable from the nucleic acid sequence of the epigenetic modification region of the plurality of epigenetic-control nucleic acid molecules in a second subset.

In some embodiments, the epigenetic modification region of the epigenetic-control nucleic acid molecules in the one or more subsets can be of same length and have the same sequence composition but the number of nucleotides with epigenetic modification can be different in each of the one or more subsets. In some embodiments, the epigenetic modification region of the epigenetic-control nucleic acid molecules in the one or more subsets can be of same length and have same number of nucleotides with epigenetic modification but the position of the nucleotides with epigenetic modification can be different in each of the one or more subsets. In some embodiments, the epigenetic modification region of the epigenetic-control nucleic acid molecules in the one or more subsets can be of same length, have same number of nucleotides with epigenetic modification and position of the nucleotides with epigenetic modification can be the same but the adjacent nucleotides on either sides of the nucleotides with epigenetic modification can be different in each of the one or more subsets.

In some embodiments, each subset of epigenetic-control nucleic acid molecules is in equimolar concentration. In some embodiments, each subset of epigenetic-control nucleic acid molecules is in non-equimolar concentration. In some embodiment, epigenetic modification is DNA methylation. In some embodiments, the nucleotides with epigenetic modification comprise methylated nucleotides. In some embodiments, the methylated nucleotide comprises 5-methylcytosine. In some embodiments, the methylated nucleotide comprises 5-hydroxymethylcytosine. In some embodiments, the methylated nucleotide comprises N⁶-methyladenine.

In some embodiments, the epigenetic-control nucleic acid molecule further comprises an identifier region. The identifier region is a region of the epigenetic-control nucleic acid molecule that is used in distinguishing an epigenetic-control nucleic acid molecule from the other epigenetic-control nucleic acid molecules. The identifier region can have molecular barcodes and/or epigenetic state barcodes. The identifier region can be present in one or both the sides of the epigenetic modification region. The molecular barcode serves as the identifier of an epigenetic-control nucleic acid molecule whereas the epigenetic state barcode serves as the identifier of the epigenetic state of the epigenetic-control nucleic acid molecule. Epigenetic state barcode is a type of barcode (nucleic acid sequence) that is used to identify the epigenetic state of the epigenetic-control nucleic acid molecule. In some embodiments, epigenetic state barcode can identify (by predetermined correlation) the number of nucleotides with epigenetic modification in the epigenetic modification region of the epigenetic-control nucleic acid molecule. In some embodiments, the epigenetic state barcode can identify the level of epigenetic modification in the epigenetic modification region of the epigenetic-control nucleic acid molecule. In some embodiments, the identifier region of the epigenetic-control nucleic acid molecule comprises epigenetic state barcode. For example, if the epigenetic modification is DNA methylation and a subset of the epigenetic-control nucleic acid molecules have 5 methylated nucleotides, then all the epigenetic-control nucleic acid molecules within that subset with have the same epigenetic state barcode. In some embodiments, the epigenetic state barcode can be used to identify the level/degree of epigenetic modification of the epigenetic modification region of the epigenetic-control nucleic acid molecule. The epigenetic-control nucleic acid molecules can be grouped into subsets based on the number of cytosine or CpG nucleotides in the epigenetic modification region. In some embodiments, within each subset, the level of methylation can vary (e.g., highly methylated, intermediately methylated, and low methylated) and each level of methylation can have a separate epigenetic state barcode. For example, within subset A, all the epigenetic-control nucleic acid molecules that are low methylated with have an epigenetic state barcode- e.g. ESB1 and all the epigenetic-control nucleic molecules that are highly methylated with have another epigenetic state barcode - e.g. ESB3. In this example, the epigenetic state barcode is used to identify the level/degree of methylation. The molecular barcodes in the identifier region can be unique barcodes (each molecule has a unique barcode) or non-unique barcodes. The molecular barcodes can be of any length between 2 and 50 nucleotides. In some embodiments, the molecular barcodes can be at least 2, at least 3, at least 4, at least 5, at least 6, at least 7, at least 8, at least 9, or at least 10 nucleotides. In some embodiments, the epigenetic state barcode can be at least 2, at least 3, at least 4, at least 5, at least 6, at least 7 or at least 8 nucleotides.

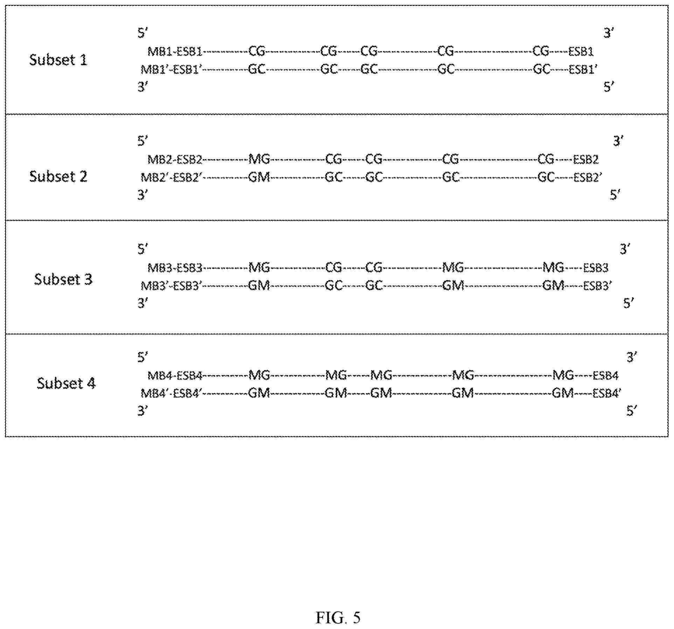

FIG. 5 is a schematic representation of epigenetic-control nucleic acid molecules suitable for use with some embodiments of the disclosure. The epigenetic-control nucleic acid molecules described here has a length similar to that of the sample being assayed and all the subsets have the same sequence composition to reduce any sequence-specific partitioning effects. In FIG. 5, as an example, the epigenetic-control nucleic acid molecules have been grouped into four subsets - Subset 1, 2, 3 and 4. The epigenetic-control nucleic acid molecules in FIG. 5 is a double-stranded DNA molecule. For illustration purposes, only one representation of the epigenetic-control nucleic acid molecules in each subset is shown in the figure. In this embodiment, the sequence of the epigenetic modification region of the epigenetic-control nucleic acid molecules is the same in all the subsets. The epigenetic modification region of epigenetic-control nucleic acid molecules in all the four subsets has 5 CpG dyads. '---' region in the double-stranded DNA sequence represents any other sequence apart from CpG dyad and M represents 5-methylcytosine, C represents cytosine and G represents guanine. In FIG. 5, the epigenetic state (level of methylation) of the epigenetic-control nucleic acid molecules in a subset is different from the epigenetic state of the other subsets. Subset 1 has zero methylated CpG dyad, Subset 2 has 1 fully methylated CpG dyad, Subset 3 has 3 fully methylated CpG dyads and Subset 4 has 5 fully methylated CpG dyads. In this embodiment, the identifier region is on both sides of the epigenetic modification region. The identifier region on both the sides have epigenetic state barcode (ESB) whereas the molecular barcode (MB) is on one side only. Molecular barcode is used as an identifier of the epigenetic-control nucleic acid molecule and each epigenetic-control nucleic acid molecule has a unique molecular barcode (i.e, molecule 1 has MB1, molecule 2 has MB2, molecule 3 has MB3 and so forth). An epigenetic state barcode may be used as an identifier of the epigenetic state of the epigenetic-control nucleic acid molecule. Here, epigenetic state barcode is used to identify the number of fully methylated CpG dyads in the epigenetic-control nucleic acid molecule. All the epigenetic-control nucleic acid molecules of subset 1 have zero methylated CpG dyads, so all the epigenetic-control nucleic acid molecules of Subset 1 have the same epigenetic state barcode - ESB1. Likewise, all the epigenetic-control nucleic acid molecules of subsets 2, 3 and 4 have 1, 3 and 5 fully methylated CpG dyads respectively. So, all the epigenetic-control nucleic acid molecules of Subsets 2, 3 and 4 have an epigenetic state barcode of ESB2, ESB3 and ESB4 respectively. In this example, the same epigenetic state barcode is on both the sides of the epigenetic modification region.

In some embodiments, the molecular barcode can be on one or both the sides of the epigenetic modification region. In some embodiments, the epigenetic state barcode can be on one or both the sides of the epigenetic modification region. In some embodiments, the epigenetic state barcode on both the sides of the epigenetic modification region can be the same or different and/or can be randomly attached.

In some embodiments, the identifier region can have an additional region facilitating binding of one or more primers (primer binding sites). In some embodiments, the primer binding sites of the identifier region in one subset is different from the primer binding sites in the other subsets. In some embodiments, if within a subset, the epigenetic-control nucleic acid molecules have different epigenetic states, then the primer binding sites can be different for each epigenetic state within the molecules i.e., each unique epigenetic state has a unique primer binding site. In some embodiments, these primer binding sites are used in analyzing the partitioning of the epigenetic-control nucleic acid molecules. In some embodiment, instead of analyzing the partitioning of the epigenetic-control nucleic acid molecules by sequencing, the partitioning of the epigenetic-control nucleic acid molecules can be analyzed by digital droplet PCR (ddPCR) using primers that bind to these primer binding states.

In some embodiments, the epigenetic-control nucleic acid molecules can be grouped into subsets such that the epigenetic-control nucleic acid molecules within each subset have the sequence but the epigenetic states of the epigenetic-control nucleic acid molecules within each subset can vary.

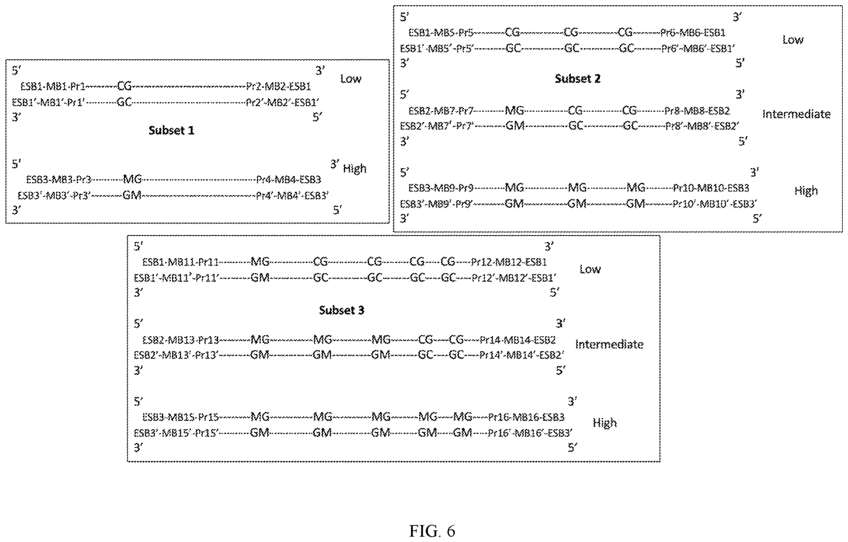

FIG. 6 is a schematic representation of epigenetic-control nucleic acid molecules that may be suitable for use with certain embodiments of the disclosure. The epigenetic-control nucleic acid molecules described herein may also take into account the influence of sequence composition and number of CpG dyads/fully methylated CpG dyads during partitioning of nucleic acid molecules. In FIG. 6, as an example, the epigenetic-control nucleic acid molecules have been grouped into three subsets - subset 1, 2 and 3. The epigenetic-control nucleic acid molecules in FIG. 6 is a double-stranded DNA molecule. For illustration purposes, only one representation of the epigenetic-control nucleic acid molecules for every epigenetic state in each subset is shown in the figure. In this embodiment, the epigenetic modification region of the epigenetic-control nucleic acid molecules in subsets 1, 2 and 3 are of different length. The epigenetic modification region of epigenetic-control nucleic acid molecules in subsets 1, 2 and 3 have 1, 3 and 5 CpG dyads respectively. '---' region in the double-stranded DNA sequence represents any other sequence apart from CpG dyad and M represents 5-methylcytosine, C represents cytosine and G represents guanine. In FIG. 6, within each subset, epigenetic-control nucleic acid molecules are in different epigenetic states - e.g., low methylated, intermediately methylated and highly methylated states. The epigenetic-control nucleic acid molecules of subset 1 are in two different epigenetic states - low methylated (zero methylated CpG dyad) and highly methylated (1 fully methylated CpG dyad). The epigenetic-control nucleic acid molecules of subset 2 are in three different epigenetic states - low methylated (zero methylated CpG dyad), intermediately methylated (1 fully methylated CpG dyad) and highly methylated (3 fully methylated CpG dyads). The epigenetic-control nucleic acid molecules of subset 3 are in three different epigenetic states - low methylated (1 fully methylated CpG dyad), intermediately methylated (3 fully methylated CpG dyads) and highly methylated (5 fully methylated CpG dyads). Here, the identifier region is on both sides of the epigenetic modification region. The identifier region on both the sides have an epigenetic state barcode (ESB) and a molecular barcode (MB). Molecular barcode is used as an identifier of the epigenetic-control nucleic acid molecule and each epigenetic-control nucleic acid molecule has a unique molecular barcode (i.e., molecule 1 has MB1, molecule 2 has MB2, molecule 3 has MB3 and so forth). Epigenetic state barcode is used as an identifier of the epigenetic state of the epigenetic-control nucleic acid molecule. Here, the epigenetic state barcode is used to identify the degree/level of methylation of the epigenetic-control nucleic acid molecules i.e. low methylated, intermediately methylated or highly methylated states. All low methylated epigenetic-control nucleic acid molecules in subsets 1, 2 and 3 have the same epigenetic state barcode - ESB1. Subsets 2 and 3 have intermediately methylated epigenetic-control nucleic acid molecules and all these molecules have the same epigenetic state barcode - ESB2 (subset 1 has no intermediately methylated state, so none of the epigenetic-control nucleic molecules will have ESB2 epigenetic state barcode). So, from the sequence of epigenetic-control nucleic acid molecule and sequence of the epigenetic state barcode, the epigenetic state of the epigenetic-control nucleic acid molecule and the subset to which the epigenetic-nucleic acid molecule belongs to can be identified.

Additionally, the identifier region may have primer binding sites. The different primer binding sites may be used for differentiating the different epigenetic states within each subset and between the subsets. For example, low methylated epigenetic-control nucleic acid molecules in subset 1 may have the primer binding sites - Pr1 and Pr2 on either sides of the epigenetic modification region. High methylated epigenetic-control nucleic acid molecules in subset 1 may have the primer binding sites - Pr3 and Pr4 on either sides of the epigenetic modification region. Likewise, in subset 2, low, intermediate and high methylated epigenetic-control nucleic acid molecules may have the primer binding sites Pr5 & Pr6, P7 & Pr8 and Pr9 & Pr 19, respectively, on either sides of the epigenetic modification region. Similarly, in subset 3, the low, intermediate and high methylated epigenetic-control nucleic acid molecules may have the primer binding sites Pr11 & Pr12, P13 & Pr14 and Pr15 & Pr16, respectively, on either sides of the epigenetic modification region. Also, from the distinct primer sets used for the different epigenetic state molecules in different subsets, one can estimate a measure of the number of epigenetic-control nucleic acid molecules belonging to particular epigenetic state in a particular subset by either ddPCR or quantitative PCR (qPCR). In this embodiment, from the epigenetic state barcode sequence and the sequence of the epigenetic modification region, the number of CpG dyads in the epigenetic modification region and the number of fully methylated CpG dyads in the epigenetic modification region can be determined.

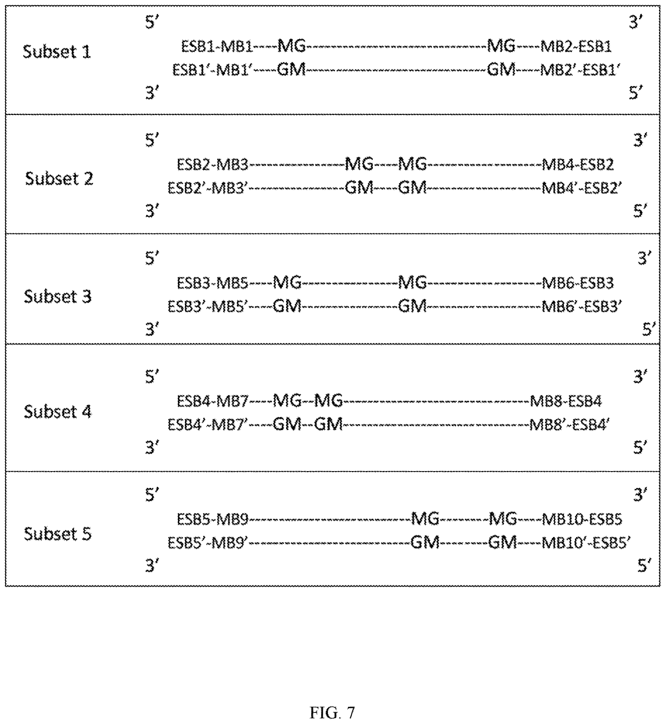

FIG. 7 is a schematic representation of epigenetic-control nucleic acid molecules suitable for use with some embodiments of the disclosure. The epigenetic-control nucleic acid molecules described herein may take into account of the position-specific effects of the fully methylated CpG dyads during partitioning of the nucleic acid molecules. In FIG. 7, the epigenetic-control nucleic acid molecules are grouped into five subsets. The sequence length and sequence composition of the epigenetic modification region of the epigenetic-control nucleic acid molecules is same in all the subsets. Each subset has two fully methylated CpG dyads but the position of the two fully methylated CpG dyads varies with subsets (i.e., the distance between the two fully methylated CpG dyads varies with subsets). In subset 1, the two fully methylated CpG dyads are far apart whereas in subset 4, the two fully methylated CpG dyads very close to each other. Here, the identifier region is on both sides of the epigenetic modification region. The identifier region on both the sides have an epigenetic state barcode (ESB) and a molecular barcode (MB). Molecular barcode is used as an identifier of the individual epigenetic-control nucleic acid molecule and each epigenetic-control nucleic acid molecule has a unique molecular barcode i.e., molecule 1 has MB1, molecule 2 has MB2, molecule 3 has MB3 and so forth. These subsets will have different binding affinities based on the influence of the fully methylated CpG dyads positions. Here, the epigenetic state barcode can be used to identify the position of fully methylated CpG dyads. All the epigenetic-control nucleic acid molecules of subset 1 have two fully methylated CpG dyads in the same position, so the epigenetic-control nucleic acid molecules of subset 1 have the same epigenetic state barcode - ESB1. Likewise, all the epigenetic-control nucleic acid molecules of subsets 2, 3, 4 and 5 have an epigenetic state barcode of ESB2, ESB3 and ESB4 respectively. In this example, the same epigenetic state barcode is on both the sides of the epigenetic modification region.

In another aspect, the present disclosure provides a population of nucleic acids, comprising: a set of epigenetic-control nucleic acid molecules, wherein the set of epigenetic-control nucleic acid molecules comprises one or more subsets of epigenetic-control nucleic acid molecules, wherein each subset comprises plurality of epigenetic-control nucleic acid molecules, and each epigenetic-control nucleic acid molecule comprises an epigenetic modification region; and a set of nucleic acid molecules in a sample of polynucleotides from a subject.

In some embodiments, epigenetic-control nucleic acid molecules can either have (i) a sequence corresponding to regions of lambda phage DNA or human genome, (ii) a non-naturally occurring sequence, and/or (iii) a combination of (i) and (ii). In some embodiments, the epigenetic-control nucleic acid molecules can comprise a non-naturally occurring sequence.

In some embodiments, the sample of polynucleotides is a sample of DNA, a sample of RNA, a sample of cell-free polynucleotides, a sample of cell-free DNA or a sample of cell-free RNA. In some embodiments, the sample of polynucleotides is a sample of cell-free DNA.

In some embodiments, the cell-free DNA is at least at least 1 ng, at least 5 ng, at least 10 ng, at least 15 ng, at least 20 ng, at least 30 ng, at least 50 ng, at least 75 ng, at least 100 ng, at least 150 ng, at least 200 ng, at least 250 ng, at least 300 ng, at least 350 ng, at least 400 ng, at least 450 ng or at least 500 ng.

In some embodiments, the amount of epigenetic-control nucleic acid molecules is at least 1 femtomoles, at least 2 femtomoles, at least 5 femtomoles, at least 10 femtomoles, at least 15 femtomoles, at least 20 femtomoles, at least 50 femtomoles, at least 75 femtomoles, at least 100 femtomoles, at least 125 femtomoles, at least 150 femtomoles or at least 200 femtomoles.

### III. General Features of the Methods

### A. Samples

A sample can be any biological sample isolated from a subject. Samples can include body tissues, whole blood, platelets, serum, plasma, stool, red blood cells, white blood cells or leucocytes, endothelial cells, tissue biopsies (e.g., biopsies from known or suspected solid tumors), cerebrospinal fluid, synovial fluid, lymphatic fluid, ascites fluid, interstitial or extracellular fluid (e.g., fluid from intercellular spaces), gingival fluid, crevicular fluid, bone marrow, pleural effusions, cerebrospinal fluid, saliva, mucous, sputum, semen, sweat, and urine. Samples may be bodily fluids, such as blood and fractions thereof, and urine. Such samples can include nucleic acids shed from tumors. The nucleic acids can include DNA and RNA and can be in double and single-stranded forms. A sample can be in the form originally isolated from a subject or can have been subjected to further processing to remove or add components, such as cells, enrich for one component relative to another, or convert one form of nucleic acid to another, such as RNA to DNA or single-stranded nucleic acids to double-stranded. Thus, for example, a bodily fluid for analysis can be plasma or serum containing cell-free nucleic acids, e.g., cell-free DNA (cfDNA).

In some embodiments, the sample volume of bodily fluid taken from a subject depends on the desired read depth for sequenced regions. Examples of volumes are about 0.4-40 milliliters (mL), about 5-20 mL, about 10-20 mL. For example, the volume can be about 0.5 mL, about 1 mL, about 5 mL, about 10 mL, about 20 mL, about 30 mL, about 40 mL, or more milliliters. A volume of sampled plasma is typically between about 5 mL to about 20 mL.

The sample can comprise various amounts of nucleic acid. Typically, the amount of nucleic acid in a given sample is equates with multiple genome equivalents. For example, a sample of about 30 nanograms (ng) DNA can contain about 10,000 (10⁴) haploid human genome equivalents and, in the case of cfDNA, about 200 billion (2 x 10¹¹) individual polynucleotide molecules. Similarly, a sample of about 100 ng of DNA can contain about 30,000 haploid human genome equivalents and, in the case of cfDNA, about 600 billion individual molecules.

In some embodiments, a sample comprises nucleic acids from different sources, e.g., from cells and from cell-free sources (e.g., blood samples, etc.). Typically, a sample includes nucleic acids carrying mutations. For example, a sample optionally comprises DNA carrying germline mutations and/or somatic mutations. Typically, a sample comprises DNA carrying cancer-associated mutations (e.g., cancer-associated somatic mutations).

Example amounts of cell-free nucleic acids in a sample before amplification typically range from about 1 femtogram (fg) to about 1 microgram (µg), e.g., about 1 picogram (pg) to about 200 nanograms (ng), about 1 ng to about 100 ng, about 10 ng to about 1000 ng. In some embodiments, a sample includes up to about 600 ng, up to about 500 ng, up to about 400 ng, up to about 300 ng, up to about 200 ng, up to about 100 ng, up to about 50 ng, or up to about 20 ng of cell-free nucleic acid molecules. Optionally, the amount is at least about 1 fg, at least about 10 fg, at least about 100 fg, at least about 1 pg, at least about 10 pg, at least about 100 pg, at least about 1 ng, at least about 10 ng, at least about 100 ng, at least about 150 ng, or at least about 200 ng of cell-free nucleic acid molecules. In some embodiments, the amount is up to about 1 fg, about 10 fg, about 100 fg, about 1 pg, about 10 pg, about 100 pg, about 1 ng, about 10 ng, about 100 ng, about 150 ng, or about 200 ng of cell-free nucleic acid molecules. In some embodiments, methods include obtaining between about 1 fg to about 200 ng cell-free nucleic acid molecules from samples.

Cell-free nucleic acids typically have a size distribution of between about 100 nucleotides in length and about 500 nucleotides in length, with molecules of about 110 nucleotides in length to about 230 nucleotides in length representing about 90% of molecules in the sample, with a mode of about 168 nucleotides length (in samples from human subjects) and a second minor peak in a range between about 240 nucleotides to about 440 nucleotides in length. In some embodiments, cell-free nucleic acids are from about 160 nucleotides to about 180 nucleotides in length, or from about 320 nucleotides to about 360 nucleotides in length, or from about 440 nucleotides to about 480 nucleotides in length.

In some embodiments, cell-free nucleic acids are isolated from bodily fluids through a partitioning step in which cell-free nucleic acids, as found in solution, are separated from intact cells and other non-soluble components of the bodily fluid. In some embodiments, partitioning includes techniques such as centrifugation or filtration. Alternatively, cells in bodily fluids may be lysed, and cell-free and cellular nucleic acids may be processed together. Generally, after addition of buffers and wash steps, cell-free nucleic acids may be precipitated with, for example, an alcohol. In some embodiments, additional clean-up steps are used, such as silica-based columns to remove contaminants or salts. Non-specific bulk carrier nucleic acids, for example, are optionally added throughout the reaction to optimize aspects of the example procedure, such as yield. After such processing, samples typically include various forms of nucleic acids including double-stranded DNA, single-stranded DNA and/or single-stranded RNA. Optionally, single-stranded DNA and/or single-stranded RNA are converted to double-stranded forms so that they are included in subsequent processing and analysis steps.

### B. Tagging

In some embodiments, the nucleic acid molecules (from the sample of polynucleotides) may be tagged with sample indexes and/or molecular barcodes (referred to generally as "tags"). Tags may be incorporated into or otherwise joined to adapters by chemical synthesis, ligation (e.g., blunt-end ligation or sticky-end ligation), or overlap extension polymerase chain reaction (PCR), among other methods. Such adapters may be ultimately joined to the target nucleic acid molecule. In other embodiments, one or more rounds of amplification cycles (e.g., PCR amplification) are generally applied to introduce sample indexes to a nucleic acid molecule using conventional nucleic acid amplification methods. The amplifications may be conducted in one or more reaction mixtures (e.g., a plurality of microwells in an array). Molecular barcodes and/or sample indexes may be introduced simultaneously, or in any sequential order. In some embodiments, molecular barcodes and/or sample indexes are introduced prior to and/or after sequence capturing steps are performed. In some embodiments, only the molecular barcodes are introduced prior to probe capturing and the sample indexes are introduced after sequence capturing steps are performed. In some embodiments, both the molecular barcodes and the sample indexes are introduced prior to performing probe-based capturing steps. In some embodiments, the sample indexes are introduced after sequence capturing steps are performed. In some embodiments, molecular barcodes are incorporated to the nucleic acid molecules (e.g. cfDNA molecules) in a sample through adapters via ligation (e.g., blunt-end ligation or sticky-end ligation). In some embodiments, sample indexes are incorporated to the nucleic acid molecules (e.g. cfDNA molecules) in a sample through overlap extension polymerase chain reaction (PCR). Typically, sequence capturing protocols involve introducing a single-stranded nucleic acid molecule complementary to a targeted nucleic acid sequence, e.g., a coding sequence of a genomic region and mutation of such region is associated with a cancer type.

In some embodiments, the tags may be located at one end or at both ends of the sample nucleic acid molecule. In some embodiments, tags are predetermined or random or semi-random sequence oligonucleotides. In some embodiments, the tags may be less than about 500, 200, 100, 50, 20, 10, 9, 8, 7, 6, 5, 4, 3, 2, or 1 nucleotides in length. The tags may be linked to sample nucleic acids randomly or non-randomly.

In some embodiments, each sample is uniquely tagged with a sample index or a combination of sample indexes. In some embodiments, each nucleic acid molecule of a sample or sub-sample is uniquely tagged with a molecular barcode or a combination of molecular barcodes. In other embodiments, a plurality of molecular barcodes may be used such that molecular barcodes are not necessarily unique to one another in the plurality (e.g., non-unique molecular barcodes). In these embodiments, molecular barcodes are generally attached (e.g., by ligation) to individual molecules such that the combination of the molecular barcode and the sequence it may be attached to creates a unique sequence that may be individually tracked. Detection of non-uniquely tagged molecular barcodes in combination with endogenous sequence information (e.g., the beginning (start) and/or end (stop) genomic location/position corresponding to the sequence of the original nucleic acid molecule in the sample, sub-sequences of sequence reads at one or both ends, length of sequence reads, and/or length of the original nucleic acid molecule in the sample) typically allows for the assignment of a unique identity to a particular molecule. In some embodiments, detection of non-uniquely tagged molecular barcodes in combination with endogenous sequence information (e.g., the beginning (start) and/or end (stop) region of the alignment of the sequence reads to the reference sequence, sub-sequences of sequence reads at one or both ends, length of sequence reads, and/or length of the original nucleic acid molecule in the sample) typically allows for the assignment of a unique identity to a particular molecule. In some embodiments, the beginning region comprises a genomic start position of the sequencing read at which the 5' end of the sequencing read is determined to start aligning to reference sequence and the end region comprises a genomic stop position of the sequencing read at which the 3' end of the sequencing read is determined to stop aligning to the reference sequence. In some embodiments, beginning region comprises the first 1, first 2, the first 5, the first 10, the first 15, the first 20, the first 25, the first 30 or at least the first 30 base positions at the 5' end of the sequencing read that align to the reference sequence. In some embodiments, the end region comprises the last 1, last 2, the last 5, the last 10, the last 15, the last 20, the last 25, the last 30 or at least the last 30 base positions at the 3' end of the sequencing read that align to the reference sequence.

The length, or number of base pairs, of an individual sequence read are also optionally used to assign a unique identity to a given molecule. As described herein, fragments from a single strand of nucleic acid having been assigned a unique identity, may thereby permit subsequent identification of fragments from the parent strand, and/or a complementary strand.

In some embodiments, molecular barcodes are introduced at an expected ratio of a set of identifiers (e.g., a combination of unique or non-unique molecular barcodes) to molecules in a sample. One example format uses from about 2 to about 1,000,000 different molecular barcode sequences, or from about 5 to about 150 different molecular barcode sequences, or from about 20 to about 50 different molecular barcode sequences, ligated to both ends of a target molecule. Alternatively, from about 25 to about 1,000,000 different molecular barcode sequences may be used. For example, 20-50 x 20-50 molecular barcode sequences (i.e., one of the 20-50 different molecular barcode sequences can be attached to each end of the target molecule) can be used. Such numbers of identifiers are typically sufficient for different molecules having the same start and stop points to have a high probability (e.g., at least 94%, 99.5%, 99.99%, or 99.999%) of receiving different combinations of identifiers. In some embodiments, about 80%, about 90%, about 95%, or about 99% of molecules have the same combinations of molecular barcodes.

In some embodiments, the assignment of unique or non-unique molecular barcodes in reactions is performed using methods and systems described in, for example, U.S. Patent Application Nos. 20010053519, 20030152490, and 20110160078, and U.S. Patent Nos. 6,582,908, 7,537,898, 9,598,731, and 9,902,992. Alternatively, in some embodiments, different nucleic acid molecules of a sample may be identified using only endogenous sequence information (e.g., start and/or stop positions, sub-sequences of one or both ends of a sequence, and/or lengths).

An epigenetic state barcode (ESB) is a type of tag that is attached to the epigenetic modification region of the epigenetic-control nucleic acid molecules. The ESB may be used as an identifier of the epigenetic state of the epigenetic-control nucleic acid molecule. It can refer to the number of nucleotides with epigenetic modification in the epigenetic modification region of the epigenetic-control nucleic acid molecule. In some embodiments, the identifier region of the epigenetic-control nucleic acid molecule comprises at least one epigenetic state barcode. In some embodiments, the ESB is a part of the identifier region of the epigenetic-control nucleic acid molecule. For example, if the epigenetic modification is DNA methylation and a subset of the epigenetic-control nucleic acid molecules have 5 methylated nucleotides, then all the epigenetic-control nucleic acid molecules within that subset with have the same epigenetic state barcode. In some embodiments, the epigenetic state barcode can be used to identify the level/degree of epigenetic modification of the epigenetic modification region of the epigenetic-control nucleic acid molecule. The epigenetic-control nucleic acid molecules can be grouped into subsets based on the number of cytosine or CpG nucleotides in the epigenetic modification region. In some embodiments, within each subset, the level of methylation can vary (for e.g., highly methylated, intermediately methylated and low methylated) and each level of methylation can have a separate epigenetic state barcode. For example, within subset A, all the epigenetic-control nucleic acid molecules that are low methylated with have an epigenetic state barcode- e.g. ESB1 and all the epigenetic-control nucleic molecules that are highly methylated with have another epigenetic state barcode - e.g. ESB3. In this example, the epigenetic state barcode is used to identify the level/degree of methylation.

In some embodiments, the assignment of unique or non-unique molecular barcodes in reactions is performed using methods and systems described in, for example, U.S. Patent Application Nos. 20010053519, 20030152490, and 20110160078, and U.S. Patent Nos. 6,582,908, 7,537,898, 9,598,731, and 9,902,992.

### C. Amplification

Sample nucleic acids may be flanked by adapters and amplified by PCR and other amplification methods using nucleic acid primers binding to primer binding sites in adapters flanking a DNA molecule to be amplified. In some embodiments, amplification methods involve cycles of extension, denaturation, and annealing resulting from thermocycling, or can be isothermal as, for example, in transcription mediated amplification. Other examples of amplification methods that may be optionally utilized include the ligase chain reaction, strand displacement amplification, nucleic acid sequence-based amplification, and self-sustained sequence-based replication.

Typically, the amplification reactions generate a plurality of non-uniquely or uniquely tagged nucleic acid amplicons with molecular barcodes and sample indexes at size ranging from about 150 nucleotides (nt), to about 700 nt, from 250 nt to about 350 nt, or from about 320 nt to about 550 nt. In some embodiments, the amplicons have a size of about 180 nt. In some embodiments, the amplicons have a size of about 200 nt.

### D. Enrichment

In some embodiments, sequences are enriched prior to sequencing the nucleic acids. Enrichment optionally performed for specific target regions or nonspecifically ("target sequences"). In some embodiments, targeted regions of interest may be enriched with nucleic acid capture probes ("baits") selected for one or more bait set panels using a differential tiling and capture scheme. A differential tiling and capture scheme generally uses bait sets of different relative concentrations to differentially tile (e.g., at different "resolutions") across genomic regions associated with the baits, subject to a set of constraints (e.g., sequencer constraints such as sequencing load, utility of each bait, etc.), and capture the targeted nucleic acids at a desired level for downstream sequencing. These targeted genomic regions of interest optionally include natural or synthetic nucleotide sequences of the nucleic acid construct. In some embodiments, biotin-labeled beads with probes to one or more regions of interest can be used to capture target sequences, and optionally followed by amplification of those regions, to enrich for the regions of interest.

Sequence capture typically involves the use of oligonucleotide probes that hybridize to the target nucleic acid sequence. In some embodiments, a probe set strategy involves tiling the probes across a region of interest. Such probes can be, for example, from about 60 to about 120 nucleotides in length. The set can have a depth (e.g., depth of coverage) of about 2X, 3X, 4X, 5X, 6X, 7X, 8X, 9X, 10X, 15X, 20X, 50X, or more than 50X. The effectiveness of sequence capture generally depends, in part, on the length of the sequence in the target molecule that is complementary (or nearly complementary) to the sequence of the probe.

### E. Sequencing

Sample nucleic acids, optionally flanked by adapters, with or without prior amplification are generally subjected to sequencing. Sequencing methods or commercially available formats that are optionally utilized include, for example, Sanger sequencing, high-throughput sequencing, pyrosequencing, sequencing-by-synthesis, single-molecule sequencing, nanopore-based sequencing, semiconductor sequencing, sequencing-by-ligation, sequencing-by-hybridization, RNA-Seq (Illumina), Digital Gene Expression (Helicos), next generation sequencing (NGS), Single Molecule Sequencing by Synthesis (SMSS) (Helicos), massively-parallel sequencing, Clonal Single Molecule Array (Solexa), shotgun sequencing, Ion Torrent, Oxford Nanopore, Roche Genia, Maxim-Gilbert sequencing, primer walking, sequencing using PacBio, SOLiD, Ion Torrent, or Nanopore platforms. Sequencing reactions can be performed in a variety of sample processing units, which may include multiple lanes, multiple channels, multiple wells, or other means of processing multiple sample sets substantially simultaneously. Sample processing units can also include multiple sample chambers to enable the processing of multiple runs simultaneously.

The sequencing reactions can be performed on one or more nucleic acid fragment types or regions known to contain markers of cancer or of other diseases. The sequencing reactions can also be performed on any nucleic acid fragment present in the sample. The sequence reactions may be performed on at least about 5%, 10%, 15%, 20%, 25%, 30%, 40%, 50%, 60%, 70%, 80%, 90%, 95%, 99%, 99.9%, or 100% of the genome. In other cases, sequence reactions may be performed on less than about 5%, 10%, 15%, 20%, 25%, 30%, 40%, 50%, 60%, 70%, 80%, 90%, 95%, 99%, 99.9%, or 100% of the genome.

Simultaneous sequencing reactions may be performed using multiplex sequencing techniques. In some embodiments, cell-free polynucleotides are sequenced with at least about 1000, 2000, 3000, 4000, 5000, 6000, 7000, 8000, 9000, 10000, 50000, or 100,000 sequencing reactions. In other embodiments, cell-free polynucleotides are sequenced with less than about 1000, 2000, 3000, 4000, 5000, 6000, 7000, 8000, 9000, 10000, 50000, or 100,000 sequencing reactions. Sequencing reactions are typically performed sequentially or simultaneously. Subsequent data analysis is generally performed on all or part of the sequencing reactions. In some embodiments, data analysis is performed on at least about 1000, 2000, 3000, 4000, 5000, 6000, 7000, 8000, 9000, 10000, 50000, or 100,000 sequencing reactions. In other embodiments, data analysis may be performed on less than about 1000, 2000, 3000, 4000, 5000, 6000, 7000, 8000, 9000, 10000, 50000, or 100,000 sequencing reactions. An example of a read depth is from about 1000 to about 50000 reads per locus (e.g., base position).

### F. Analysis

Sequencing may generate a plurality of sequencing reads or reads. Sequencing reads or reads may include sequences of nucleotide data less than about 150 bases in length, or less than about 90 bases in length. In some embodiments, reads are between about 80 bases and about 90 bases, e.g., about 85 bases in length. In some embodiments, methods of the present disclosure are applied to very short reads, e.g., less than about 50 bases or about 30 bases in length. Sequencing read data can include the sequence data as well as meta information. Sequence read data can be stored in any suitable file format including, for example, VCF files, FASTA files, or FASTQ files.

FASTA may refer to a computer program for searching sequence databases, and the name FASTA may also refer to a standard file format. For example, FASTA is described by, for example, Pearson & Lipman, 1988, Improved tools for biological sequence comparison, PNAS 85:2444-2448. A sequence in FASTA format begins with a single-line description, followed by lines of sequence data. The description line is distinguished from the sequence data by a greater-than (">") symbol in the first column. The word following the ">" symbol is the identifier of the sequence, and the rest of the line is the description (both are optional). There should be no space between the ">" and the first letter of the identifier. It is recommended that all lines of text be shorter than 80 characters. The sequence ends if another line starting with a ">" appears; this indicates the start of another sequence.

The FASTQ format is a text-based format for storing both a biological sequence (usually nucleotide sequence) and its corresponding quality scores. It is similar to the FASTA format but with quality scores following the sequence data. Both the sequence letter and quality score are encoded with a single ASCII character for brevity. The FASTQ format is a *de facto* standard for storing the output of high throughput sequencing instruments such as the Illumina Genome Analyzer, as described by, for example, Cock et al. ("The Sanger FASTQ file format for sequences with quality scores, and the SolexalIllumina FASTQ variants," Nucleic Acids Res 38(6):1767-1771, 2009).

For FASTA and FASTQ files, meta information includes the description line and not the lines of sequence data. In some embodiments, for FASTQ files, the meta information includes the quality scores. For FASTA and FASTQ files, the sequence data begins after the description line and is present typically using some subset of IUPAC ambiguity codes optionally with "-". In an embodiment, the sequence data may use the A, T, C, G, and N characters, optionally including "-" or U as-needed (e.g., to represent gaps or uracil).

In some embodiments, the at least one master sequence read file and the output file are stored as plain text files (e.g., using encoding such as ASCII; ISO/IEC 646; EBCDIC; UTF-8; or UTF-16). A computer system provided by the present disclosure may include a text editor program capable of opening the plain text files. A text editor program may refer to a computer program capable of presenting contents of a text file (such as a plain text file) on a computer screen, allowing a human to edit the text (e.g., using a monitor, keyboard, and mouse). Examples of text editors include, without limitation, Microsoft Word, emacs, pico, vi, BBEdit, and TextWrangler. The text editor program may be capable of displaying the plain text files on a computer screen, showing the meta information and the sequence reads in a human-readable format (e.g., not binary encoded but instead using alphanumeric characters as they may be used in print or human writing).

While methods have been discussed with reference to FASTA or FASTQ files, methods and systems of the present disclosure may be used to compress any suitable sequence file format including, for example, files in the Variant Call Format (VCF) format. A typical VCF file may include a header section and a data section. The header contains an arbitrary number of meta-information lines, each starting with characters `##', and a TAB delimited field definition line starting with a single '#' character. The field definition line names eight mandatory columns and the body section contains lines of data populating the columns defined by the field definition line. The VCF format is described by, for example, Danecek et al. ("The variant call format and VCF tools," Bioinformatics 27(15):2156-2158, 2011). The header section may be treated as the meta information to write to the compressed files and the data section may be treated as the lines, each of which will be stored in a master file only if unique.

Some embodiments provide for the assembly of sequencing reads. In assembly by alignment, for example, the sequencing reads are aligned to each other or aligned to a reference sequence. By aligning each read, in turn to a reference genome, all of the reads are positioned in relationship to each other to create the assembly. In addition, aligning or mapping the sequencing read to a reference sequence can also be used to identify variant sequences within the sequencing read. Identifying variant sequences can be used in combination with the methods and systems described herein to further aid in the diagnosis or prognosis of a disease or condition, or for guiding treatment decisions.

In some embodiments, any or all of the steps are automated. Alternatively, methods of the present disclosure may be embodied wholly or partially in one or more dedicated programs, for example, each optionally written in a compiled language such as C++, then compiled and distributed as a binary. Methods of the present disclosure may be implemented wholly or in part as modules within, or by invoking functionality within, existing sequence analysis platforms. In some embodiments, methods of the present disclosure include a number of steps that are all invoked automatically responsive to a single starting cue (e.g., one or a combination of triggering events sourced from human activity, another computer program, or a machine). Thus, the present disclosure provides methods in which any or the steps or any combination of the steps can occur automatically responsive to a cue. "Automatically" generally means without intervening human input, influence, or interaction (e.g., responsive only to original or pre-cue human activity).

The methods of the present disclosure may also encompass various forms of output, which includes an accurate and sensitive interpretation of a subject's nucleic acid sample. The output of retrieval can be provided in the format of a computer file. In some embodiments, the output is a FASTA file, a FASTQ file, or a VCF file. The output may be processed to produce a text file, or an XML file containing sequence data such as a sequence of the nucleic acid aligned to a sequence of the reference genome. In other embodiments, processing yields output containing coordinates or a string describing one or more mutations in the subject nucleic acid relative to the reference genome. Alignment strings may include Simple UnGapped Alignment Report (SUGAR), Verbose Useful Labeled Gapped Alignment Report (VULGAR), and Compact Idiosyncratic Gapped Alignment Report (CIGAR) (as described by, for example, Ning et al., Genome Research 11(10): 1725-9, 2001). These strings may be implemented, for example, in the Exonerate sequence alignment software from the European Bioinformatics Institute (Hinxton, UK).

In some embodiments, a sequence alignment is produced-such as, for example, a sequence alignment map (SAM) or binary alignment map (BAM) file-comprising a CIGAR string (the SAM format is described, e.g., by Li et al., "The Sequence Alignment/Map format and SAMtools," Bioinformatics, 25(16):2078-9, 2009). In some embodiments, CIGAR displays or includes gapped alignments one-per-line CIGAR is a compressed pairwise alignment format reported as a CIGAR string. A CIGAR string may be useful for representing long (e.g., genomic) pairwise alignments. A CIGAR string may be used in SAM format to represent alignments of reads to a reference genome sequence.

A CIGAR string may follow an established motif. Each character is preceded by a number, giving the base counts of the event. Characters used can include M, I, D, N, and S (M=match; I=insertion; D=deletion; N=gap; S=substitution). The CIGAR string defines the sequence of matches/mismatches and deletions (or gaps). For example, the CIGAR string 2MD3M2D2M may indicate that the alignment contains 2 matches, 1 deletion (number 1 is omitted in order to save some space), 3 matches, 2 deletions, and 2 matches.

In some embodiments, a nucleic acid population is prepared for sequencing by enzymatically forming blunt-ends on double-stranded nucleic acids with single-stranded overhangs at one or both ends. In these embodiments, the population is typically treated with an enzyme having a 5'-3' DNA polymerase activity and a 3'-5' exonuclease activity in the presence of the nucleotides (e.g., A, C, G, and T or U). Examples of enzymes or catalytic fragments thereof that may be optionally used include Klenow large fragment and T4 polymerase. At 5' overhangs, the enzyme typically extends the recessed 3' end on the opposing strand until it is flush with the 5' end to produce a blunt end. At 3' overhangs, the enzyme generally digests from the 3' end up to and sometimes beyond the 5' end of the opposing strand. If this digestion proceeds beyond the 5' end of the opposing strand, the gap can be filled in by an enzyme having the same polymerase activity that is used for 5' overhangs. The formation of blunt ends on double-stranded nucleic acids facilitates, for example, the attachment of adapters and subsequent amplification.

In some embodiments, nucleic acid populations are subjected to additional processing, such as the conversion of single-stranded nucleic acids to double-stranded nucleic acids and/or conversion of RNA to DNA (e.g., complementary DNA or cDNA). These forms of nucleic acid are also optionally linked to adapters and amplified.

With or without prior amplification, nucleic acids subject to the process of forming blunt-ends described above, and optionally other nucleic acids in a sample, can be sequenced to produce sequenced nucleic acids. A sequenced nucleic acid can refer either to the sequence of a nucleic acid (e.g., sequence information) or a nucleic acid whose sequence has been determined. Sequencing can be performed so as to provide sequence data of individual nucleic acid molecules in a sample either directly or indirectly from a consensus sequence of amplification products of an individual nucleic acid molecule in the sample.

In some embodiments, double-stranded nucleic acids with single-stranded overhangs in a sample after blunt-end formation are linked at both ends to adapters including barcodes, and the sequencing determines nucleic acid sequences as well as in-line barcodes introduced by the adapters. The blunt-end DNA molecules are optionally ligated to a blunt end of an at least partially double-stranded adapter (e.g., a Y-shaped or bell-shaped adapter). Alternatively, blunt ends of sample nucleic acids and adapters can be tailed with complementary nucleotides to facilitate ligation (for e.g., sticky-end ligation).

The nucleic acid sample is typically contacted with a sufficient number of adapters that there is a low probability (e.g., less than about 1 or 0.1 %) that any two copies of the same nucleic acid receive the same combination of adapter barcodes from the adapters linked at both ends. The use of adapters in this manner may permit identification of families of nucleic acid sequences with the same start and stop points on a reference nucleic acid and linked to the same combination of barcodes. Such a family may represent sequences of amplification products of a nucleic acid in the sample before amplification. The sequences of family members can be compiled to derive consensus nucleotide(s) or a complete consensus sequence for a nucleic acid molecule in the original sample, as modified by blunt-end formation and adapter attachment. In other words, the nucleotide occupying a specified position of a nucleic acid in the sample can be determined to be the consensus of nucleotides occupying that corresponding position in family member sequences. Families can include sequences of one or both strands of a double-stranded nucleic acid. If members of a family include sequences of both strands from a double-stranded nucleic acid, sequences of one strand may be converted to their complements for purposes of compiling sequences to derive consensus nucleotide(s) or sequences. Some families include only a single member sequence. In this case, this sequence can be taken as the sequence of a nucleic acid in the sample before amplification. Alternatively, families with only a single member sequence can be eliminated from subsequent analysis.

Nucleotide variations (e.g., SNVs or indels) in sequenced nucleic acids can be determined by comparing sequenced nucleic acids with a reference sequence. The reference sequence is often a known sequence, e.g., a known whole or partial genome sequence from a subject (e.g., a whole genome sequence of a human subject). The reference sequence can be, for example, hG19 or hG38. The sequenced nucleic acids can represent sequences determined directly for a nucleic acid in a sample, or a consensus of sequences of amplification products of such a nucleic acid, as described above. A comparison can be performed at one or more designated positions on a reference sequence. A subset of sequenced nucleic acids can be identified including a position corresponding with a designated position of the reference sequence when the respective sequences are maximally aligned. Within such a subset it can be determined which, if any, sequenced nucleic acids include a nucleotide variation at the designated position, and optionally which if any, include a reference nucleotide (e.g., same as in the reference sequence). If the number of sequenced nucleic acids in the subset including a nucleotide variant exceeding a selected threshold, then a variant nucleotide can be called at the designated position. The threshold can be a simple number, such as at least 1, 2, 3, 4, 5, 6, 7, 8, 9, or 10 sequenced nucleic acids within the subset including the nucleotide variant or it can be a ratio, such as at least 0.5, 1, 2, 3, 4, 5, 10, 15, or 20, of sequenced nucleic acids within the subset that include the nucleotide variant, among other possibilities. The comparison can be repeated for any designated position of interest in the reference sequence. Sometimes a comparison can be performed for designated positions occupying at least about 20, 100, 200, or 300 contiguous positions on a reference sequence, e.g., about 20-500, or about 50-300 contiguous positions.

Additional details regarding nucleic acid sequencing, including the formats and applications described herein, are also provided in, for example, Levy et al., Annual Review of Genomics and Human Genetics, 17: 95-115 (2016), Liu et al., J. of Biomedicine and Biotechnology, Volume 2012, Article ID 251364:1-11 (2012), Voelkerding et al., Clinical Chem., 55: 641-658 (2009), MacLean et al., Nature Rev. Microbiol., 7: 287-296 (2009), Astier et al., J Am Chem Soc., 128(5):1705-10 (2006), U.S. Pat. No. 6,210,891, U.S. Pat. No. 6,258,568, U.S. Pat. No. 6,833,246, U.S. Pat. No. 7,115,400, U.S. Pat. No. 6,969,488, U.S. Pat. No. 5,912,148, U.S. Pat. No. 6,130,073, U.S. Pat. No. 7,169,560, U.S. Pat. No. 7,282,337, U.S. Pat. No. 7,482,120, U.S. Pat. No. 7,501,245, U.S. Pat. No. 6,818,395, U.S. Pat. No. 6,911,345, U.S. Pat. No. 7,501,245, U.S. Pat. No. 7,329,492, U.S. Pat. No. 7,170,050, U.S. Pat. No. 7,302,146, U.S. Pat. No. 7,313,308, and U.S. Pat. No. 7,476,503.

### IV. Computer Systems

Methods of the present disclosure can be implemented using, or with the aid of, computer systems. For example, such methods may comprise (a) adding a set of epigenetic-control nucleic acid molecules to the nucleic acid molecules in the sample of polynucleotides, whereby producing a spiked-in sample; (b) partitioning nucleic acid molecules of the spiked-in sample into a plurality of partitioned sets; (c) enriching a subset of molecules from the plurality of partitioned sets to generate a plurality of enriched molecules, wherein the plurality of enriched molecules comprises a group of epigenetic-control nucleic acid molecules and a group of nucleic acid molecules from the sample of polynucleotides; (d) sequencing the plurality of enriched molecules to produce a plurality of sequencing reads; (e) analyzing the plurality of sequencing reads to generate a plurality of epigenetic partition scores of the epigenetic-control nucleic acid molecules; and (f) comparing the plurality of epigenetic partition scores with a plurality of epigenetic partition cut-offs, can be performed with a computer processor. In this embodiment, the system comprises components for adding epigenetic control nucleic acid molecules, partitioning, enriching and sequencing.

In another embodiment, a system for evaluating a partitioning method of nucleic acid molecules in a sample of polynucleotides based on epigenetic state, comprising: a communication interface that receives, over a communication network, a set of sequencing reads of a spiked-in sample generated by a nucleic acid sequencer, wherein the set of sequencing reads comprise (i) at least a first population of sequencing reads generated from polynucleotides originating from the sample, wherein the sequencing reads from the first population comprise a tag sequence and sequence derived from polynucleotide originating from the sample; and (ii) at least a second population of sequencing reads generated from epigenetic-control nucleic acid molecules, wherein the sequencing reads generated from the second population comprise an epigenetic modification region and optionally, an identifier region; a computer in communication with the communication interface, wherein the computer comprises one or more computer processors and a computer readable medium comprising machine-executable code that, upon execution by the one or more computer processors, implements a method comprising: (i) receiving, over the communication network, the set of sequencing reads from the first and second populations of sequencing reads by the nucleic acid sequencer; (ii) analyzing at least a subset of the set of sequencing reads to generate one or more epigenetic partition scores of the epigenetic-control nucleic acid molecules and/or endogenous control molecules; and (iii) comparing the one or more epigenetic partition scores with one or more epigenetic partition cut-offs.

In another embodiment, a system, comprising a controller comprising, or capable of accessing, computer readable media comprising non-transitory computer-executable instructions which, when executed by at least one electronic processor perform at least: (a) obtaining a set of sequencing reads of a spiked-in sample generated by a nucleic acid sequencer, wherein the spiked-in sample comprises polynucleotides of a sample and epigenetic-control nucleic acid molecules and the set of sequencing reads comprises (i) a first population of sequencing reads generated from polynucleotides of a sample and (ii) a second population of sequencing reads generated from epigenetic-control nucleic acid molecules; (b) analyzing at least a subset of the set of sequencing reads to generate one or more epigenetic partition scores of the epigenetic-control nucleic acid molecules and/or endogenous control molecules; and (c) comparing the one or more epigenetic partition scores with one or more epigenetic partition cut-offs.

In another embodiment, a system, comprising a controller comprising, or capable of accessing, computer readable media comprising non-transitory computer-executable instructions which, when executed by at least one electronic processor performs at least: (a) obtaining a set of sequencing reads of a sample generated by a nucleic acid sequencer, wherein the set of sequencing reads comprises sequencing reads generated from polynucleotides of the sample; (b) analyzing at least a subset of the set of sequencing reads to generate one or more epigenetic partition scores of endogenous control molecules; and (c) comparing the one or more epigenetic partition scores with one or more epigenetic partition cut-offs.

In some embodiments, the system further comprises g) generating an outcome status of the partitioning method based on the comparison of the epigenetic partition scores. In some embodiments, the outcome status of the partitioning method is classified as (i) successful, if the one or more epigenetic partition scores of the epigenetic-control nucleic acid molecules and/or the set of endogenous control molecules is within the corresponding epigenetic partition cut-off; or (ii) unsuccessful, if at least one of the one or more epigenetic partition scores of the epigenetic control molecules and/or the endogenous control molecules is outside the corresponding epigenetic partition cut-offs.

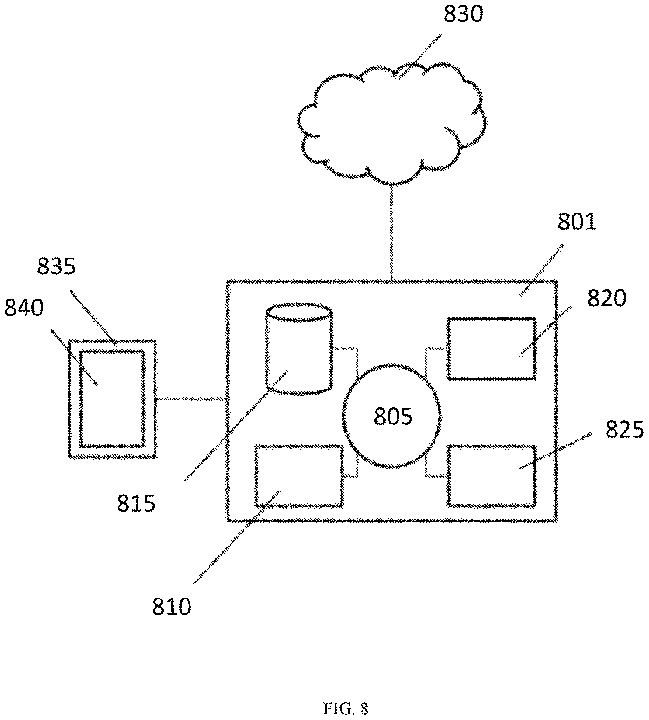

FIG. 8 shows a computer system 801 that is programmed or otherwise configured to implement the methods of the present disclosure. The computer system 801 can regulate various aspects sample preparation, sequencing, and/or analysis. In some examples, the computer system 801 is configured to perform sample preparation and sample analysis, including nucleic acid sequencing.

The computer system 801 includes a central processing unit (CPU, also "processor" and "computer processor" herein) 805, which can be a single core or multi core processor, or a plurality of processors for parallel processing. The computer system 801 also includes memory or memory location 810 (e.g., random-access memory, read-only memory, flash memory), electronic storage unit 815 (e.g., hard disk), communication interface 820 (e.g., network adapter) for communicating with one or more other systems, and peripheral devices 825, such as cache, other memory, data storage, and/or electronic display adapters. The memory 810, storage unit 815, interface 820, and peripheral devices 825 are in communication with the CPU 805 through a communication network or bus (solid lines), such as a motherboard. The storage unit 815 can be a data storage unit (or data repository) for storing data. The computer system 801 can be operatively coupled to a computer network 430 with the aid of the communication interface 820. The computer network 830 can be the Internet, an internet and/or extranet, or an intranet and/or extranet that is in communication with the Internet. The computer network 830 in some cases is a telecommunication and/or data network. The computer network 830 can include one or more computer servers, which can enable distributed computing, such as cloud computing. The computer network 830, in some cases with the aid of the computer system 801, can implement a peer-to-peer network, which may enable devices coupled to the computer system 801 to behave as a client or a server.

The CPU 805 can execute a sequence of machine-readable instructions, which can be embodied in a program or software. The instructions may be stored in a memory location, such as the memory 810. Examples of operations performed by the CPU 405 can include fetch, decode, execute, and writeback.

The storage unit 815 can store files, such as drivers, libraries, and saved programs. The storage unit 815 can store programs generated by users and recorded sessions, as well as output(s) associated with the programs. The storage unit 815 can store user data, e.g., user preferences and user programs. The computer system 801 in some cases can include one or more additional data storage units that are external to the computer system 801, such as located on a remote server that is in communication with the computer system 801 through an intranet or the Internet. Data may be transferred from one location to another using, for example, a communication network or physical data transfer (e.g., using a hard drive, thumb drive, or other data storage mechanism).

The computer system 801 can communicate with one or more remote computer systems through the network 830. For embodiment, the computer system 801 can communicate with a remote computer system of a user (e.g., operator). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 801 via the network 830.

Methods as described herein can be implemented by way of machine (e.g., computer processor) executable code stored on an electronic storage location of the computer system 801, such as, for example, on the memory 810 or electronic storage unit 815. The machine executable or machine-readable code can be provided in the form of software. During use, the code can be executed by the processor 805. In some cases, the code can be retrieved from the storage unit 815 and stored on the memory 810 for ready access by the processor 805. In some situations, the electronic storage unit 815 can be precluded, and machine-executable instructions are stored on memory 810.

In an aspect, the present disclosure provides a non-transitory computer-readable medium comprising computer-executable instructions which, when executed by at least one electronic processor, perform a method comprising: (a) obtaining a set of sequencing reads generated by a nucleic acid sequencer; (b) analyzing at least a subset of the set of sequencing reads to generate one or more epigenetic partition scores of the epigenetic-control nucleic acid molecules; and (f) comparing the one or more epigenetic partition scores with one or more epigenetic partition cut-offs.

The code can be pre-compiled and configured for use with a machine have a processer adapted to execute the code or can be compiled during runtime. The code can be supplied in a programming language that can be selected to enable the code to execute in a pre-compiled or as-compiled fashion.

Aspects of the systems and methods provided herein, such as the computer system 801, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming.

All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as those used across physical interfaces between local devices, through wired and optical landline networks, and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine-readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards, paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 801 can include or be in communication with an electronic display that comprises a user interface (UI) for providing, for example, one or more results of sample analysis. Examples of UIs include, without limitation, a graphical user interface (GUI) and web-based user interface.

Additional details relating to computer systems and networks, databases, and computer program products are also provided in, for example, Peterson, Computer Networks: A Systems Approach, Morgan Kaufmann, 5th Ed. (2011), Kurose, Computer Networking: A Top-Down Approach, Pearson, 7th Ed. (2016), Elmasri, Fundamentals of Database Systems, Addison Wesley, 6th Ed. (2010), Coronel, Database Systems: Design, Implementation, & Management, Cengage Learning, 11th Ed. (2014), Tucker, Programming Languages, McGraw-Hill Science/Engineering/Math, 2nd Ed. (2006), and Rhoton, Cloud Computing Architected: Solution Design Handbook, Recursive Press (2011).

### V. Applications

### A. Cancer and Other Diseases

In some embodiments, the methods and systems disclosed herein may be used to identify customized or targeted therapies to treat a given disease or condition in patients based on the classification of a nucleic acid variant as being of somatic or germline origin. Typically, the disease under consideration is a type of cancer. Non-limiting examples of such cancers include biliary tract cancer, bladder cancer, transitional cell carcinoma, urothelial carcinoma, brain cancer, gliomas, astrocytomas, breast carcinoma, metaplastic carcinoma, cervical cancer, cervical squamous cell carcinoma, rectal cancer, colorectal carcinoma, colon cancer, hereditary nonpolyposis colorectal cancer, colorectal adenocarcinomas, gastrointestinal stromal tumors (GISTs), endometrial carcinoma, endometrial stromal sarcomas, esophageal cancer, esophageal squamous cell carcinoma, esophageal adenocarcinoma, ocular melanoma, uveal melanoma, gallbladder carcinomas, gallbladder adenocarcinoma, renal cell carcinoma, clear cell renal cell carcinoma, transitional cell carcinoma, urothelial carcinomas, Wilms tumor, leukemia, acute lymphocytic leukemia (ALL), acute myeloid leukemia (AML), chronic lymphocytic leukemia (CLL), chronic myeloid leukemia (CML), chronic myelomonocytic leukemia (CMML), liver cancer, liver carcinoma, hepatoma, hepatocellular carcinoma, cholangiocarcinoma, hepatoblastoma, Lung cancer, non-small cell lung cancer (NSCLC), mesothelioma, B-cell lymphomas, non-Hodgkin lymphoma, diffuse large B-cell lymphoma, Mantle cell lymphoma, T cell lymphomas, non-Hodgkin lymphoma, precursor T-lymphoblastic lymphomalleukemia, peripheral T cell lymphomas, multiple myeloma, nasopharyngeal carcinoma (NPC), neuroblastoma, oropharyngeal cancer, oral cavity squamous cell carcinomas, osteosarcoma, ovarian carcinoma, pancreatic cancer, pancreatic ductal adenocarcinoma, pseudopapillary neoplasms, acinar cell carcinomas. Prostate cancer, prostate adenocarcinoma, skin cancer, melanoma, malignant melanoma, cutaneous melanoma, small intestine carcinomas, stomach cancer, gastric carcinoma, gastrointestinal stromal tumor (GIST), uterine cancer, or uterine sarcoma.

Non-limiting examples of other genetic-based diseases, disorders, or conditions that are optionally evaluated using the methods and systems disclosed herein include achondroplasia, alpha-1 antitrypsin deficiency, antiphospholipid syndrome, autism, autosomal dominant polycystic kidney disease, Charcot-Marie-Tooth (CMT), cri du chat, Crohn's disease, cystic fibrosis, Dercum disease, down syndrome, Duane syndrome, Duchenne muscular dystrophy, Factor V Leiden thrombophilia, familial hypercholesterolemia, familial mediterranean fever, fragile X syndrome, Gaucher disease, hemochromatosis, hemophilia, holoprosencephaly, Huntington's disease, Klinefelter syndrome, Marfan syndrome, myotonic dystrophy, neurofibromatosis, Noonan syndrome, osteogenesis imperfecta, Parkinson's disease, phenylketonuria, Poland anomaly, porphyria, progeria, retinitis pigmentosa, severe combined immunodeficiency (scid), sickle cell disease, spinal muscular atrophy, Tay-Sachs, thalassemia, trimethylaminuria, Turner syndrome, velocardiofacial syndrome, WAGR syndrome, Wilson disease, or the like.

### B. Therapies and Related Administration

In certain embodiments, the methods disclosed herein relate to identifying and administering customized therapies to patients given the status of a nucleic acid variant as being of somatic or germline origin. In some embodiments, essentially any cancer therapy (e.g., surgical therapy, radiation therapy, chemotherapy, and/or the like) may be included as part of these methods. Typically, customized therapies include at least one immunotherapy (or an immunotherapeutic agent). Immunotherapy refers generally to methods of enhancing an immune response against a given cancer type. In certain embodiments, immunotherapy refers to methods of enhancing a T cell response against a tumor or cancer.

In certain embodiments, the status of a nucleic acid variant from a sample from a subject as being of somatic or germline origin may be compared with a database of comparator results from a reference population to identify customized or targeted therapies for that subject. Typically, the reference population includes patients with the same cancer or disease type as the test subject and/or patients who are receiving, or who have received, the same therapy as the test subject. A customized or targeted therapy (or therapies) may be identified when the nucleic variant and the comparator results satisfy certain classification criteria (e.g., are a substantial or approximate match).

In certain embodiments, the customized therapies described herein are typically administered parenterally (e.g., intravenously or subcutaneously). Pharmaceutical compositions containing a immunotherapeutic agent are typically administered intravenously. Certain therapeutic agents are administered orally. However, customized therapies (e.g., immunotherapeutic agents, etc.) may also be administered by any method known in the art, including, for example, buccal, sublingual, rectal, vaginal, intraurethral, topical, intraocular, intranasal, and/or intraauricular, which administration may include tablets, capsules, granules, aqueous suspensions, gels, sprays, suppositories, salves, ointments, or the like.

### EXAMPLES

### Example 1: Evaluating the partitioning of a cell-free DNA sample

A cell-free DNA sample from a patient is analyzed here. A spiked-in sample is created by combining the cell-free DNA sample with a set of epigenetic-control nucleic acid molecules. In this example, the epigenetic-control nucleic acid molecules are double stranded DNA molecules and the set of epigenetic-control nucleic acid molecules is a pool of 6 different subsets (Subset 1 to Subset 6) of epigenetic-control nucleic acid molecules. Subset 1, subset 2, subset 3, subset 4, subset 5 and subset 6 comprises epigenetic-control nucleic acid molecules with 0, 1, 3, 5, 7, and 9 methylated cytosines (5-methylcytosine) in the epigenetic modification region. The epigenetic-control nucleic acid molecules have a molecular barcode on one end of the epigenetic modification region and the epigenetic state barcode is present on both the ends of the epigenetic modification region. The molecular barcode used here is a unique molecular barcode i.e., each epigenetic control-nucleic acid molecule has a distinct molecular barcode.

This spiked-in sample is then combined with methyl binding domain (MBD) buffers and magnetic beads conjugated with MBD proteins and incubated overnight. Methylated DNA (if present, in the cell-free DNA sample) and methylated epigenetic-control nucleic acid molecules are bound by the MBD protein during this incubation. Non-methylated or less methylated DNA is washed away from the beads with buffers containing increasing concentrations of salt. Finally, a high salt buffer is used to wash the heavily methylated DNA away from the MBD protein. These washes result in three partitions (three partitioned sets - hypo, intermediate and hyper) of increasingly methylated DNA. The partitioned DNA present in the partitioned set comprises of DNA from the cell-free DNA sample and epigenetic-control nucleic acid molecules. The partitioned DNA in the three partitioned sets are cleaned, to remove salt, and concentrated in preparation for the enzymatic steps of library preparation.

After concentrating the DNA in the partitioned sets, the end overhangs of partitioned DNA are extended, and adenosine residues are added to the 3' ends of fragments. The 5' end of each fragment is phosphorylated. These modifications make the partitioned DNA ligatable. DNA ligase and adapters are added to ligate each partitioned DNA molecule with an adapter on each end. These adapters contain non-unique barcodes and each partitioned set is ligated with adapters having non-unique barcodes that is distinguishable from the barcodes in the adapters used in the other partitioned sets. After ligation, the 3 partitioned sets are pooled together and are amplified by PCR.

Following PCR, amplified DNA is again cleaned and concentrated prior to enrichment. Once concentrated, the amplified DNA is combined with salt buffer and biotinylated RNA probes targeting specific regions of interest and the epigenetic-control nucleic acid molecules and this mixture is incubated overnight. The biotinylated RNA probes are captured by streptavidin magnetic beads and separated from the amplified DNA that was not captured by a series of salt washes, thereby enriching the sample. After enrichment, sample indices are incorporated to the enriched molecules via PCR amplification. After PCR amplification, the amplified molecules from different samples (within a batch) are pooled together and is sequenced using Illumina NovaSeq sequencer.

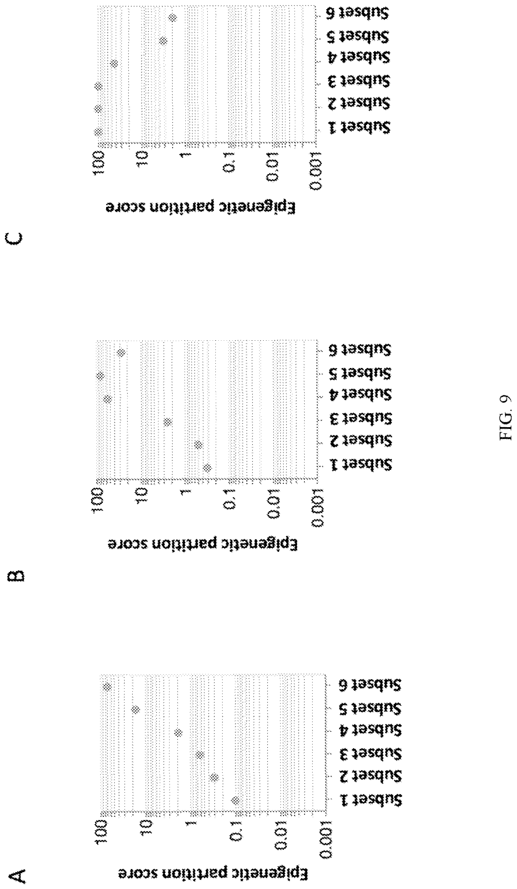

The sequence reads generated by the sequencer are then analyzed using bioinformatic tools/algorithms to generate an epigenetic partition score of the epigenetic-control nucleic acid molecules belonging to each subset present in each of the three partitioned sets. FIG. 9A shows a graphical plot of the epigenetic partition scores of the epigenetic-control nucleic acid molecules belonging to each of the six subsets (Subset 1, Subset 2, Subset 3, Subset 4, Subset 5 and Subset 6), described in this example, in the hyper partitioned set. FIG. 9B shows a graphical plot of the epigenetic partition scores of the epigenetic-control nucleic acid molecules belonging to each of the six subsets in the intermediate partitioned set. FIG. 9C shows a graphical plot of the epigenetic partition scores of the epigenetic-control nucleic acid molecules belonging to each of the six subsets in the hypo partitioned set. As shown in FIG. 9, the epigenetic partition score of Subset 1 in hyper partitioned set, intermediate partitioned set and hypo partitioned set is about 0.1%, 0.3% and 99.6% respectively. The predetermined epigenetic partition cut-off for subset 1 in the hyper partitioned set, intermediate partitioned set and hypo partitioned set is ≤ 0.3%, ≤ 0.5% and ≥ 97% respectively. Here, the epigenetic partition scores are expressed in terms of percentage. The epigenetic partition scores of Subset 1 in each of the partitioned sets is compared with the corresponding epigenetic cut-offs for Subset 1 - i.e., the epigenetic partition score (0.1%) of Subset 1 in hyper partitioned set is compared with the epigenetic partition cut-off (≤ 0.3%) for Subset 1 in hyper partitioned set. The epigenetic partition score (0.1%) of Subset 1 in hyper partitioned set is within the epigenetic partition cut-off (≤ 0.3%) for Subset 1 in hyper partitioned set. Likewise, the epigenetic partition scores of Subset 1 in intermediate partitioned set and hypo partitioned set is compared with the respective epigenetic partition cut-offs for Subset 1 in intermediate partitioned set and hypo partitioned set. Similarly, the epigenetic partition scores of Subset 2, Subset 3, Subset 4, Subset 5 and Subset 6 are compared with the respective epigenetic partition cut-offs in all the three partitioned sets. So, we have a total of 18 epigenetic partition scores (6 x 3 = 18, for six subsets in three partitioned sets) and each of these epigenetic partition scores is compared with the corresponding epigenetic partition cut-offs. All the 18 epigenetic partition scores are found to be within the respective epigenetic partition cut-offs. Hence, the partitioning method performed on the cell-free DNA sample analyzed here is classified as being a success.

### Example 2: Evaluating the partitioning of cell-free DNA samples

A set of cell-free DNA samples from a set of patients are analyzed here. In this example, epigenetic-control nucleic acid molecules are not used. Instead, the endogenous control molecules in the cell-free DNA sample are used for evaluating the partitioning of cell-free DNA samples. Cell-free DNA sample of each patient is combined with methyl binding domain (MBD) buffers and magnetic beads conjugated with a MBDs protein and incubated overnight. Methylated DNA (if present, in the cell-free DNA sample) and methylated epigenetic-control nucleic acid molecules are bound by the MBD protein during this incubation. Non-methylated or less methylated DNA is washed away from the beads with buffers containing increasing concentrations of salt. Finally, a high salt buffer is used to wash the heavily methylated DNA away from the MBD protein. These washes result in three partitions (three partitioned sets - hypo, intermediate and hyper) of increasingly methylated DNA. The partitioned DNA present in the partitioned set comprises of DNA from the cell-free DNA sample and epigenetic-control nucleic acid molecules. The partitioned DNA in the three partitioned sets are cleaned, to remove salt, and concentrated in preparation for the enzymatic steps of library preparation.

After concentrating the DNA in the partitioned sets, the end overhangs of partitioned DNA are extended, and adenosine residues are added to the 3' ends of fragments. The 5' end of each fragment is phosphorylated. These modifications make the partitioned DNA ligatable. DNA ligase and adapters are added to ligate each partitioned DNA molecule with an adapter on each end. These adapters contain non-unique barcodes and each partitioned set is ligated with adapters having non-unique barcodes that is distinguishable from the barcodes in the adapters used in the other partitioned sets. After ligation, the 3 partitioned sets are pooled together and are amplified by PCR.

Following PCR, amplified DNA is again cleaned and concentrated prior to enrichment. Once concentrated, the amplified DNA is combined with salt buffer and biotinylated RNA probes targeting specific regions of interest and the epigenetic-control nucleic acid molecules and this mixture is incubated overnight. The biotinylated RNA probes are captured by streptavidin magnetic beads and separated from the amplified DNA that was not captured by a series of salt washes, thereby enriching the sample. After enrichment, sample indices are incorporated to the enriched molecules via PCR amplification. After PCR amplification, the amplified molecules from different samples (within a batch) are pooled together and is sequenced using Illumina NovaSeq sequencer.

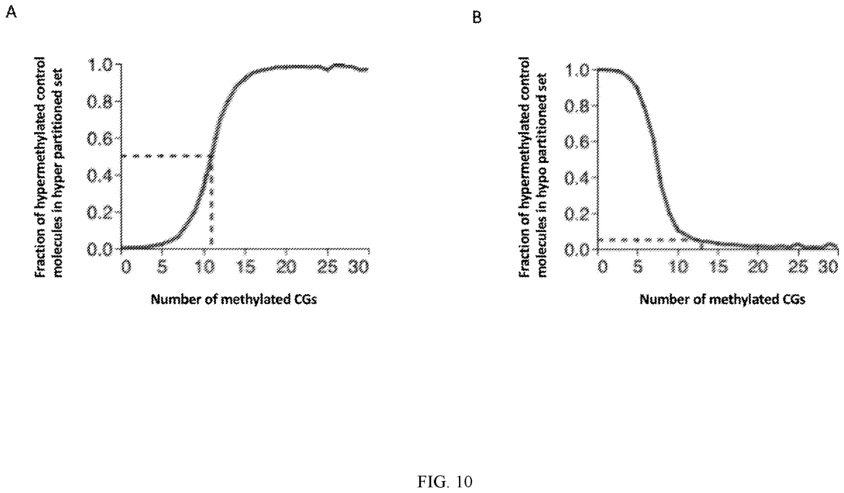

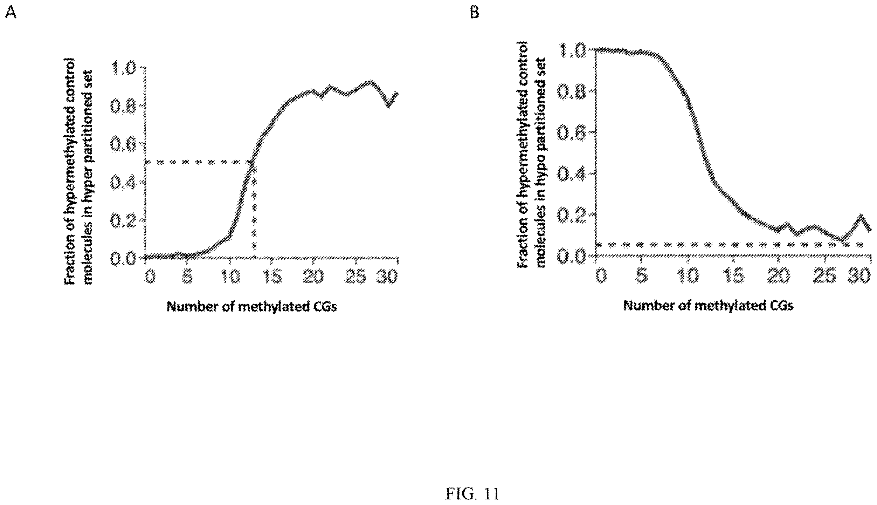

The sequence reads generated by the sequencer are then analyzed using bioinformatic tools/algorithms to generate one or more epigenetic partition score of the endogenous control molecules. In this example, *methyl-half* and *methyl-5* are used as epigenetic partition scores. FIG. 10A shows a graphical plot of the fraction of hypermethylated control molecules of Sample 1 in the hyper partitioned set and the *methyl-half* score of Sample 1 is 11. FIG. 10B shows a graphical plot of the fraction of hypermethylated molecules of Sample 1 in the hypo partitioned set and the *methyl-5* score of Sample 1 is 13. FIG. 11A shows a graphical plot of the fraction of hypermethylated control molecules of Sample 2 in the hyper partitioned set and the *methyl-half* score of Sample 2 is 13. FIG. 11B shows a graphical plot of the fraction of hypermethylated molecules of Sample 2 in the hypo partitioned set and the *methyl-5* score of Sample 2 is unable to be determined (as shown in FIG. 11B). In this example, the epigenetic partition cut-offs for *methyl-half* and *methyl-5* are 15 and 20 methylated CGs respectively. The methyl-half and methyl-5 scores of Sample 1 is within the corresponding epigenetic partition cut-offs. But, for Sample 2, the methyl-half score is within its corresponding epigenetic partition cut-off but the methyl-5 score is not within its corresponding epigenetic partition cut-off. Hence, the partitioning method of Sample 1 is classified as being a success and the partitioning method of Sample 2 is classified as being unsuccessful.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the disclosure described herein may be employed in practicing the invention. It is therefore contemplated that the disclosure shall also cover any such alternatives, modifications, variations or equivalents. It is intended that the following claims define the scope of the invention and that methods and structures within the scope of these claims and their equivalents be covered thereby.

## Claims

1. A method for evaluating partitioning of nucleic acid molecules in a sample of polynucleotides based on epigenetic state, comprising:
a. partitioning nucleic acid molecules from at least a subset of the sample of polynucleotides into a plurality of partitioned sets;
b. enriching at least a subset of molecules comprising specific target genomic regions from the plurality of partitioned sets to generate a set of enriched molecules, wherein the set of enriched molecules comprises a group of nucleic acid molecules from the sample of polynucleotides, wherein the group of nucleic acid molecules from the sample of polynucleotides comprises a set of endogenous control molecules, which correspond to at least one human genomic region with a non-variable epigenetic state;
c. sequencing at least a subset of the set of enriched molecules to produce a plurality of sequencing reads;
d. analyzing at least a subset of the set of sequencing reads to generate one or more epigenetic partition scores for the set of endogenous control molecules; and
e. comparing the one or more epigenetic partition scores with one or more epigenetic partition cut-offs.

2. The method of claim 1, further comprising an initial step of adding a set of epigenetic-control nucleic acid molecules to the nucleic acid molecules in the sample of polynucleotides, thereby producing a spiked-in sample, and wherein
step a. is performed on the spiked-in sample;
within step b., the set of enriched molecules further comprises a group of epigenetic-control nucleic acid molecules; and
step d. additionally generates one or more epigenetic partition scores for the epigenetic-control nucleic acid molecules.

3. The method of claim 2, wherein
i) the set of epigenetic-control nucleic acid molecules comprises two or more subsets of epigenetic-control nucleic acid molecules, wherein a subset of the two or more subsets of epigenetic-control nucleic acid molecules comprises a plurality of epigenetic-control nucleic acid molecules comprising an epigenetic modification region, optionally wherein
a) each subset of epigenetic-control nucleic acid molecules is in equimolar concentration; or each subset of epigenetic-control nucleic acid molecules is in non-equimolar concentration; and/or
b) the epigenetic modification region of the plurality of epigenetic-control nucleic acid molecules in the two or more subsets comprises identical nucleic acid sequence; or the epigenetic modification region of the plurality of epigenetic-control nucleic acid molecules in a first subset comprises a nucleic acid sequence distinguishable from the nucleic acid sequence of the epigenetic modification region of the plurality of epigenetic-control nucleic acid molecules in a second subset;
ii) the epigenetic-control nucleic acid molecule further comprises an identifier region, for example wherein the identifier region is on one or both sides of the epigenetic modification region of the epigenetic-control nucleic acid molecules, optionally wherein the identifier region of the epigenetic-control nucleic acid molecules
a) comprises a molecular barcode;
b) further comprises at least one epigenetic state barcode; and/or
c) comprises one or more primer binding sites;
iii) the epigenetic modification region of the epigenetic-control nucleic acid molecules in at least one subset comprises at least one nucleotide with epigenetic modification; and/or
iv) the plurality of partitioned sets comprises nucleic acid molecules of the spiked-in sample partitioned based on the methylation level of the nucleic acid molecules.

4. The method of any one of the preceding claims, wherein the partitioning comprises partitioning the nucleic acid molecules based on a differential binding affinity of the nucleic acid molecules to a binding agent that preferentially binds to nucleic acid molecules comprising nucleotides with epigenetic modification.

5. The method of any one of the preceding claims, further comprising tagging the nucleic acid molecules in a partitioned set of the plurality of partitioned sets with a set of tags to produce a population of tagged nucleic acid molecules, wherein the tagged nucleic acid molecules comprise one or more tags; optionally wherein
the set of tags used in a first partitioned set of the plurality of partitioned sets is different from the set of tags used in a second partitioned set of the plurality of partitioned sets; and further optionally
the set of tags are attached to the nucleic acid molecules by ligation of adapters to the nucleic acid molecules, wherein the adapters comprise one or more tags.

6. The method of any one of the preceding claims, further comprising
g. classifying the method as (i) being a success, if the one or more epigenetic partition scores of the epigenetic-control nucleic acid molecules and/or the set of endogenous control molecules is within the corresponding epigenetic partition cut-offs; or (ii) being unsuccessful, if at least one of the one or more epigenetic partition scores of the epigenetic control molecules and/or the set of endogenous control molecules is outside the corresponding epigenetic partition cut-off.

7. The method of any one of the preceding claims, wherein
i) the epigenetic modification is DNA methylation;
ii) the nucleotide with epigenetic modification comprises a methylated nucleotide, for example wherein
the methylated nucleotide comprises 5-methylcytosine; or
the methylated nucleotide comprises 5-hydroxymethylcytosine; and/or
iii) the epigenetic state is methylation level of the nucleic acid molecules.

8. The method of any one of the preceding claims,
wherein the epigenetic partition score comprises a fraction or percentage of
i) the number of hypermethylated epigenetic-control nucleic acid molecules and/or hypermethylated control molecules in a partitioned set; or
ii) the number of hypomethylated epigenetic-control nucleic acid molecules and/or hypomethylated control molecules in a partitioned set; and/or
wherein the partitioned set is
a) hypermethylated partitioned set; or
b) hypomethylated partitioned set.

9. The method of any one of the preceding claims, wherein the epigenetic partition score is
i) *0 CG score,* optionally wherein the epigenetic partition cut-off for the *0 CG score* is 0.01%, 0.02%, 0.05%, 0.1%, 0.2%, 0.3%, 0.4%, 0.5%, 0.6%, 0.7%, 0.8%, 0.9%, 1%, 2%, 5%, at least 5% or at least 10%;
ii) *hypo score,* optionally wherein the epigenetic partition cut-off for the *hypo score* is 0.1%, 0.5%, 1%, 2%, 3%, 4%, 5%, 7% or at least 10%;
iii) *methyl-half,* optionally wherein the epigenetic partition cut-off for the *methyl-half* is 5, 10, 15, 20, 25, 30, 35 or 40 mCGs; or
iv) *methyl-5,* optionally wherein the epigenetic partition cut-off for the *methyl-5* is 5, 10, 20, 30, 40 or 50 mCGs.

10. The method of any one of the preceding claims, wherein the sequencing of the plurality of enriched molecules is performed by a nucleic acid sequencer, for example wherein the nucleic acid sequencer is a next generation sequencer.

11. The method of any one of the preceding claims, wherein the sample of polynucleotides is
i) selected from the group consisting of a sample of DNA, a sample of RNA, a sample of polynucleotides, a sample of cell-free DNA, and a sample of cell-free RNA; and/or
ii) a cell-free DNA, optionally wherein the cell-free DNA is between 1 ng and 500 ng.

12. The method of any one of the preceding claims, further comprising
i) generating a report which optionally includes information on, and/or information derived from, the partitioning of nucleic acid molecules; and optionally
ii) communicating the report to a third party, such as the subject from whom the sample derived or a health care practitioner.

13. A system, comprising a controller comprising, or capable of accessing, computer readable media comprising non-transitory computer-executable instructions which, when executed by at least one electronic processor performs at least:
a. obtaining a set of sequencing reads of a sample generated by a nucleic acid sequencer, wherein the set of sequencing reads comprises sequencing reads generated from polynucleotides of the sample, and wherein the sample was partitioned and enriched prior to sequencing according to steps a and b of claim 1;
b. analyzing at least a subset of the set of sequencing reads to generate one or more epigenetic partition scores of endogenous control molecules;
c. comparing the one or more epigenetic partition scores with one or more epigenetic partition cut-offs; and
d. generating an outcome status of the partitioning method based on the comparison of the epigenetic partition scores, wherein the outcome status of the partitioning method is classified as (i) successful, if the one or more epigenetic partition scores of the set of endogenous control molecules is within the corresponding epigenetic partition cut-offs; or (ii) unsuccessful, if at least one of the one or more epigenetic partition scores of the endogenous control molecules is outside the corresponding epigenetic partition cut-off.

14. The system of claim 13, wherein
the sample is a spiked-in sample and was partitioned and enriched according to steps a and b of claim 2;
step b additionally generates one or more epigenetic partition scores for the epigenetic-control nucleic acid molecules; and
the outcome status in step d is classified as (i) successful, if the one or more epigenetic partition scores of the epigenetic-control nucleic acid molecules and/or the set of endogenous control molecules is within the corresponding epigenetic partition cut-offs; or (ii) unsuccessful, if at least one of the one or more epigenetic partition scores of the epigenetic control molecules and/or the endogenous control molecules is outside the corresponding epigenetic partition cut-off.

15. The system of claim 13 or claim 14, wherein the epigenetic partition score is
i) *0 CG score,* optionally wherein the epigenetic partition cut-off for the *0 CG score* is 0.01%, 0.02%, 0.05%, 0.1%, 0.2%, 0.3%, 0.4%, 0.5%, 0.6%, 0.7%, 0.8%, 0.9%, 1%, 2%, 5%, at least 5% or at least 10%;
ii) *hypo score,* optionally wherein the epigenetic partition cut-off for the *hypo score* is 0.1%, 0.5%, 1%, 2%, 3%, 4%, 5%, 7% or at least 10%;
iii) *methyl-half,* optionally wherein the epigenetic partition cut-off for the *methyl-half* is 5, 10, 15, 20, 25, 30, 35 or 40 mCGs; or
iv) *methyl-5,* optionally wherein the epigenetic partition cut-off for the *methyl-5* is 5, 10, 20, 30, 40 or 50 mCGs.

## Patentansprüche

1. Verfahren zur Beurteilung der Partitionierung von Nukleinsäuremolekülen in einer Probe von Polynukleotiden basierend auf dem epigenetischen Status, umfassend:
a. Partitionieren von Nukleinsäuremolekülen aus mindestens einer Teilmenge der Probe von Polynukleotiden in eine Vielzahl partitionierter Sätze;
b. Anreichern mindestens einer Teilmenge von spezifische genomische Zielregionen umfassenden Molekülen aus der Vielzahl partitionierter Sätze zum Erzeugen eines Satzes von angereicherten Molekülen, wobei der Satz von angereicherten Molekülen eine Gruppe von Nukleinsäuremolekülen aus der Probe von Polynukleotiden umfasst, wobei die Gruppe von Nukleinsäuremolekülen aus der Probe von Polynukleotiden einen Satz von endogenen Kontrollmolekülen umfasst, die mindestens einer humanen genomischen Region mit einem nicht-variablen epigenetischen Status entsprechen;
c. Sequenzieren mindestens einer Teilmenge des Satzes von angereicherten Molekülen zum Erzeugen einer Vielzahl von Sequenzierungsablesungen;
d. Analysieren mindestens einer Teilmenge des Satzes von Sequenzierungsablesungen zum Erstellen eines oder mehrerer epigenetischer Partitions-Scores für den Satz von endogenen Kontrollmolekülen; und
e. Vergleichen des einen oder der mehreren epigenetischen Partitions-Scores mit einem oder mehreren epigenetischen Partitions-Cutoffs.

2. Verfahren nach Anspruch 1, ferner umfassend einen anfänglichen Schritt des Hinzusetzens eines Satzes von Epigenetik-Kontrollnukleinsäuremolekülen zu den Nukleinsäuremolekülen in der Probe von Polynukleotiden, wodurch eine "spiked-in" (versetzte) Probe erzeugt wird, und wobei
Schritt a. an der versetzten Probe durchgeführt wird;
innerhalb von Schritt b. der Satz von angereicherten Molekülen ferner eine Gruppe von Epigenetik-Kontrollnukleinsäuremolekülen umfasst; und
Schritt d. zusätzlich einen oder mehrere epigenetische Partitions-Scores für die Epigenetik-Kontrollnukleinsäuremoleküle erstellt.

3. Verfahren nach Anspruch 2, wobei
i) der Satz von Epigenetik-Kontrollnukleinsäuremolekülen zwei oder mehr Teilmengen von Epigenetik-Kontrollnukleinsäuremolekülen umfasst, wobei eine Teilmenge von den zwei oder mehr Teilmengen von Epigenetik-Kontrollnukleinsäuremolekülen eine Vielzahl von Epigenetik-Kontrollnukleinsäuremolekülen, die eine epigenetische Modifikationsregion umfassen, umfasst, optional wobei
a) jede Teilmenge von Epigenetik-Kontrollnukleinsäuremolekülen in äquimolarer Konzentration vorliegt; oder jede Teilmenge von Epigenetik-Kontrollnukleinsäuremolekülen in nicht-äquimolarer Konzentration vorliegt; und/oder
b) die epigenetische Modifikationsregion der Vielzahl von Epigenetik-Kontrollnukleinsäuremolekülen in den zwei oder mehr Teilmengen eine identische Nukleinsäuresequenz umfasst; oder die epigenetische Modifikationsregion der Vielzahl von Epigenetik-Kontrollnukleinsäuremolekülen in einer ersten Teilmenge eine Nukleinsäuresequenz umfasst, die von der Nukleinsäuresequenz der epigenetischen Modifikationsregion der Vielzahl von Epigenetik-Kontrollnukleinsäuremolekülen in einer zweiten Teilmenge unterscheidbar ist;
ii) das Epigenetik-Kontrollnukleinsäuremolekül ferner eine Identifikatorregion umfasst, zum Beispiel wobei die Identifikatorregion auf einer oder beiden Seiten der epigenetischen Modifikationsregion der Epigenetik-Kontrollnukleinsäuremoleküle vorliegt, optional wobei die Identifikatorregion der Epigenetik-Kontrollnukleinsäuremoleküle
a) einen molekularen Barcode umfasst;
b) ferner mindestens einen Barcode für den epigenetischen Status umfasst; und/oder
c) eine oder mehrere Primerbindungsstellen umfasst;
iii) die epigenetische Modifikationsregion der Epigenetik-Kontrollnukleinsäuremoleküle in mindestens einer Teilmenge mindestens ein Nukleotid mit epigenetischer Modifikation umfasst; und/oder
iv) die Vielzahl partitionierter Sätze Nukleinsäuremoleküle der versetzten Probe, partitioniert basierend auf dem Methylierungsspiegel der Nukleinsäuremoleküle, umfasst.

4. Verfahren nach einem der vorhergehenden Ansprüche, wobei das Partitionieren ein Partitionieren der Nukleinsäuremoleküle basierend auf einer differentiellen Bindungsaffinität der Nukleinsäuremoleküle an ein Bindungsmittel umfasst, das präferentiell an Nukleinsäuremoleküle bindet, die Nukleotide mit epigenetischer Modifikation umfassen.

5. Verfahren nach einem der vorhergehenden Ansprüche, ferner umfassend das Taggen der Nukleinsäuremoleküle in einem partitionierten Satz aus der Vielzahl partitionierter Sätze mit einem Satz von Tags zum Erzeugen einer Population getaggter Nukleinsäuremoleküle, wobei die getaggten Nukleinsäuremoleküle ein oder mehrere Tags umfassen; optional wobei
der Satz von Tags, verwendet in einem ersten partitionierten Satz aus der Vielzahl partitionierter Sätze, verschieden ist von dem Satz von Tags, verwendet in einem zweiten partitionierten Satz aus der Vielzahl partitionierter Sätze; und ferner optional
der Satz von Tags an die Nukleinsäuremoleküle durch Ligation von Adaptoren an die Nukleinsäuremoleküle angeheftet wird, wobei die Adaptoren ein oder mehrere Tags umfassen.

6. Verfahren nach einem der vorhergehenden Ansprüche, ferner umfassend
g. Klassifizieren des Verfahrens als (i) erfolgreich, wenn der eine oder die mehreren epigenetischen Partitions-Scores von den Epigenetik-Kontrollnukleinsäuremolekülen und/oder dem Satz von endogenen Kontrollmolekülen innerhalb der entsprechenden epigenetischen Partitions-Cutoffs liegt/liegen; oder (ii) nicht erfolgreich, wenn mindestens einer von dem einen oder den mehreren epigenetischen Partitions-Scores der Epigenetik-Kontrollmoleküle und/oder des Satzes von endogenen Kontrollmolekülen außerhalb des entsprechenden epigenetischen Partitions-Cutoffs liegt.

7. Verfahren nach einem der vorhergehenden Ansprüche, wobei
i) es sich bei der epigenetischen Modifikation um DNA-Methylierung handelt;
ii) das Nukleotid mit epigenetischer Modifikation ein methyliertes Nukleotid umfasst, zum Beispiel wobei
das methylierte Nukleotid 5-Methylcytosin umfasst; oder
das methylierte Nukleotid 5-Hydroxymethylcytosin umfasst; und/oder
iii) es sich bei dem epigenetischen Status um den Methylierungsspiegel der Nukleinsäuremoleküle handelt.

8. Verfahren nach einem der vorhergehenden Ansprüche, wobei der epigenetische Partitions-Score einen Bruchteil oder Prozentsatz umfasst von
i) der Anzahl von hypermethylierten Epigenetik-Kontrollnukleinsäuremolekülen und/oder hypermethylierten Kontrollmolekülen in einem partitionierten Satz; oder
ii) der Anzahl von hypomethylierten Epigenetik-Kontrollnukleinsäuremolekülen und/oder hypomethylierten Kontrollmolekülen in einem partitionierten Satz; und/oder
wobei es sich bei dem partitionierten Satz handelt um einen
a) hypermethylierten partitionierten Satz; oder
b) hypomethylierten partitionierten Satz.

9. Verfahren nach einem der vorhergehenden Ansprüche, wobei es sich bei dem epigenetischen Partitions-Score handelt um
i) *O-CG-Score,* optional wobei der epigenetische Partitions-Cutoff für den *O-CG-Score* sich auf 0,01%, 0,02%, 0,05%, 0,1%, 0,2%, 0,3%, 0,4%, 0,5%, 0,6%, 0,7%, 0,8%, 0,9%, 1%, 2%, 5%, mindestens 5% oder mindestens 10% beläuft;
ii) *Hypo-Score,* optional wobei der epigenetische Partitions-Cutoff für den *Hypo-Score* sich auf 0,1%, 0,5%, 1%, 2%, 3%, 4%, 5%, 7% oder mindestens 10% beläuft;
iii) *Methyl-half,* optional wobei der epigenetische Partitions-Cutoff für den *Methyl-half* sich auf 5, 10, 15, 20, 25, 30, 35 oder 40 mCGs beläuft; oder
iv) *Methyl-5,* optional wobei der epigenetische Partitions-Cutoff für den *Methyl-5* sich auf 5, 10, 20, 30, 40 oder 50 mCGs beläuft.

10. Verfahren nach einem der vorhergehenden Ansprüche, wobei das Sequenzieren der Vielzahl von angereicherten Molekülen mithilfe eines Nukleinsäure-Sequenzierers durchgeführt wird, wobei der Nukleinsäure-Sequenzierer zum Beispiel ein Next-Generation-Sequenzierer ist.

11. Verfahren nach einem der vorhergehenden Ansprüche, wobei die Probe von Polynukleotiden
i) ausgewählt ist aus der Gruppe bestehend aus einer Probe von DNA, einer Probe von RNA, einer Probe von Polynukleotiden, einer Probe von zellfreier DNA und einer Probe von zellfreier RNA; und/oder
ii) eine zellfreie DNA ist, optional wobei die zellfreie DNA zwischen 1 ng und 500 ng beträgt.

12. Verfahren nach einem der vorhergehenden Ansprüche, ferner umfassend
i) Erstellen eines Berichts, der optional Informationen zu, und/oder Informationen abgeleitet aus, der Partitionierung von Nukleinsäuremolekülen beinhaltet; und optional
ii) Kommunizieren des Berichts an eine Drittpartei, wie etwa das Subjekt, aus dem die Probe abgeleitet wurde, oder einen Gesundheitspflege-Praktiker.

13. System, umfassend einen Controller, umfassend, oder fähig zum Zugriff auf, computerlesbare Medien, umfassend nichtflüchtige Computer-ausführbare Instruktionen, die, wenn sie durch mindestens einen elektronischen Prozessor ausgeführt werden, zumindest Folgendes vollführen:
a. Erhalten eines Satzes von Sequenzierungsablesungen von einer Probe, erstellt durch einen Nukleinsäure-Sequenzierer, wobei der Satz von Sequenzierungsablesungen aus Polynukleotiden der Probe erstellte Sequenzierungsablesungen umfasst, und wobei vor dem Sequenzieren die Probe gemäß der Schritte a und b von Anspruch 1 partitioniert and angereichert wurde;
b. Analysieren mindestens einer Teilmenge des Satzes von Sequenzierungsablesungen zum Erstellen eines oder mehrerer epigenetischer Partitions-Scores von endogenen Kontrollmolekülen;
c. Vergleichen des einen oder der mehreren epigenetischen Partitions-Scores mit einem oder mehreren epigenetischen Partitions-Cutoffs; und
d. Erstellen eines Ergebnis-Status des Partitionierungsverfahrens basierend auf dem Vergleich der epigenetischen Partitions-Scores, wobei der Ergebnis-Status des Partitionierungsverfahrens klassifiziert wird als (i) erfolgreich, wenn der eine oder die mehreren epigenetischen Partitions-Scores des Satzes von endogenen Kontrollmolekülen innerhalb der entsprechenden epigenetischen Partitions-Cutoffs liegt/liegen; oder (ii) nicht erfolgreich, wenn mindestens einer von dem einen oder den mehreren epigenetischen Partitions-Scores der endogenen Kontrollmoleküle außerhalb des entsprechenden epigenetischen Partitions-Cutoffs liegt.

14. System von Anspruch 13, wobei
die Probe eine versetzte Probe ist und gemäß der Schritte a und b von Anspruch 2 partitioniert and angereichert wurde;
Schritt b zusätzlich einen oder mehrere epigenetische Partitions-Scores für die Epigenetik-Kontrollnukleinsäuremoleküle erstellt; und
der Ergebnis-Status in Schritt d klassifiziert wird als (i) erfolgreich, wenn der eine oder die mehreren epigenetischen Partitions-Scores der Epigenetik-Kontrollnukleinsäuremoleküle und/oder des Satzes von endogenen Kontrollmolekülen innerhalb der entsprechenden epigenetischen Partitions-Cutoffs liegt/liegen; oder (ii) nicht erfolgreich, wenn mindestens einer von dem einen oder den mehreren epigenetischen Partitions-Scores der Epigenetik-Kontrollmoleküle und/oder der endogenen Kontrollmoleküle außerhalb des entsprechenden epigenetischen Partitions-Cutoffs liegt.

15. System von Anspruch 13 oder Anspruch 14, wobei es sich bei dem epigenetischen Partitions-Score handelt um
i) *O-CG-Score,* optional wobei der epigenetische Partitions-Cutoff für den *O-CG-Score* sich auf 0,01%, 0,02%, 0,05%, 0,1%, 0,2%, 0,3%, 0,4%, 0,5%, 0,6%, 0,7%, 0,8%, 0,9%, 1%, 2%, 5%, mindestens 5% oder mindestens 10% beläuft;
ii) *Hypo-Score,* optional wobei der epigenetische Partitions-Cutoff für den *Hypo-Score* sich auf 0,1%, 0,5%, 1%, 2%, 3%, 4%, 5%, 7% oder mindestens 10% beläuft;
iii) *Methyl-half,* optional wobei der epigenetische Partitions-Cutoff für den *Methyl-half* sich auf 5, 10, 15, 20, 25, 30, 35 oder 40 mCGs beläuft; oder
iv) *Methyl-5,* optional wobei der epigenetische Partitions-Cutoff für den *Methyl-5* sich auf 5, 10, 20, 30, 40 oder 50 mCGs beläuft.

## Revendications

1. Procédé permettant d'évaluer le partitionnement de molécules d'acide nucléique présentes dans un échantillon de polynucléotides sur la base d'un état épigénétique, comprenant :
a. le partitionnement de molécules d'acide nucléique provenant d'au moins un sous-ensemble de l'échantillon de polynucléotides en une pluralité d'ensembles partitionnés ;
b. l'enrichissement d'au moins un sous-ensemble de molécules comprenant des régions génomiques cibles spécifiques provenant de la pluralité d'ensembles partitionnés pour générer un ensemble de molécules enrichies, l'ensemble de molécules enrichies comprenant un groupe de molécules d'acide nucléique provenant de l'échantillon de polynucléotides, le groupe de molécules d'acide nucléique provenant de l'échantillon de polynucléotides comprenant un ensemble de molécules témoins endogènes, qui correspondent à au moins une région génomique humaine présentant un état épigénétique non variable ;
c. le séquençage d'au moins un sous-ensemble de l'ensemble de molécules enrichies pour générer une pluralité de lectures de séquençage ;
d. l'analyse d'au moins un sous-ensemble de l'ensemble de lectures de séquençage pour générer un ou plusieurs scores de partition épigénétique pour l'ensemble de molécules témoins endogènes ; et
e. la comparaison du ou des scores de partition épigénétique avec un ou plusieurs seuils de coupure de partition épigénétique.

2. Procédé selon la revendication 1, comprenant en outre une étape initiale d'ajout d'un ensemble de molécules d'acide nucléique témoins épigénétiques aux molécules d'acide nucléique présentes dans l'échantillon de polynucléotides, ce qui produit de cette manière un échantillon dopé, et dans lequel
l'étape a. est effectuée sur l'échantillon dopé ; au sein de l'étape b., l'ensemble de molécules enrichies comprend en outre un groupe de molécules d'acide nucléique témoins épigénétiques ; et
l'étape d. génère de plus un ou plusieurs scores de partition épigénétique pour les molécules d'acide nucléique témoins épigénétiques.

3. Procédé selon la revendication 2, dans lequel
i) l'ensemble de molécules d'acide nucléique témoins épigénétiques comprend deux ou plus de deux sous-ensembles de molécules d'acide nucléique témoins épigénétiques, un sous-ensemble des deux ou plus de deux sous-ensembles de molécules d'acide nucléique témoins épigénétiques comprenant une pluralité de molécules d'acide nucléique témoins épigénétiques comprenant une région de modification épigénétique, éventuellement
a) chaque sous-ensemble de molécules d'acide nucléique témoins épigénétiques étant en une concentration équimolaire ; ou chaque sous-ensemble de molécules d'acide nucléique témoins épigénétiques étant en une concentration non équimolaire ; et/ou
b) la région de modification épigénétique de la pluralité de molécules d'acide nucléique témoins épigénétiques présentes dans les deux ou plus de deux sous-ensembles comprenant une séquence d'acide nucléique identique ; ou la région de modification épigénétique de la pluralité de molécules d'acide nucléique témoins épigénétiques présentes dans un premier sous-ensemble comprenant une séquence d'acide nucléique pouvant être distinguée de la séquence d'acide nucléique de la région de modification épigénétique de la pluralité de molécules d'acide nucléique témoins épigénétiques présentes dans un second sous-ensemble ;
ii) la molécule d'acide nucléique témoin épigénétique comprend en outre une région d'identification, par exemple la région d'identification étant sur un côté ou les deux côtés de la région de modification épigénétique des molécules d'acide nucléique témoins épigénétiques, éventuellement la région d'identification des molécules d'acide nucléique témoins épigénétiques
a) comprenant un code à barres moléculaire ;
b) comprenant en outre au moins un code à barres d'état épigénétique ; et/ou
c) comprenant un ou plusieurs sites de liaison à des amorces ;
iii) la région de modification épigénétique des molécules d'acide nucléique témoins épigénétiques présentes dans au moins un sous-ensemble comprend au moins un nucléotide présentant une modification épigénétique ; et/ou
iv) la pluralité d'ensembles partitionnés comprend des molécules d'acide nucléique de l'échantillon dopé partitionné sur la base du niveau de méthylation des molécules d'acide nucléique.

4. Procédé selon l'une quelconque des revendications précédentes, dans lequel le partitionnement comprend le partitionnement des molécules d'acide nucléique sur la base d'une affinité de liaison différentielle des molécules d'acide nucléique à un agent de liaison qui se lie préférentiellement à des molécules d'acide nucléique comprenant des nucléotides présentant une modification épigénétique.

5. Procédé selon l'une quelconque des revendications précédentes, comprenant en outre le marquage des molécules d'acide nucléique présentes dans un ensemble partitionné de la pluralité d'ensembles partitionnés avec un ensemble de marqueurs pour produire une population de molécules d'acide nucléique marquées, dans lequel les molécules d'acide nucléique marquées comprennent un ou plusieurs marqueurs ; éventuellement dans lequel
l'ensemble de marqueurs utilisés dans un premier ensemble partitionné de la pluralité d'ensembles partitionnés est différent de l'ensemble de marqueurs utilisés dans un second ensemble partitionné de la pluralité d'ensembles partitionnés ; et éventuellement en outre
l'ensemble de marqueurs sont attachés aux molécules d'acide nucléique par ligation d'adaptateurs aux molécules d'acide nucléique, les adaptateurs comprenant un ou plusieurs marqueurs.

6. Procédé selon l'une quelconque des revendications précédentes, comprenant en outre
g. la classification du procédé comme (i) étant un succès, si le ou les scores de partition épigénétique des molécules d'acide nucléique témoins épigénétiques et/ou de l'ensemble de molécules témoins endogènes sont dans les limites des seuils de coupure de partition épigénétique correspondants ; ou (ii) n'étant pas un succès, si au moins l'un du ou des scores de partition épigénétique des molécules témoins épigénétiques et/ou de l'ensemble de molécules témoins endogènes est à l'extérieur du seuil de coupure de partition épigénétique correspondant.

7. Procédé selon l'une quelconque des revendications précédentes, dans lequel
i) la modification épigénétique est la méthylation d'ADN ;
ii) le nucléotide présentant une modification épigénétique comprend un nucléotide méthylé, par exemple
le nucléotide méthylé comprenant une 5-méthylcytosine ; ou
le nucléotide méthylé comprenant une 5-hydroxyméthylcytosine ; et/ou
iii) l'état épigénétique est le niveau de méthylation des molécules d'acide nucléique.

8. Procédé selon l'une quelconque des revendications précédentes,
dans lequel le score de partition épigénétique comprend une fraction ou un pourcentage
i) du nombre de molécules d'acide nucléique témoins épigénétiques hyperméthylées et/ou de molécules témoins hyperméthylées dans un ensemble partitionné ; ou
ii) du nombre de molécules d'acide nucléique témoins épigénétiques hypométhylées et/ou de molécules témoins hypométhylées dans un ensemble partitionné ; et/ou
dans lequel l'ensemble partitionné est
a) un ensemble partitionné hyperméthylé ; ou
b) un ensemble partitionné hypométhylé.

9. Procédé selon l'une quelconque des revendications précédentes, dans lequel le score de partition épigénétique est
i) un *score de 0 CG,* éventuellement le seuil de coupure de partition épigénétique pour le *score de 0 CG* étant de 0,01 %, 0,02 %, 0,05 %, 0,1 %, 0,2 %, 0,3 %, 0,4 %, 0,5 %, 0,6 %, 0,7 %, 0,8 %, 0,9 %, 1 %, 2 %, 5 %, au moins 5 % ou au moins 10 % ;
ii) un *score d'hypo,* éventuellement le seuil de coupure de partition épigénétique pour le *score d'hypo* étant de 0,1 %, 0,5 %, 1 %, 2 %, 3 %, 4 %, 5 %, 7 % ou au moins 10 % ;
iii) un *méthyl-moitié,* éventuellement le seuil de coupure de partition épigénétique pour le *méthyl-moitié* étant de 5, 10, 15, 20, 25, 30, 35 ou 40 mCG ; ou
iv) un *méthyl-5,* éventuellement le seuil de coupure de partition épigénétique pour le *méthyl-5* étant de 5, 10, 20, 30, 40 ou 50 mCG.

10. Procédé selon l'une quelconque des revendications précédentes, dans lequel le séquençage de la pluralité de molécules enrichies est effectué par un séquenceur d'acides nucléiques, par exemple le séquenceur d'acides nucléiques étant un séquenceur de nouvelle génération.

11. Procédé selon l'une quelconque des revendications précédentes, dans lequel l'échantillon de polynucléotides est
i) choisi dans le groupe constitué par un échantillon d'ADN, un échantillon d'ARN, un échantillon de polynucléotides, un échantillon d'ADN acellulaire et un échantillon d'ARN acellulaire ; et/ou
ii) un ADN acellulaire, éventuellement l'ADN acellulaire étant compris entre 1 ng et 500 ng.

12. Procédé selon l'une quelconque des revendications précédentes, comprenant en outre
i) la génération d'un rapport qui renferme éventuellement des informations sur le partitionnement de molécules d'acide nucléique et/ou des informations dérivées de celui-ci ; et éventuellement
ii) la communication du rapport à un tiers, tel que le sujet dont est issu l'échantillon ou un professionnel de la santé.

13. Système, comprenant un dispositif de commande comprenant un support lisible par ordinateur, ou capable d'avoir accès à celui-ci, comprenant des instructions exécutables par ordinateur non transitoires qui, lorsqu'elles sont exécutées par au moins un processeur électronique, effectuent au moins :
a. l'obtention d'un ensemble de lectures de séquençage d'un échantillon générées par un séquenceur d'acides nucléiques, l'ensemble de lectures de séquençage comprenant des lectures de séquençage générées à partir de polynucléotides de l'échantillon et l'échantillon ayant été partitionné et enrichi avant le séquençage selon les étapes a et b de la revendication 1 ;
b. l'analyse d'au moins un sous-ensemble de l'ensemble de lectures de séquençage pour générer un ou plusieurs scores de partition épigénétique de molécules témoins endogènes ;
c. la comparaison du ou des scores de partition épigénétique avec un ou plusieurs seuils de coupure de partition épigénétique ; et
d. la génération d'un état de résultat du procédé de partitionnement sur la base de la comparaison des scores de partition épigénétique, l'état de résultat du procédé de partitionnement étant classé comme (i) étant un succès, si le ou les scores de partition épigénétique de l'ensemble de molécules témoins endogènes sont dans les limites des seuils de coupure de partition épigénétique correspondants ; ou (ii) n'étant pas un succès, si au moins l'un du ou des scores de partition épigénétique des molécules témoins endogènes est à l'extérieur du seuil de coupure de partition épigénétique correspondant.

14. Système selon la revendication 13, dans lequel
l'échantillon est un échantillon dopé et a été partitionné et enrichi selon les étapes a et b de la revendication 2 ;
l'étape b génère de plus un ou plusieurs scores de partition épigénétique pour les molécules d'acide nucléique témoins épigénétiques ; et
l'état de résultat à l'étape d est classé comme (i) étant un succès, si le ou les scores de partition épigénétique des molécules d'acide nucléique témoins épigénétiques et/ou de l'ensemble de molécules témoins endogènes sont dans les limites des seuils de coupure de partition épigénétique correspondants ; ou (ii) n'étant pas un succès, si au moins l'un du ou des scores de partition épigénétique des molécules témoins épigénétiques et/ou des molécules témoins endogènes est à l'extérieur du seuil de coupure de partition épigénétique correspondant.

15. Système selon la revendication 13 ou la revendication 14, dans lequel le score de partition épigénétique est
i) un *score de 0 CG,* éventuellement le seuil de coupure de partition épigénétique pour le *score de 0 CG* étant de 0,01 %, 0,02 %, 0,05 %, 0,1 %, 0,2 %, 0,3 %, 0,4 %, 0,5 %, 0,6 %, 0,7 %, 0,8 %, 0,9 %, 1 %, 2 %, 5 %, au moins 5 % ou au moins 10 % ;
ii) un *score d'hypo,* éventuellement le seuil de coupure de partition épigénétique pour le *score d'hypo* étant de 0,1 %, 0,5 %, 1 %, 2 %, 3 %, 4 %, 5 %, 7 % ou au moins 10 % ;
iii) un *méthyl-moitié,* éventuellement le seuil de coupure de partition épigénétique pour le *méthyl-moitié* étant de 5, 10, 15, 20, 25, 30, 35 ou 40 mCG ; ou
iv) un *méthyl-5,* éventuellement le seuil de coupure de partition épigénétique pour le *méthyl-5* étant de 5, 10, 20, 30, 40 ou 50 mCG.
